(19) Europäisches Patentamt
European Patent Office
Office européen des brevets

(11) EP 3 519 415 B1

(12) # EUROPÄISCHE PATENTSCHRIFT

(45) Veröffentlichungstag und Bekanntmachung des Hinweises auf die Patenterteilung:
**14.12.2022 Patentblatt 2022/50**

(21) Anmeldenummer: **17771473.0**

(22) Anmeldetag: **27.09.2017**

(51) Internationale Patentklassifikation (IPC):
***C07D 491/04*** (2006.01) ***C07D 495/04*** (2006.01)
***C07D 519/00*** (2006.01) ***C09K 11/06*** (2006.01)
***H01L 51/00*** (2006.01) ***H01L 51/50*** (2006.01)
***H05B 33/10*** (2006.01)

(52) Gemeinsame Patentklassifikation (CPC):
**C07D 491/04; C07D 495/04; C07D 519/00; C09K 11/02; H01L 51/0071; H01L 51/0072; H01L 51/504;** H01L 51/5016; H01L 2251/5384; Y02E 10/549

(86) Internationale Anmeldenummer:
**PCT/EP2017/074437**

(87) Internationale Veröffentlichungsnummer:
**WO 2018/060218 (05.04.2018 Gazette 2018/14)**

(54) **CARBAZOLE MIT DIAZADIBENZOFURAN- ODER DIAZADIBENZOTHIOPHEN-STRUKTUREN**

CARBAZOLES WITH DIAZADIBENZOFURANE OR DIAZADIBENZOTHIOPHENE STRUCTURES

CARBAZOLES COMPRENANT DES STRUCTURES DIAZADIBENZOFURANE OU DIAZADIBENZOTHIOPHÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.09.2016 EP 16191707**

(43) Veröffentlichungstag der Anmeldung:
**07.08.2019 Patentblatt 2019/32**

(73) Patentinhaber: **Merck Patent GmbH**
**64293 Darmstadt (DE)**

(72) Erfinder:
- **PARHAM, Amir**
  **60486 Frankfurt am Main (DE)**
- **EBERLE, Thomas**
  **76829 Landau (DE)**
- **JATSCH, Anja**
  **60489 Frankfurt am Main (DE)**
- **GROSSMANN, Tobias**
  **64297 Darmstadt (DE)**
- **KROEBER, Jonas**
  **60311 Frankfurt am Main (DE)**

(56) Entgegenhaltungen:
**EP-A1- 2 910 555** **WO-A1-2015/108301**
**WO-A1-2017/119654** **US-A1- 2015 325 799**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 519 415 B1

## Beschreibung

Die vorliegende Erfindung beschreibt Diazadibenzofuran- oder Diazadibenzothiophenderivate mit Carbazolstrukturen, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energieund Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden als Matrixmaterialien für phosphoreszierende Verbindungen sowie als Elektronentransportmaterialien häufig heteroaromatische Verbindungen eingesetzt. Bekannt für diese Funktion sind beispielsweise Diazadibenzofuranderivate, welche mit Carbazolgruppen substituiert sind, wie in JP 5604848 B2, WO 2015/182872 A1, KR20160026661 und WO 2014/157599 A1 offenbart. Allerdings zeigen die Diazadibenzofurangruppen nicht zwingend eine doppelte Substitution in dem Diazaphenylrest der Diazadibenzofurangruppe mit Aryl- oder Heteroarylgruppen und eine weitetre Substiturion am Stickstoff-freien Benzolring der Diazabenzofuraneinheit. Ferner zeigen einige der dargelegten Verbindungen zwei Diazaphenylreste in der Diazadibenzofurangruppe.

WO 2017/119654 A1 beschreibt Verbindungen mit Diazadibenzofuran- und Diazadibenzothiophen-Einheiten sowie ein organisches elektrisches mit einer ersten und zweiten Elektrode und einer organischen Materialschicht zwischen der ersten und zweiten Elektrode, umfassend die besagte Verbindung.

US 2015/325799 A1 beschreibt kondensierte zyklische Verbindungen, die Diazadibenzofuran- und Diazadibenzothiophen-Einheiten enthalten, und organische lichtemittierende Vorrichtungen, die diese umfassen.

WO 2015/108301 A1 beschreibt neuartige kondensierte Ringverbindungen mit Diazadibenzofuran- und Diazadibenzothiophen-Einheiten und organische lichtemittierende Vorrichtungen, die diese umfassen.

EP 2910555 A1 stellt Verbindungen auf Basis von Azadibenzothiophenen, Azadibenzofuranen und Azadibenzoselenophene mit mindestens zwei Stickstoffatomen in den Aza-Ringen vor. Die Verbindungen können in grün, rot, gelb und weiß emittierende Vorrichtungen als elektronentransportierende Wirtsmaterialien eingesetzt werden.

Generell besteht bei diesen Materialien, beispielsweise für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen.

Ferner kann eine weitere Aufgabe der vorliegenden Erfindung darin gesehen werden, Verbindungen bereitzustellen, welche sich für den Einsatz in einer phosphoreszierenden oder fluoreszierenden OLED insbesondere als Elektronentransportmaterialien eignen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten

Leistungsfähigkeit möglichst kostengünstig und in konstanter Qualität bereitzustellen.

Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen und den Nachteil aus dem Stand der Technik beseitigen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Gegenstand der vorliegenden Erfindung ist daher eine Verbindung, umfassend Strukturen der Formeln (Ic), (IIc) und/oder (IIIc)

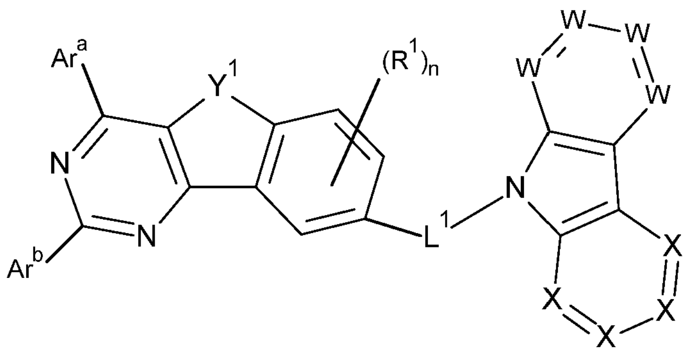

Formel (Ic)

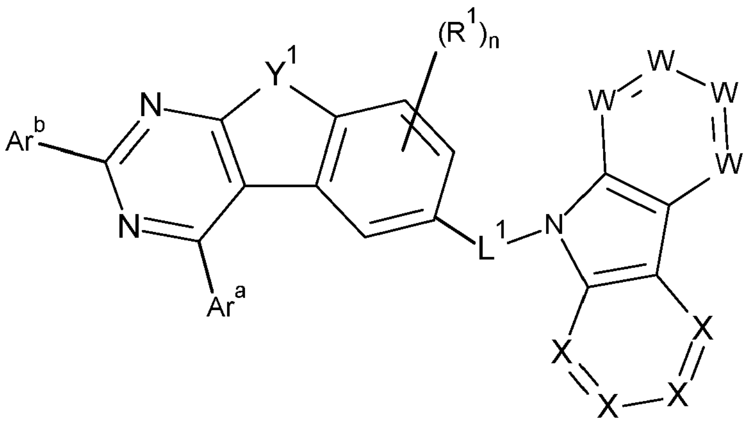

Formel (IIc)

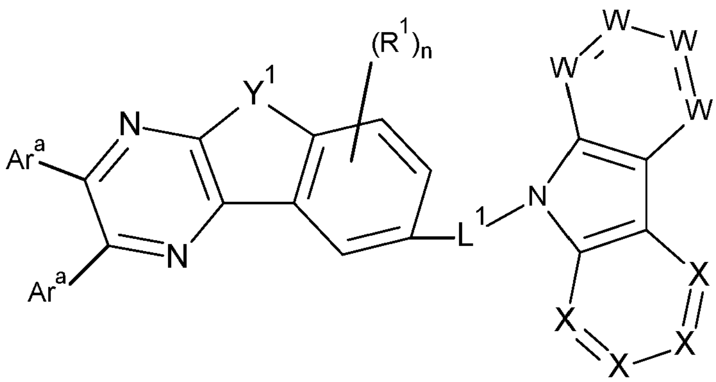

Formel (IIIc)

wobei für die verwendeten Symbole gilt:

$Y^1$ ist O oder S;

W ist bei jedem Auftreten gleich oder verschieden N oder CR1, vorzugsweise CR1, mit der Maßgabe, dass nicht mehr als zwei der Gruppen W in einem Cyclus für N stehen;

X ist bei jedem Auftreten gleich oder verschieden N oder CR1, vorzugsweise CR1, mit der Maßgabe, dass nicht mehr als zwei der Gruppen X in einem Cyclus für N stehen;

$L^1$ ist eine Bindung oder ein aromatisches oder heteroaromatisches Ringsystem mit 5 bis 30 aromatischen Ringatomen, welches durch einen oder mehrere Reste $R^1$ substituiert sein kann;

$Ar^a$ ist bei jedem Auftreten gleich oder verschieden CN, F oder ein aromatisches oder heteroaromatisches Ringsystem mit 5 bis 30 aromatischen Ringatomen, welches durch einen oder mehrere Reste $R^1$ substituiert sein kann;

$Ar^b$ ist bei jedem Auftreten gleich oder verschieden CN, F oder ein aromatisches oder heteroaromatisches Ringsystem mit 5 bis 30 aromatischen Ringatomen, welches durch einen oder mehrere Reste $R^1$ substituiert sein kann;

$R^1$ ist bei jedem Auftreten gleich oder verschieden H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(A_r{}^1)_2$, $B(Ar^1)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C=C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder einer Aralkyl- oder Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere Substituenten $R^1$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten $R^2$ substituiert sein kann; dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, CHO, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische

Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, -C=C-, $Si(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder polycyclisches, aliphatisches oder aromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

n ist 0, 1, 2 oder 3, vorzugsweise 0, 1 oder 2, besonders bevorzugt 0 oder 1, speziell bevorzugt 0;

wobei unter einem aromatischen oder heteroaromatischen Ringsystem ein System zu verstehen ist, das nicht notwendigerweise nur Aryloder Heteroarylgruppen enthält, sondern in dem auch eine oder mehrere Aryloder Heteroarylgruppen durch ein C-, N- oder O-Atom oder eine Carbonylgruppe unterbrochen sein können; wobei Systeme, in denen zwei oder mehrere Arylgruppen durch eine lineare oder cyclische Alkylgruppe oder durch eine Silylgruppe unterbrochen sind, ebenso als aromatische Ringsysteme zu verstehen sind; und
wobei Systeme, in denen zwei oder mehrere Aryl- oder Heteroarylgruppen direkt aneinander gebunden sind, ebenfalls als aromatisches bzw. heteroaromatisches Ringsystem zu verstehen sind.
Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

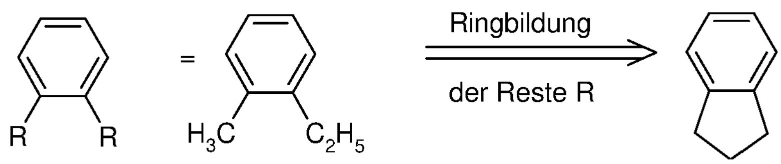


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

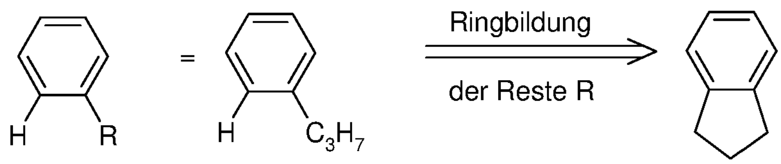


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindestens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für

Heteroarylgruppen sowie für kondensierte Ringsysteme, die auch Heteroatome enthalten können, jedoch nicht müssen.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryloder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält , wenn nicht anders angegeben, 6 bis 40 C-Atome im Ringsystem. Ein heteroaromatisches Ringsystem im Sinne dieser Erfindung enthält , wenn nicht anders angegeben, 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), nämlich ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die erfindungsgemäßen Verbindungen umfassen Strukturen gemäß mindestens einer der Formeln (Ic), (IIc)

und/oder (IIIc)

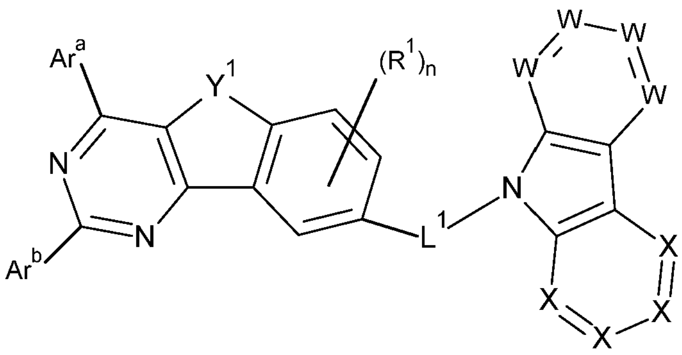

Formel (Ic)

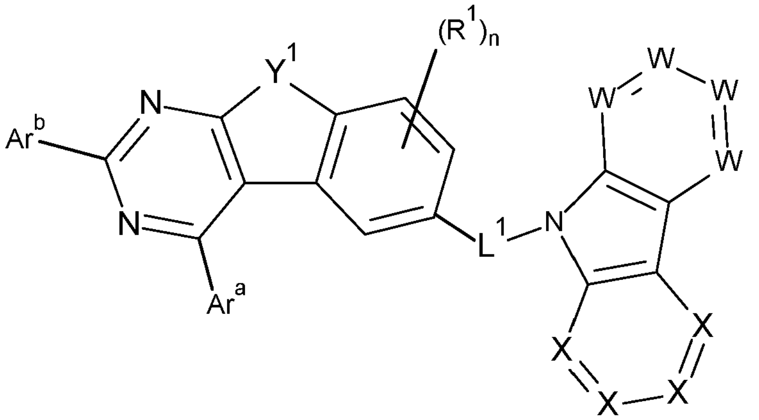

Formel (IIc)

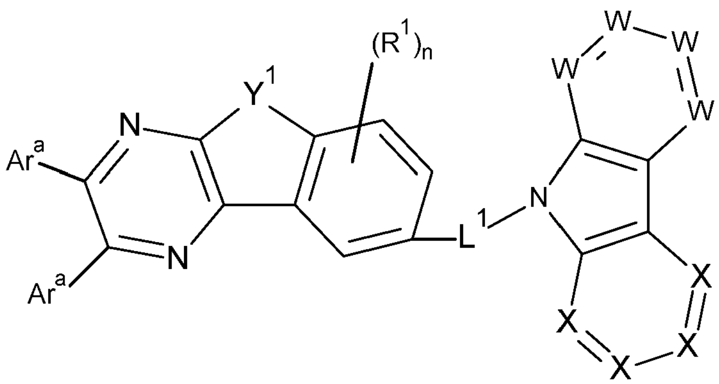

Formel (IIIc)

worin die Symbole X, $Ar^a$, $Ar^b$, $Y^1$, $L^1$, $R^1$, n und W die zuvor dargelegte Bedeutung aufweisen, wobei Strukturen der Formel (Ic) und/oder (IIc) bevorzugt sind.

Ferner kann vorgesehen sein, dass die erfindungsgemäße Verbindung mindestens eine der Strukturen der Formeln (IVc), (Vc) und/oder (VIc) umfasst

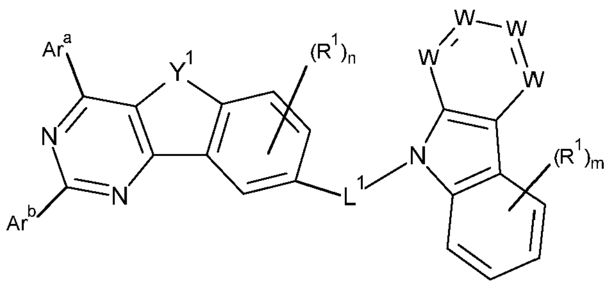

Formel (IVc)

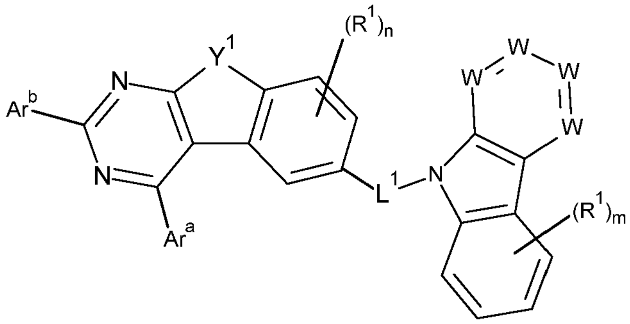

Formel (Vc)

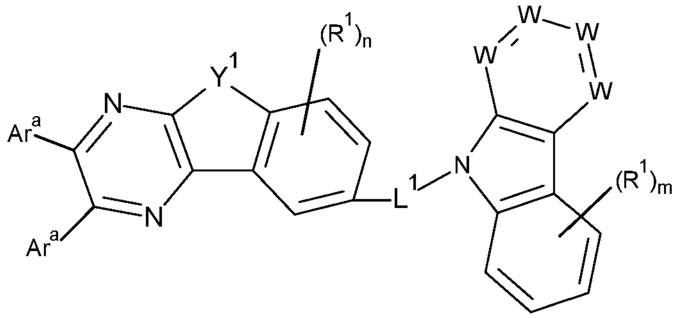

Formel (VIc)

worin die Symbole $Ar^a$, $Ar^b$, $Y^1$, $L^1$, $R^1$, n und W die zuvor dargelegte Bedeutung aufweisen und m 0, 1, 2, 3 oder 4, vorzugsweise 0, 1, 2 oder 3, besonders bevorzugt 0, 1 oder 2 ist, wobei Strukturen der Formel (IVc) und/oder (Vc) bevorzugt sind.

Weiterhin kann vorgesehen sein, dass die Substituenten $R^1$ der Strukturen gemäß einer der Formeln (Ic), (IIc), (IIIc), (IVc), (Vc) und/oder (VIc), die nicht Teil einer durch das Symbol W dargestellten Gruppe $CR^1$ sind, mit den Ringatomen der jeweiligen Ringstruktur kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Substituenten $R^1$ der Strukturen gemäß einer der Formeln (Ic), (IIc), (IIIc), (IVc), (Vc) und/oder (VIc), die nicht Teil einer durch das Symbol W dargestellten Gruppe $CR^1$ sind, mit den Ringatomen der jeweiligen Ringstruktur kein Ringsystem bilden. Dies schließt die Bildung eines Ringsystems mit möglichen Substituenten $R^2$, $R^3$ ein, die an die Reste $R^1$ gebunden sein können.

Ferner kann vorgesehen sein, dass die Summe der Indices n und m in den Strukturen der Formeln (Ic), (IIc), (IIIc), (IVc), (Vc) und/oder (VIc) jeweils höchstens 3, vorzugsweise höchstens 2 und besonders bevorzugt höchstens 1 beträgt.

In den Strukturen der Formeln (Ic), (IIc), (IIIc), (IVc), (Vc) und/oder (VIc) können zwei benachbarte Gruppen W jeweils für $CR^1$ stehen und zusammen eine Gruppe der Formel (W-1) bilden

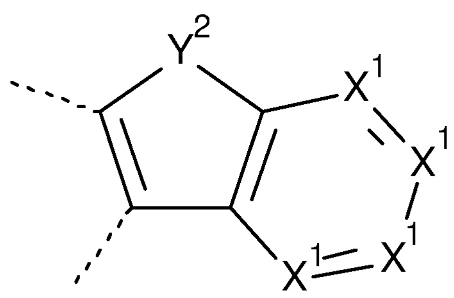

Formel (W-1)

worin

- $Y^2$ N(Ar), O, S oder $C(R^2)_2$, vorzugsweise $C(R^2)_2$ ist,
- $X^1$ bei jedem Auftreten gleich oder verschieden N oder $CR^2$, vorzugsweise $CR^2$ ist, mit der Maßgabe, dass nicht mehr als zwei der Gruppen X in einem Cyclus für N stehen, wobei $R^2$ die zuvor genannte Bedeutung aufweist,
- Ar bei jedem Auftreten gleich oder verschieden ein aromatisches oder heteroaromatisches Ringsystem mit 5 bis 30 aromatischen Ringatomen ist, welches durch einen oder mehrere Reste $R^2$ substituiert sein kann, die Reste $R^2$ die zuvor genannte Bedeutung aufweisen, und

die gestrichelten Linien die Bindungen zu den benachbarten Atomen darstellen. Vorzugsweise weisen die Verbindungen umfassend Strukturen gemäß der Formel (Ic), (IIc), (IIIc), (IVc), (Vc) und/oder (VIc) vorzugsweise höchstens eine Gruppe der Formel (W-1) pro Struktur auf.

Darüber hinaus sind Verbindungen bevorzugt, die dadurch gekennzeichnet sind, dass in Formeln (Ic), (IIc), (IIIc), (IVc), (Vc) und/oder (VIc) nicht mehr als zwei, vorzugsweise nicht mehr als eine Gruppe W für N steht, vorzugsweise alle W für $CR^1$ stehen, wobei vorzugsweise höchstens 4, besonders bevorzugt höchstens 3 und speziell bevorzugt höchstens 2 der Gruppen $CR^1$ für die W steht, ungleich der Gruppe CH ist.

Ferner kann vorgesehen sein, dass die Reste $R^1$ der Gruppen W in den Formeln (Ic), (IIc), (IIIc), (IVc), (Vc) und/oder (VIc) mit den Ringatomen der Ringstruktur kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Reste $R^1$ der Gruppen W in den Formeln (Ic), (IIc), (IIIc), (IVc), (Vc) und/oder (VIc) mit den Ringatomen der Ringstruktur kein Ringsystem bilden. Dies schließt die Bildung eines Ringsystems mit möglichen Substituenten $R^2$, $R^3$ ein, die an die Reste $R^1$ gebunden sein können.

Die Reste $Ar^a$ und/oder $Ar^b$ in den Strukturen gemäß Formeln (Ic), (IIc), (IIIc), (IVc), (Vc) und/oder (VIc) stellen bei jedem Auftreten gleich oder verschieden CN, F oder ein aromatisches oder heteroaromatisches Ringsystem mit 5 bis 30 aromatischen Ringatomen dar, welches durch einen oder mehrere Reste $R^1$ substituiert sein kann. Hierbei stehen die Symbole $Ar^a$ und/oder $Ar^b$ vorzugsweise für ein aromatisches oder heteroaromatisches Ringsystem mit 5 bis 30 aromatischen Ringatomen.

Vorzugsweise kann in Strukturen gemäß Formeln (Ic), (IIc), (IIIc), (IVc), (Vc), (VIc) und/oder (W-1) eine der Gruppen Ar, $Ar^a$ und/oder $Ar^b$ höchstens 5, vorzugsweise höchstens 3 und besonders bevorzugt höchstens 1 Heteroatom aufweist, wobei dies Substituenten $R^1$, $R^2$ und $R^3$ einschließt, die an diese Gruppen gebunden sein können. Speziell bevorzugt weisen die Gruppen Ar, $Ar^a$ und/oder $Ar^b$ kein Heteroatom auf, wobei dies Substituenten $R^1$, $R^2$ und $R^3$ einschließt, die an diese Gruppen gebunden sein können.

Ferner kann vorgesehen sein, dass die Gruppen Ar, $Ar^a$ und/oder $Ar^b$ in Strukturen gemäß Formeln (Ic), (IIc), (IIIc), (IVc), (Vc), (VIc)und/oder (W-1) insgesamt höchstens 5, vorzugsweise höchstens 3 und besonders bevorzugt höchstens 1 Heteroatom aufweisen, wobei dies Substituenten $R^1$, $R^2$ und $R^3$ einschließt, die an diese Gruppen gebunden sein können.

Weiterhin kann vorgesehen sein, dass die Gruppen Ar, $Ar^a$ und/oder $Ar^b$ in Strukturen gemäß Formeln (Ic), (IIc), (IIIc), (IVc), (Vc), (VIc)und/oder (W-1) insgesamt höchstens 50, vorzugsweise höchstens 40 und besonders bevorzugt höchstens 22 aromatische Ringatome aufweisen, wobei dies Substituenten $R^1$, $R^2$ und $R^3$ einschließt, die an diese Gruppen gebunden sein können.

Darüber hinaus kann vorgesehen sein, dass der Rest Ar in der Struktur gemäß Formel (W-1) keine Carbazol-Gruppe umfasst, wobei dies Substituenten $R^2$ und $R^3$, die an den Rest Ar gebunden sein können, einschließt.

Vorzugsweise umfasst der Rest $Ar^a$ in Strukturen gemäß Formeln (Ic), (IIc), (IIIc), (IVc), (Vc) und/oder (VIc)keine Carbazol-Gruppe umfasst, wobei dies Substituenten $R^1$, $R^2$ und $R^3$, die an den Rest $Ar^a$ gebunden sein können, einschließt.

In einer weiterhin bevorzugten Ausgestaltung kann die Gruppe $Ar^b$ in Strukturen gemäß Formeln (Ic), (IIc), (IIIc), (IVc), (Vc) und/oder (VIc) eine Gruppe der Formel ($Ar^b$-1) darstellen

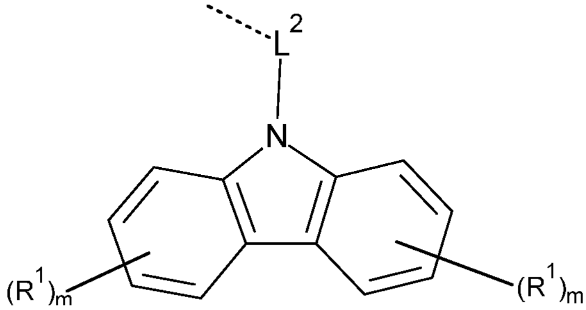

Formel ($Ar^b$-1)

worin $L^2$ eine Bindung oder ein aromatisches oder heteroaromatisches Ringsystem mit 5 bis 14 aromatischen oder heteroaromatischen Ringatomen ist, welches durch einen oder mehrere Reste $R^1$ substituiert sein kann, das Symbol $R^1$ die zuvor genannte Bedeutung aufweist, m 0, 1, 2, 3 oder 4, vorzugsweise 0, 1 oder 2 ist und die gestrichelte Linie die Bindung zur Diazadibenzofuranoder Diazadibenzothiophen-Gruppe darstellt.

Vorzugsweise kann vorgesehen sein, dass die Summe der Indices m in den Strukturen der Formeln ($Ar^b$-1) jeweils höchstens 3, vorzugsweise höchstens 2 und besonders bevorzugt höchstens 1 beträgt.

Ferner kann vorgesehen sein, dass die Reste $R^1$ in den Strukturen der Formeln ($Ar^b$-1) mit den Ringatomen der Ringstruktur, an die der Rest $R^1$ bindet, kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$, $R^3$ ein, die an die Reste $R^1$ gebunden sein können. Bevorzugt kann vorgesehen sein, dass die Reste $R^1$ in den Strukturen der Formeln ($Ar^b$-1) mit den Ringatomen der Ringstruktur kein Ringsystem bilden. Dies schließt die Bildung eines Ringsystems mit möglichen Substituenten $R^2$, $R^3$ ein, die an die Reste $R^1$ gebunden sein können.

Für Strukturen der Formeln (Ic), (IIc), (IIIc), (IVc), (Vc) und/oder (VIc) kann der Rest $Ar^a$ ebenfalls für eine Gruppe der Formel ($Ar^b$-1) stehen. Dies ist jedoch im Allgemeinen nicht bevorzugt. Vorzugsweise kann demgemäß vorgesehen sein, dass der Rest $Ar^a$ in den Strukturen der Formeln (Ic), (IIc), (IIIc), (IVc), (Vc) und/oder (VIc) keine Carbazol-Gruppe umfasst, wobei dies Substituenten $R^1$, $R^2$ und $R^3$, die an den Rest $Ar^a$ gebunden sein können, einschließt.

Ferner kann vorgesehen sein, dass der Rest Ar, $Ar^a$ und/oder $Ar^b$ ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3-oder 4-Dibenzofuranyl, 1-, 2-, 3- oder 4-Dibenzothienyl und 1-, 2-, 3- oder 4-Carbazolyl, Spirobifluorenyl, Fluorenyl, Dibenzofuranyl, Dibenzothiophenyl, Anthracenyl, Phenanthrenyl und/oder Triphenylenyl, die jeweils durch einen oder mehrere Reste $R^2$ substituiert sein können, bevorzugt aber unsubstituiert sind, wobei Spirobifluoren-, Fluoren-, Dibenzofuran-, Dibenzothiophen-, Anthracen-, Phenanthren-, Triphenylen-Gruppen besonders bevorzugt sind.

In einer weiteren bevorzugten Ausführungsform enthalten weder $Ar^a$ noch $Ar^b$ in den oben genannten Verbindungen eine Carbazol-, Dibenzofuranoder Dibenzothiophengruppe. Ganz bevorzugt ist, wenn weder $Ar^a$ noch $Ar^b$ in den oben genannten Verbindungen eine kondensierte heteroaromatische Gruppe enthalten. Besonders bevorzugt ist, wenn weder $Ar^a$ noch $Ar^b$ in den oben genannten Verbindungen eine heteroaromatische Gruppe enthalten.

Gemäß einer bevorzugten Ausgestaltung sind Verbindungen, umfassend Strukturen gemäß Formel (Ic), (IIc), (IIIc), (IVc), (Vc) und/oder (VIc) durch Strukturen der Formel (Ic), (IIc), (IIIc), (IVc), (Vc) und/oder (VIc) darstellbar. Vorzugsweise weisen Verbindungen, umfassend Strukturen gemäß Formel (Ic), (IIc), (IIIc), (IVc), (Vc) und/oder (VIc), ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

Wenn W für $CR^1$ steht bzw. wenn die aromatische und/oder heteroaromatische Gruppen durch Substituenten $R^1$ substituiert sind, dann sind diese Substituenten $R^1$ bevorzugt gewählt aus der Gruppe bestehend aus H, D, F, CN, $N(Ar^1)_2$, $C(=O)Ar^1$, $P(=O)(Ar^1)_2$, einer geradkettigen Alkyloder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen,

die jeweils mit einem oder mehreren Resten $R^2$ substituiert sein kann, wobei eine oder mehrere nichtbenachbarte $CH_2$-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten $R^2$ substituiert sein kann, bevorzugt aber unsubstituiert ist, oder einer Aralkyl- oder Heteroaralkylgruppe mit 5 bis 25 aromatischen Ringatomen, die mit einem oder mehreren Resten $R^2$ substituiert sein kann; dabei können optional zwei Substituenten $R^1$, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten $R^1$ substituiert sein kann; wobei $Ar^1$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, eine Aryloxygruppe mit 5 bis 60 aromatischen Ringatomen, die mit einem oder mehreren Resten $R^2$ substituiert sein kann, oder einer Aralkylgruppe mit 5 bis 60 aromatischen Ringatomen, die jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit einem oder mehreren Resten $R^3$ substituiert sein kann, wobei das Symbol $R^2$ die zuvor genannte Bedeutung aufweisen. Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Besonders bevorzugt sind diese Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist; dabei können optional zwei Substituenten $R^1$, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten $R^2$ substituiert sein kann, bevorzugt aber unsubstituiert ist, wobei $Ar^1$ die zuvor dargelegte Bedeutung aufweisen kann.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Weiterhin kann vorgesehen sein, dass in einer Struktur gemäß Formel (Ic), (IIc), (IIIc), (IVc), (Vc) und/oder (VIc) mindestens ein Rest $R^1$, Ar, $Ar^1$, $Ar^a$ oder $Ar^b$ für eine Gruppe steht, die ausgewählt ist aus den Formeln ($R^1$-1) bis ($R^1$- 87)

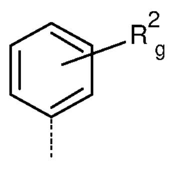

Formel ($R^1$-1)

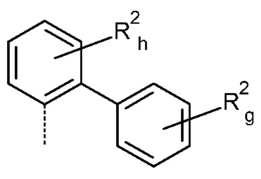

Formel ($R^1$-2)

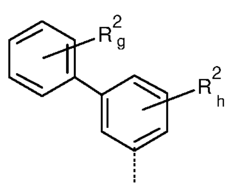

Formel ($R^1$-3)

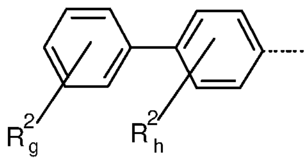

Formel ($R^1$-4)

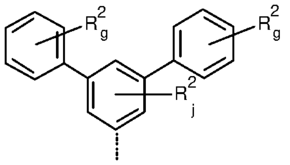

Formel ($R^1$-5)

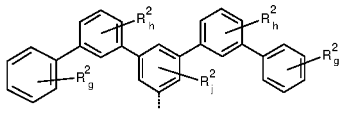

Formel ($R^1$-6)

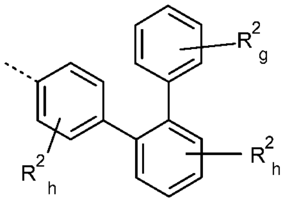

Formel ($R^1$-7)

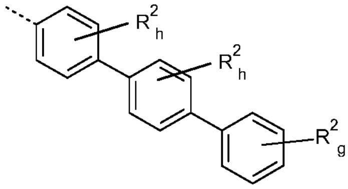

Formel ($R^1$-8)

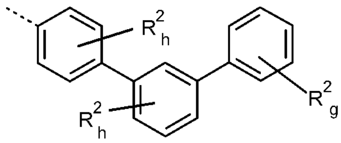

Formel ($R^1$-9)

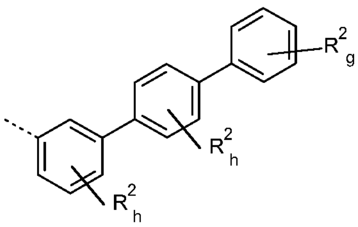

Formel ($R^1$-10)

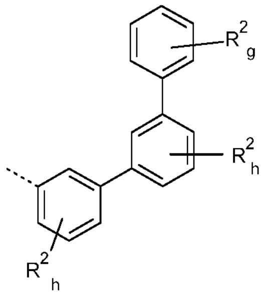

Formel ($R^1$-11)

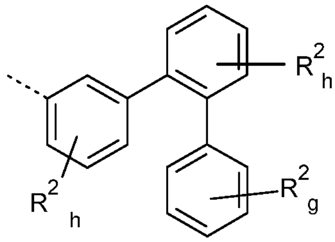

Formel ($R^1$-12)

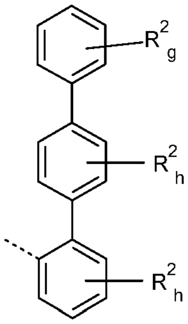

Formel ($R^1$-13)

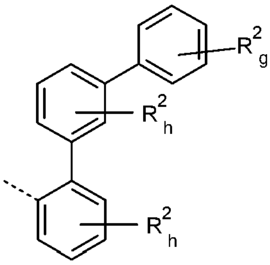

Formel ($R^1$-14)

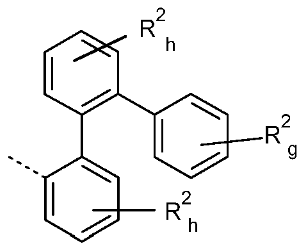

Formel ($R^1$-15)

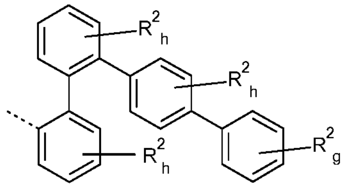

Formel ($R^1$-16)

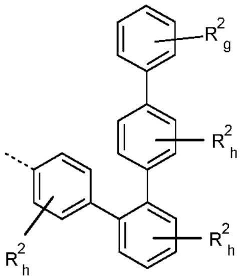

Formel ($R^1$-17)

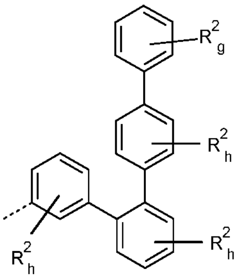

Formel ($R^1$-18)

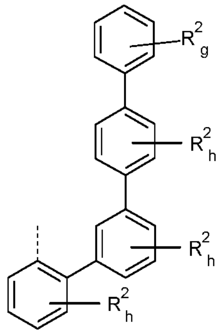

Formel ($R^1$-19)

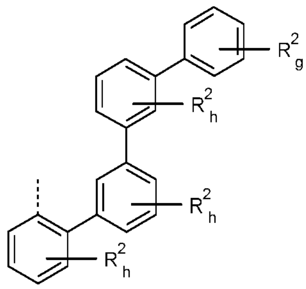

Formel ($R^1$-20)

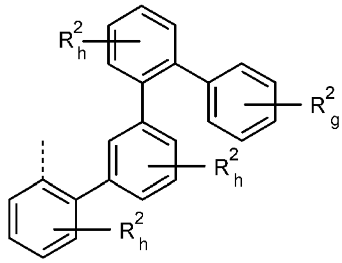

Formel ($R^1$-21)

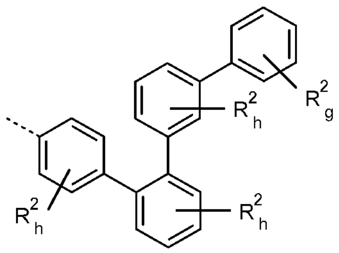

Formel ($R^1$-22)

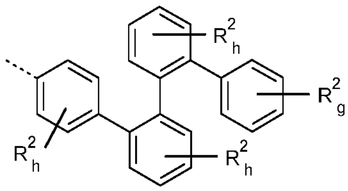

Formel ($R^1$-23)

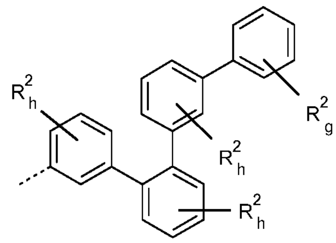

Formel ($R^1$-24)

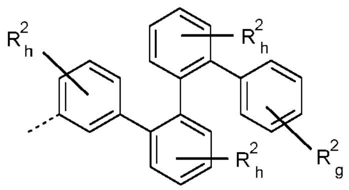

Formel ($R^1$-25)

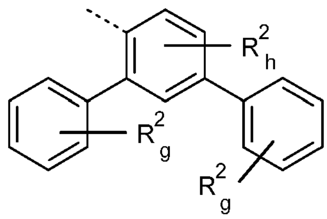

Formel ($R^1$-26)

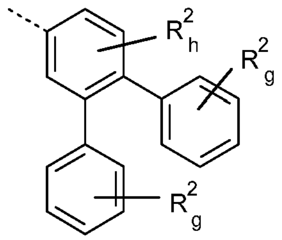

Formel ($R^1$-27)

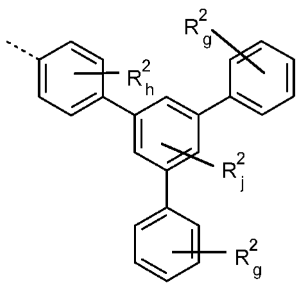

Formel ($R^1$-28)

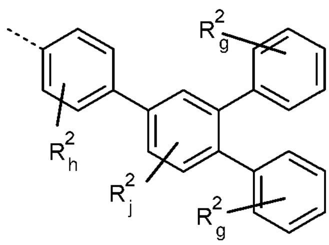

Formel ($R^1$-29)

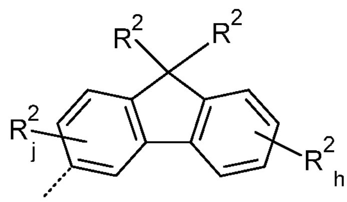

Formel ($R^1$-30)

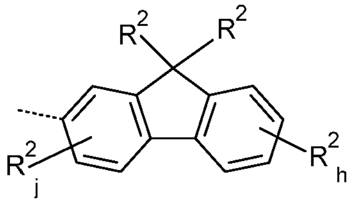
Formel ($R^1$-31)

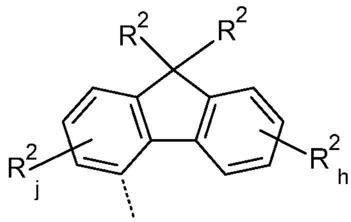
Formel ($R^1$-32)

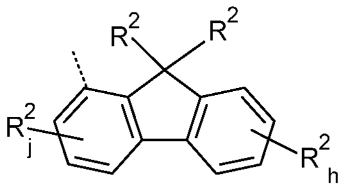
Formel ($R^1$-33)

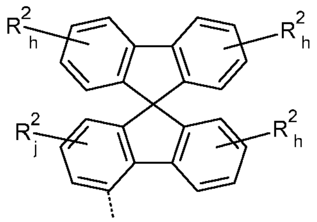
Formel ($R^1$-34)

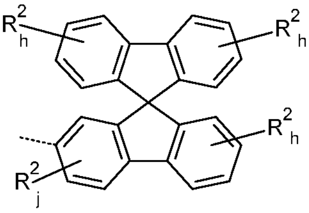
Formel ($R^1$-35)

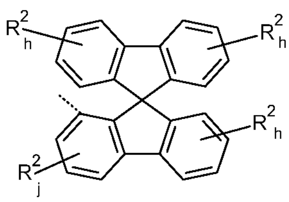
Formel ($R^1$-36)

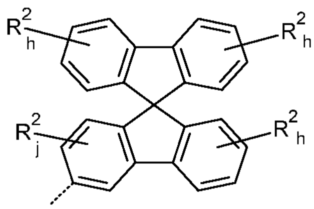
Formel ($R^1$-37)

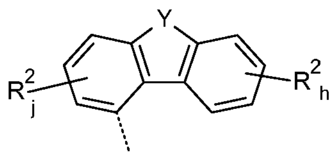
Formel ($R^1$-38)

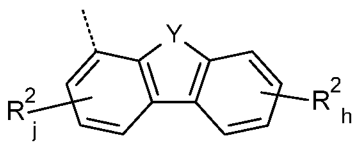
Formel ($R^1$-39)

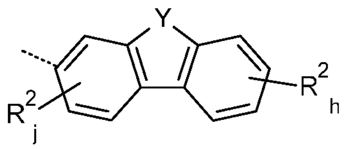
Formel ($R^1$-40)

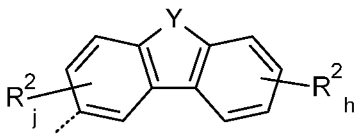
Formel ($R^1$-41)

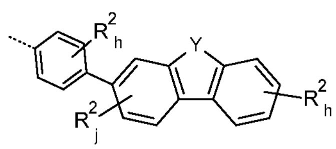
Formel ($R^1$-42)

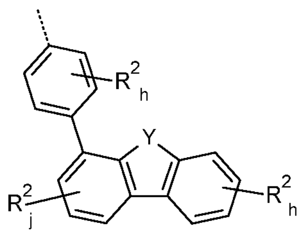
Formel ($R^1$-43)

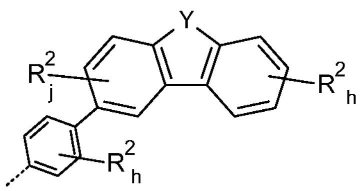
Formel ($R^1$-44)

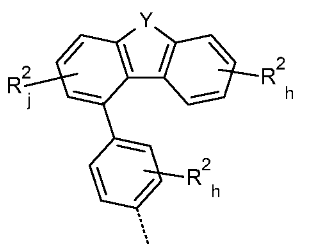
Formel ($R^1$-45)

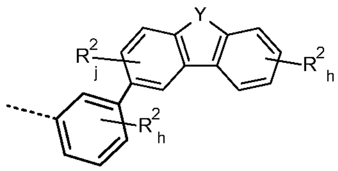

Formel ($R^1$-46)

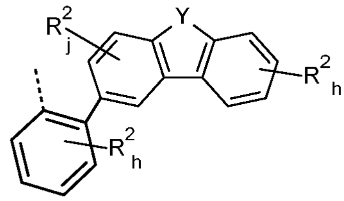

Formel ($R^1$-47)

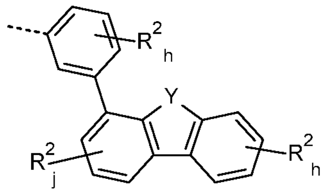

Formel ($R^1$-48)

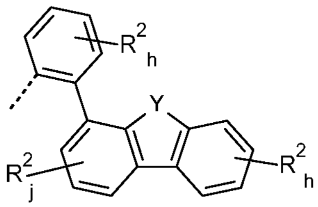

Formel ($R^1$-49)

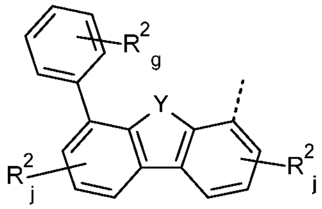

Formel ($R^1$-50)

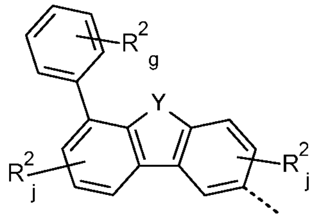

Formel ($R^1$-51)

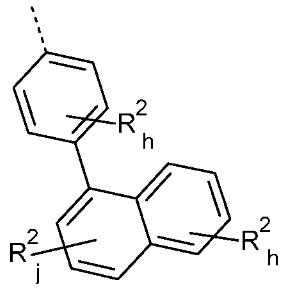

Formel ($R^1$-52)

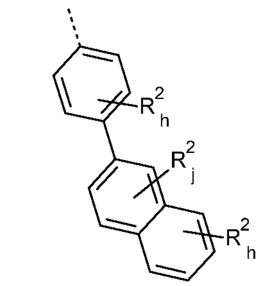

Formel ($R^1$-53)

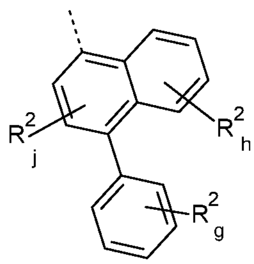

Formel ($R^1$-54)

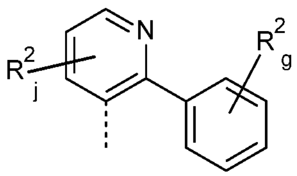

Formel ($R^1$-55)

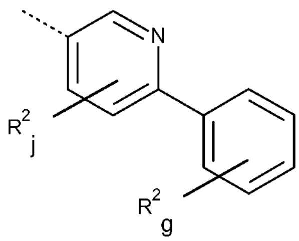

Formel ($R^1$-56)

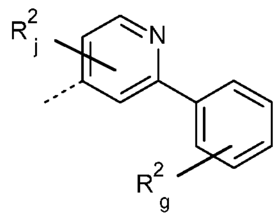

Formel ($R^1$-57)

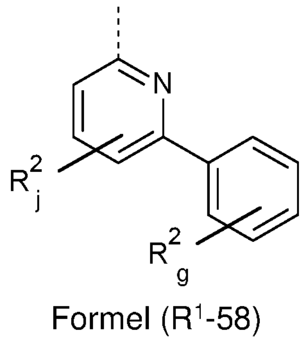

Formel ($R^1$-58)

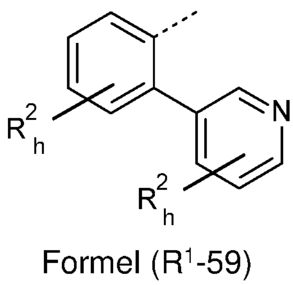

Formel ($R^1$-59)

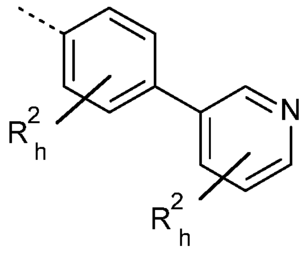

Formel ($R^1$-60)

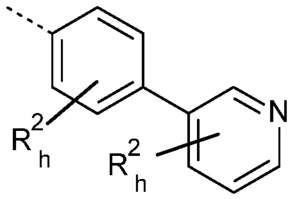
Formel ($R^1$-61)

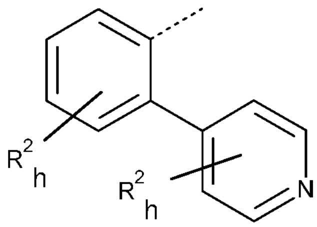
Formel ($R^1$-62)

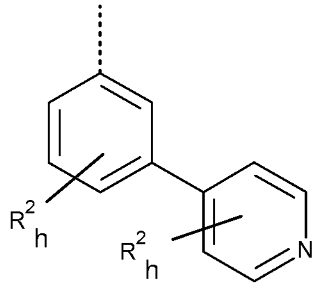
Formel ($R^1$-63)

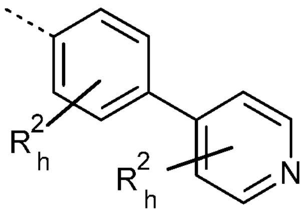
Formel ($R^1$-64)

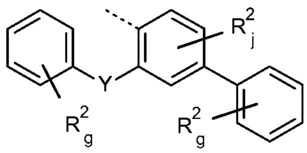
Formel ($R^1$-65)

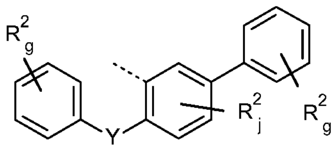
Formel ($R^1$-66)

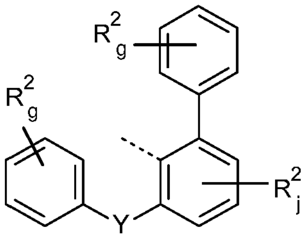
Formel ($R^1$-67)

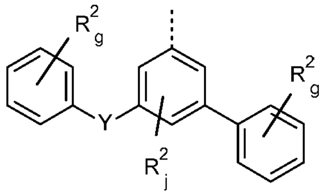
Formel ($R^1$-68)

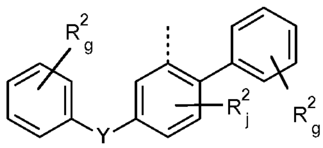
Formel ($R^1$-69)

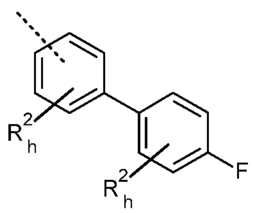
Formel ($R^1$-70)

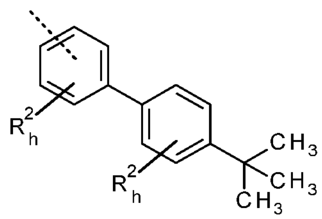
Formel ($R^1$-71)

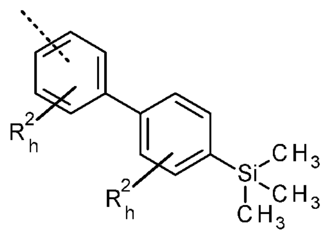
Formel ($R^1$-72)

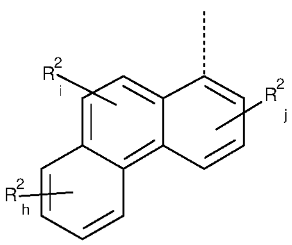
Formel ($R^1$-73)

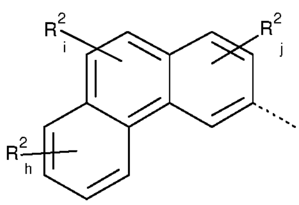
Formel ($R^1$-74)

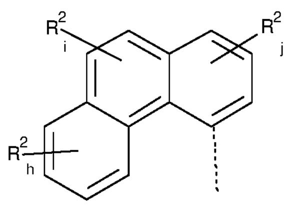
Formel ($R^1$-75)

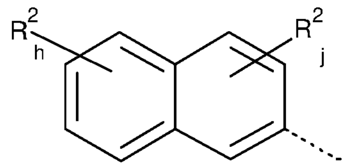

Formel ($R^1$-76)

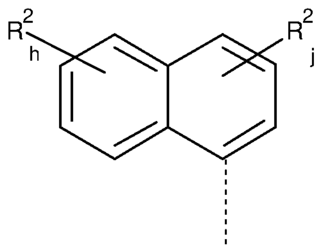

Formel ($R^1$-77)

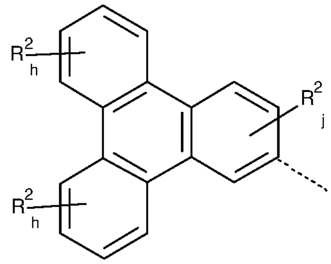

Formel ($R^1$-78)

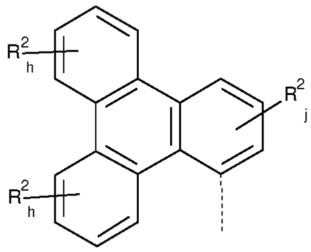

Formel ($R^1$-79)

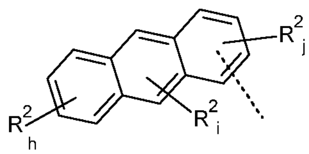

Formel ($R^1$-80)

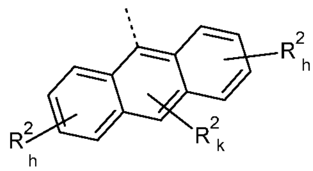

Formel ($R^1$-81)

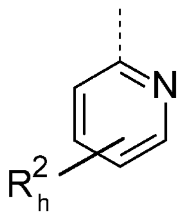

Formel ($R^1$-82)

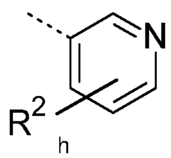

Formel ($R^1$-83)

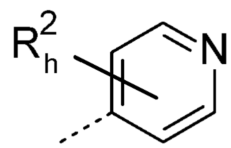

Formel ($R^1$-84)

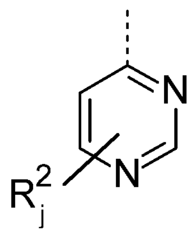

Formel ($R^1$-85)

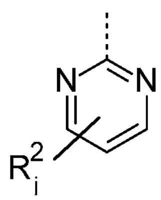

Formel ($R^1$-86)

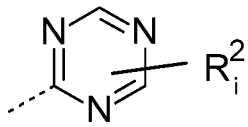

Formel ($R^1$-87)

wobei für die verwendeten Symbole gilt:

- Y ist O, S oder $NR^2$, vorzugsweise O oder S;
- k ist bei jedem Auftreten unabhängig 0 oder 1
- i ist bei jedem Auftreten unabhängig 0, 1 oder 2, bevorzugt 0 oder 1;
- j ist bei jedem Auftreten unabhängig 0, 1, 2 oder 3, vorzugsweise 0, 1 oder 2, besonders bevorzugt 0 oder 1;
- h ist bei jedem Auftreten unabhängig 0, 1, 2, 3 oder 4, vorzugsweise 0, 1 oder 2;
- g ist bei jedem Auftreten unabhängig 0, 1, 2, 3, 4 oder 5, vorzugsweise 0, 1 oder 2;
- $R^2$ kann die zuvor genannte, insbesondere für Formel (Ic), (IIc) oder (IIIc) genannte Bedeutung aufweisen und

die gestrichelte Bindung markiert die Anbindungsposition.

Vorzugsweise kann vorgesehen sein, dass die Summe der Indices k, i, j, h und g in den Strukturen der Formel ($R^1$-1) bis ($R^1$-87) jeweils höchstens 3, vorzugsweise höchstens 2 und besonders bevorzugt höchstens 1 beträgt.

Bevorzugt bilden die Reste $R^2$ in den Formeln ($R^1$-1) bis ($R^1$-87) mit den Ringatomen der Arylgruppe oder

Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

Besonders bevorzugt sind die Reste $Ar^a$ und/oder $Ar^b$ in den Formeln (Ic), (IIc), (IIIc), (IVc), (Vc) und/oder (VIc) ausgewählt aus einer Gruppe gemäß den Formeln ($R^1$-1) bis ($R^1$-48) und ($R^1$-73) bis ($R^1$-87), speziell bevorzugt ($R^1$-1), ($R^1$-38) bis ($R^1$-48) und ($R^1$-73) bis ($R^1$-81). Hierbei gelten die zuvor für die Gruppen der Formeln ($R^1$-1) bis ($R^1$-87) dargelegten Bevorzugungen hinsichtlich der Summe der Indices und der an diese Gruppen gebundenen Reste $R^2$.

Besonders bevorzugt sind die Reste Ar und/oder $Ar^1$ in den Formeln (Ic), (IIc), (IIIc), (IVc), (Vc) und/oder (VIc) ausgewählt aus einer Gruppe gemäß den Formeln ($R^1$-1) bis ($R^1$-54), insbesondere bevorzugt ($R^1$-1) bis ($R^1$-51), speziell bevorzugt ($R^1$-1) bis ($R^1$-37), wobei Reste gemäß ($R^1$-1) ganz besonders bevorzugt sind. Hierbei gelten die zuvor für die Gruppen der Formeln ($R^1$-1) bis ($R^1$-87) dargelegten Bevorzugungen hinsichtlich der Summe der Indices und der an diese Gruppen gebundenen Reste $R^2$.

Bevorzugt kann die Gruppe $L^1$ oder $L^2$ mit den beiden Aryl- oder Heteroarylgruppen, an den die Gruppe $L^1$ oder $L^2$ gemäß Formel (Ic), (IIc), (IIIc), (IVc), (Vc), (VIc) und/oder ($Ar^b$-1) gebunden ist, eine durchgängige Konjugation ausbilden. Eine durchgängige Konjugation der aromatischen beziehungsweise heteroaromatischen Systeme wird ausgebildet sobald direkte Bindungen zwischen benachbarten aromatischen oder heteroaromatischen Ringen gebildet werden. Eine weitere Verknüpfung zwischen den zuvor genannten konjugierten Gruppen, die beispielsweise über ein S-, N- oder O-Atom oder eine Carbonylgruppe erfolgt, schadet einer Konjugation nicht. Bei einem Fluorensystem sind die beiden aromatischen Ringe unmittelbar gebunden, wobei das $sp^3$ hybridisierte Kohlenstoffatom in Position 9 zwar eine Kondensation dieser Ringe unterbindet, jedoch eine Konjugation erfolgen kann, da dieses $sp^3$ hybridisierte Kohlenstoffatom in Position 9 nicht zwingend zwischen den beiden Aryl- oder Heteroarylgruppen liegt. Im Gegensatz hierzu kann bei einer zweiten Spirobifluorenstruktur eine durchgängige Konjugation ausgebildet werden, falls die Verbindung zwischen den beiden Aryl- oder Heteroarylgruppen über die gleiche Phenylgruppe der Spirobifluorenstruktur oder über Phenylgruppen der Spirobifluorenstruktur, die unmittelbar aneinander gebunden sind und in einer Ebene liegen, erfolgt. Falls die Verbindung zwischen den beiden Aryl- oder Heteroarylgruppen über verschiedene Phenylgruppen der zweiten Spirobifluorenstruktur erfolgt, die über das $sp^3$ hybridisierte Kohlenstoffatom in Position 9 verbunden sind, ist die Konjugation unterbrochen.

In einer bevorzugten Ausführungsform der Erfindung steht $L^1$ und/oder $L^2$ für eine Bindung.

In einer weiteren bevorzugten Ausführungsform der Erfindung steht $L^1$ und/oder $L^2$ für ein aromatisches oder heteroaromatisches Ringsystem mit 5 bis 14 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann, bevorzugt aber unsubstituiert ist, wobei $R^1$ die zuvor genannte Bedeutung aufweisen kann. Besonders bevorzugt steht $L^1$ und/oder $L^2$ für ein aromatisches Ringsystem mit 6 bis 10 aromatischen Ringatomen oder ein heteroaromatisches Ringsystem mit 6 bis 13 heteroaromatischen Ringatomen, das jeweils durch einen oder mehrere Reste $R^2$ substituiert sein kann, bevorzugt aber unsubstituiert ist, wobei $R^2$ die zuvor genannte Bedeutung aufweisen kann.

Weiterhin bevorzugt steht das unter anderem in den Strukturen gemäß Formeln (Ic), (IIc), (IIIc), (IVc), (Vc), (VIc) und/oder ($Ar^b$-1) dargelegte Symbol $L^1$ bei jedem Auftreten für einen Aryl- oder Heteroarylrest mit 5 bis 24 Ringatomen, oder das dargelegte Symbol $L^1$ und/oder $L^2$ gleich oder verschieden bei jedem Auftreten für einen Aryl- oder Heteroarylrest mit vorzugsweise 6 bis 13 Ringatomen, besonders bevorzugt 6 bis 10 Ringatomen, so dass eine aromatische oder heteroaromatische Gruppe eines aromatischen oder heteroaromatische Ringsystems direkt, d.h. über ein Atom der aromatischen oder heteroaromatische Gruppe, an das jeweilige Atom der weiteren Gruppe gebunden ist.

Weiterhin kann vorgesehen sein, dass die unter anderem in den Strukturen gemäß Formeln (Ic), (IIc), (IIIc), (IVc), (Vc), (VIc) und/oder ($Ar^b$-1) dargelegte Gruppe $L^1$ und/oder $L^2$ ein aromatisches Ringsystem mit höchstens zwei kondensierten aromatischen und/oder heteroaromatischen Ringen, vorzugsweise kein kondensiertes aromatisches oder heteroaromatisches Ringsystem umfasst. Demgemäß sind Naphthylstrukturen gegenüber Antracenstrukturen bevorzugt. Weiterhin sind Fluorenyl-, Spirobifluorenyl-, Dibenzofuranyl- und/oder Dibenzothienyl-Strukturen gegenüber Naphthylstrukturen bevorzugt.

Besonders bevorzugt sind Strukturen, die keine Kondensation aufweisen, wie beispielsweise Phenyl-, Biphenyl-, Terphenyl- und/oder Quaterphenyl-Strukturen.

Beispiele für geeignete aromatische oder heteroaromatische Ringsysteme $L^1$ und/oder $L^2$ , bezüglich $L^2$ nur insoweit sie 5 bis 14 Ringatome haben, sind ausgewählt aus der Gruppe bestehend aus ortho-, meta- oder para-Phenylen, ortho-, meta- oder para-Biphenylen, Terphenylen, insbesondere verzweigtes Terphenylen, Quaterphenylen, insbesondere verzweigtes Quaterphenylen, Fluorenylen, Spirobifluorenylen, Dibenzofuranylen, Dibenzothienylen und Carbazolylen, die jeweils durch einen oder mehrere Reste $R^2$ substituiert sein können, bevorzugt aber unsubstituiert sind.

Ferner kann vorgesehen sein, dass die unter anderem in den Strukturen gemäß Formeln (Ic), (IIc), (IIIc), (IVc), (Vc), (VIc) und/oder ($Ar^b$-1) dargelegte Gruppe $L^1$ und/oder $L^2$ höchstens 1 Stickstoffatom, bevorzugt höchstens 2 Heteroatome, insbesondere bevorzugt höchstens ein Heteroatom und besonders bevorzugt kein Heteroatom aufweist.

Bevorzugt sind Verbindungen umfassend Strukturen der Formeln (Ic), (IIc), (IIIc), (IVc), (Vc) und/oder (VIc), in

denen die Gruppe $L^1$ und/oder $L^2$ gemäß Formel (Ic), (IIc), (IIIc), (IVc), (Vc), (VIc) und/oder ($Ar^b$-1) für eine Gruppe steht, die ausgewählt ist aus den Formeln ($L^1$-1) bis ($L^1$-108), wobei für $L^2$ nur diejenigen von ($L^1$-1) bis (L-108) in Frage kommen, die 5 bis 14 Ringatome besitzen,

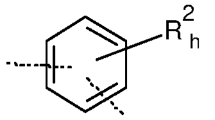

Formel ($L^1$-1)

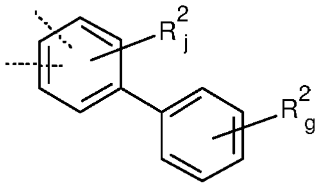

Formel ($L^1$-2)

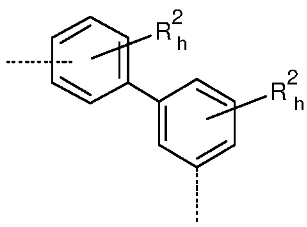

Formel ($L^1$-3)

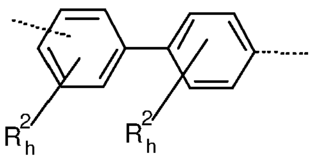

Formel ($L^1$-4)

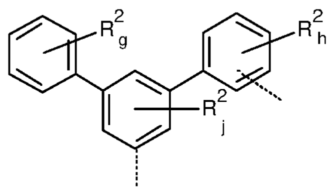

Formel ($L^1$-5)

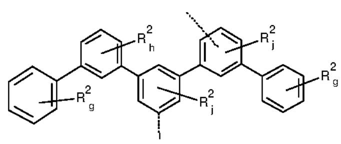

Formel ($L^1$-6)

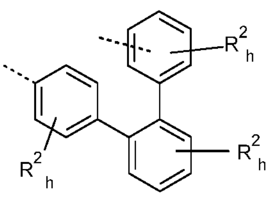

Formel ($L^1$-7)

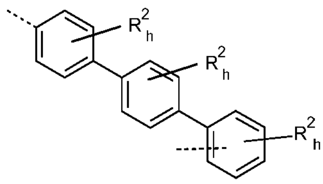

Formel ($L^1$-8)

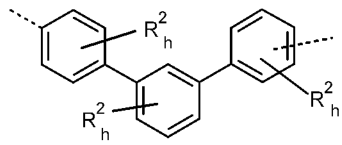

Formel ($L^1$-9)

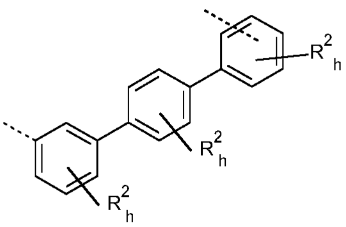

Formel ($L^1$-10)

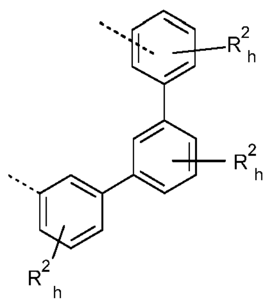

Formel ($L^1$-11)

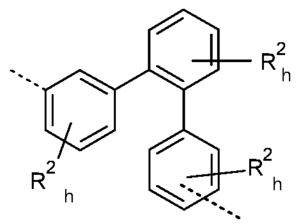

Formel ($L^1$-12)

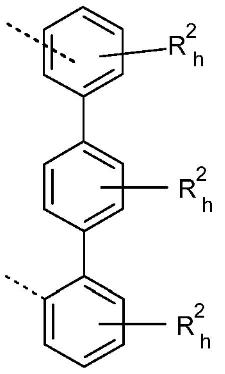

Formel ($L^1$-13)

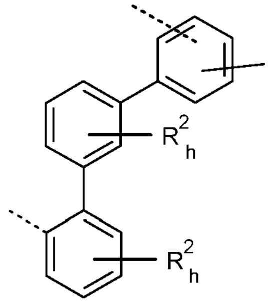

Formel ($L^1$-14)

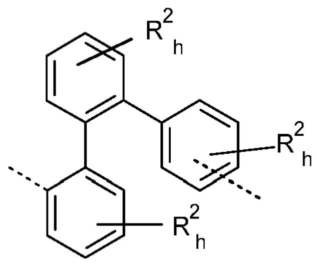

Formel ($L^1$-15)

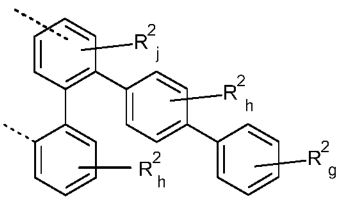

Formel ($L^1$-16)

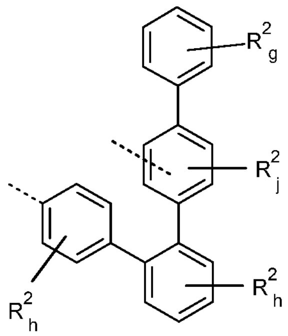

Formel ($L^1$-17)

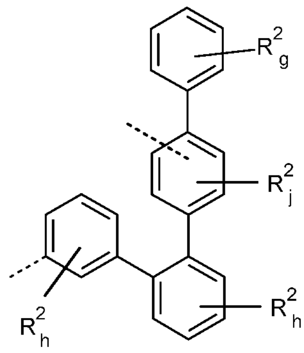

Formel ($L^1$-18)

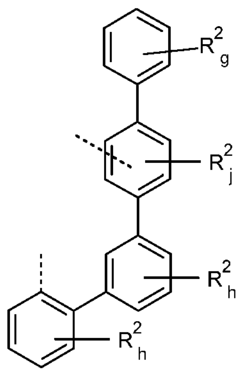

Formel ($L^1$-19)

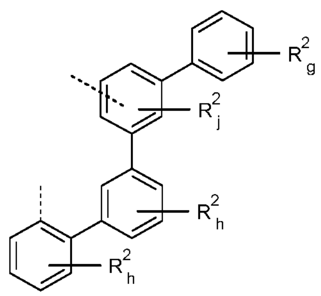

Formel ($L^1$-20)

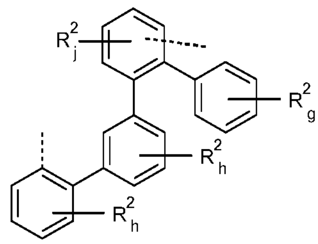

Formel ($L^1$-21)

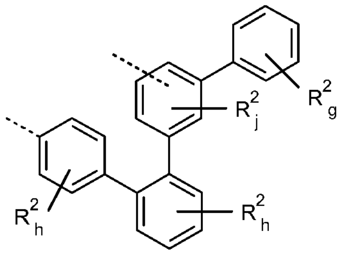

Formel ($L^1$-22)

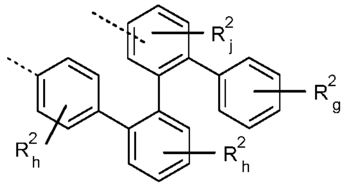

Formel ($L^1$-23)

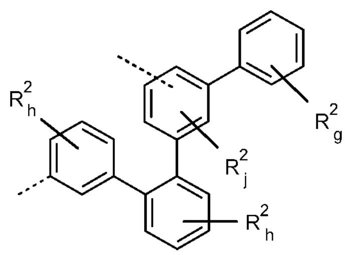

Formel ($L^1$-24)

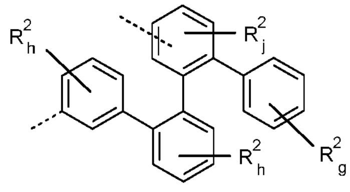

Formel ($L^1$-25)

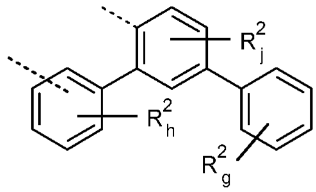

Formel ($L^1$-26)

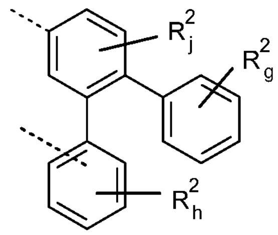

Formel ($L^1$-27)

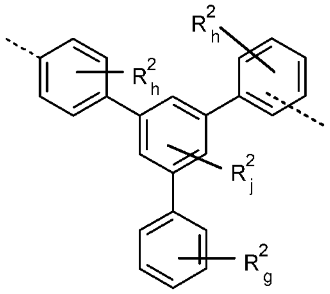

Formel ($L^1$-28)

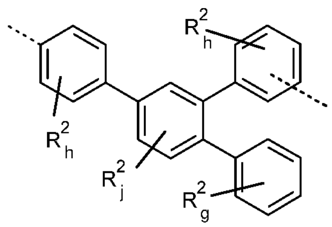

Formel ($L^1$-29)

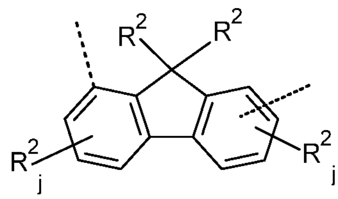

Formel ($L^1$-30)

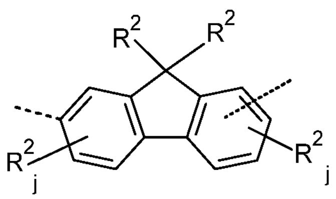

Formel ($L^1$-31)

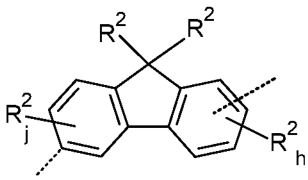

Formel ($L^1$-32)

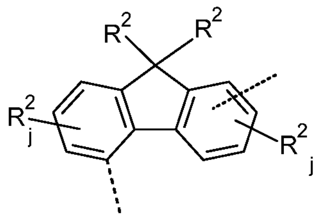

Formel ($L^1$-33)

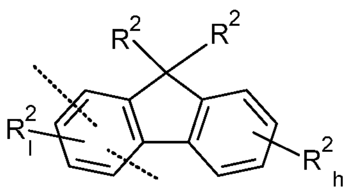

Formel ($L^1$-34)

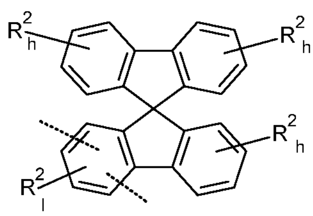

Formel ($L^1$-35)

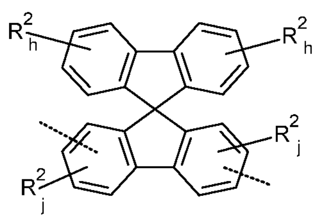

Formel ($L^1$-36)

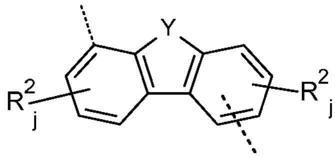

Formel ($L^1$-37)

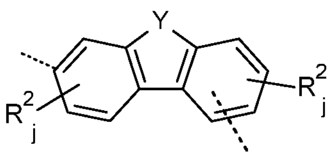

Formel ($L^1$-38)

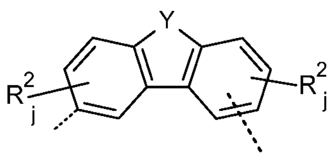

Formel ($L^1$-39)

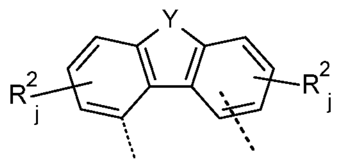
Formel (L1-40)

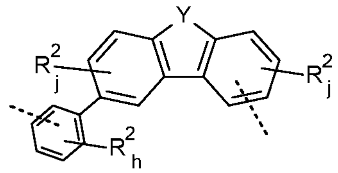
Formel (L1-41)

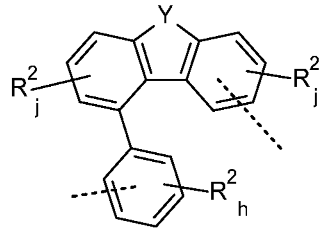
Formel (L1-42)

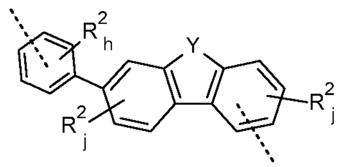
Formel (L1-43)

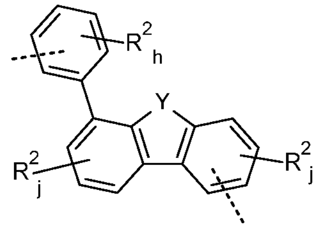
Formel (L1-44)

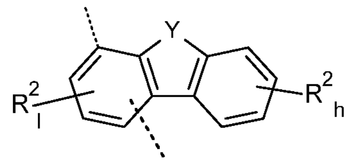
Formel (L1-45)

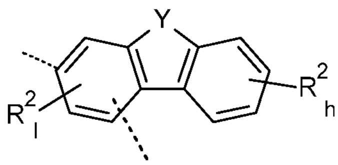
Formel (L1-46)

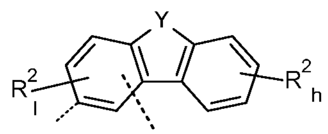
Formel (L1-47)

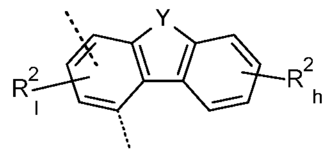
Formel (L1-48)

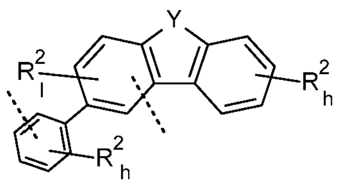
Formel (L1-49)

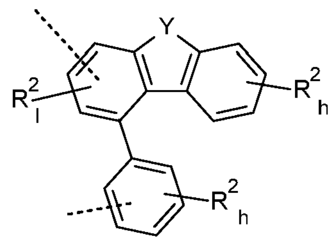
Formel (L1-50)

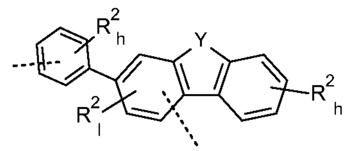
Formel (L1-51)

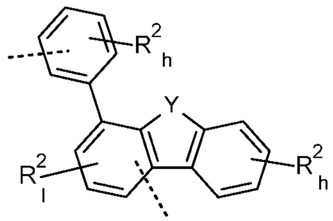
Formel (L1-52)

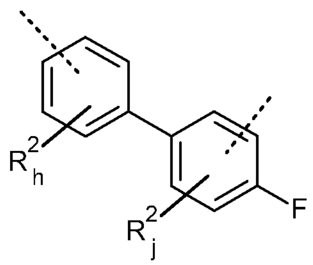
Formel (L1-53)

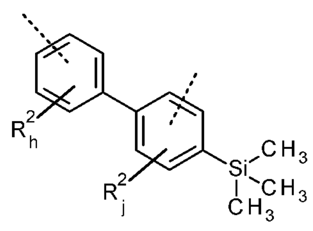
Formel (L1-54)

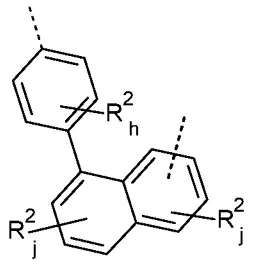
Formel ($L^1$-55)

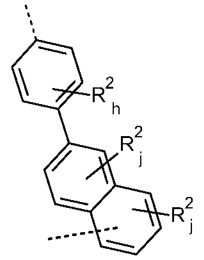
Formel ($L^1$-56)

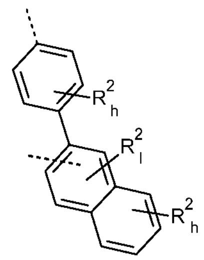
Formel ($L^1$-57)

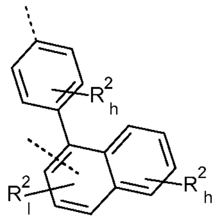
Formel ($L^1$-58)

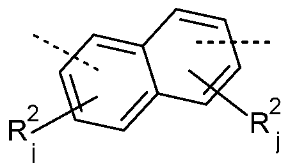
Formel ($L^1$-59)

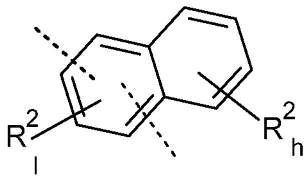
Formel ($L^1$-60)

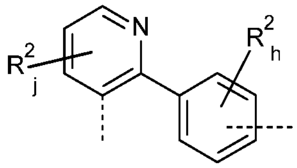
Formel ($L^1$-61)

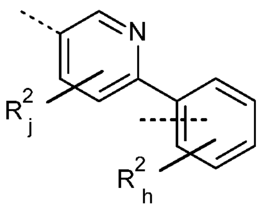
Formel ($L^1$-62)

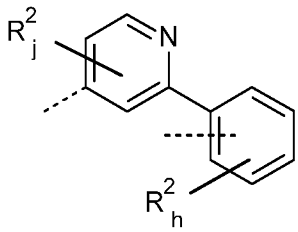
Formel ($L^1$-63)

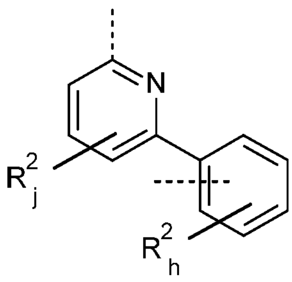
Formel ($L^1$-64)

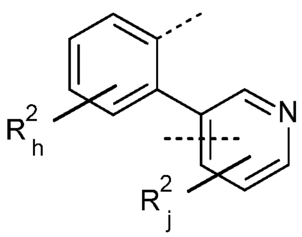
Formel ($L^1$-65)

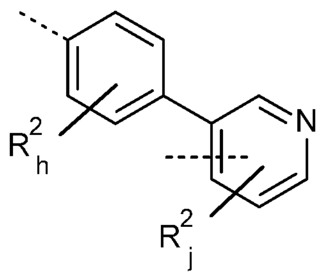
Formel ($L^1$-66)

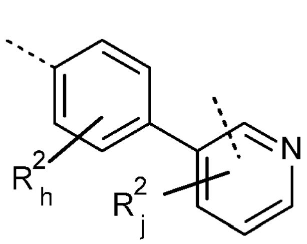
Formel ($L^1$-67)

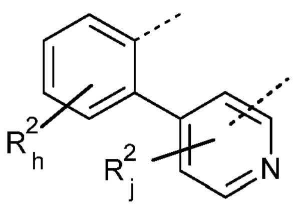
Formel ($L^1$-68)

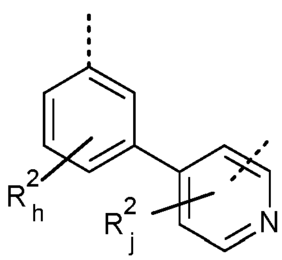
Formel ($L^1$-69)

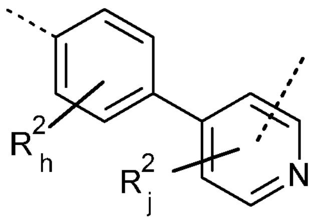

Formel ($L^1$-70)

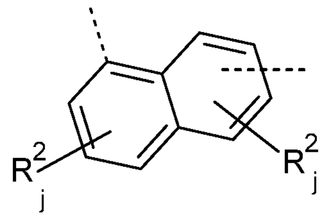

Formel ($L^1$-71)

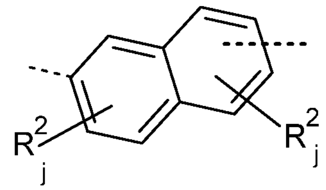

Formel ($L^1$-72)

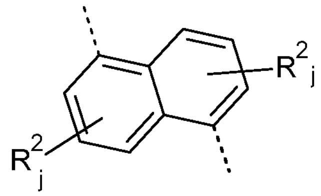

Formel ($L^1$-73)

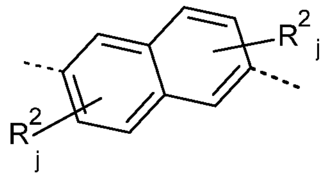

Formel ($L^1$-74)

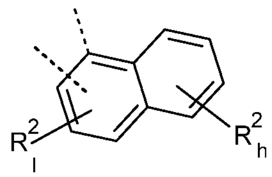

Formel ($L^1$-75)

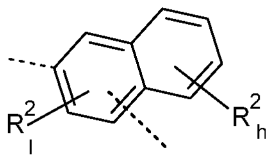

Formel ($L^1$-76)

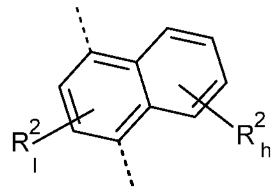

Formel ($L^1$-77)

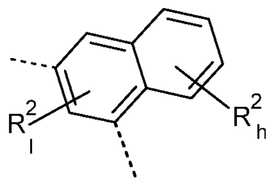

Formel ($L^1$-78)

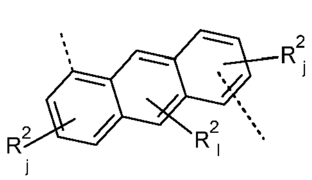

Formel ($L^1$-79)

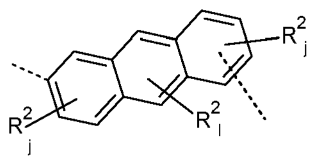

Formel ($L^1$-80)

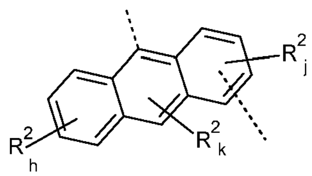

Formel ($L^1$-81)

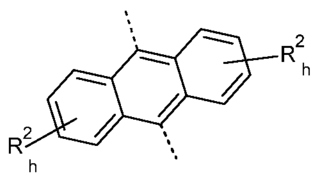

Formel ($L^1$-82)

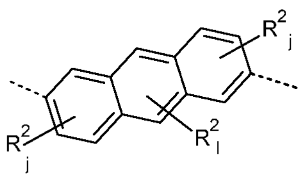

Formel ($L^1$-83)

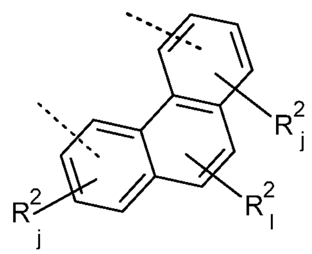

Formel ($L^1$-84)

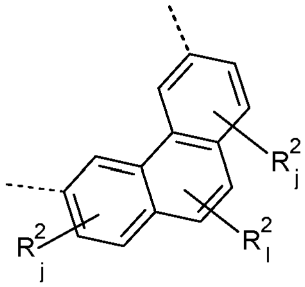
Formel ($L^1$-85)

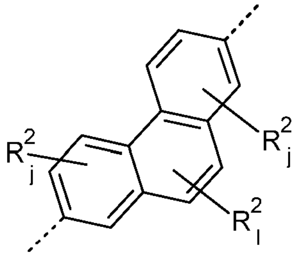
Formel ($L^1$-86)

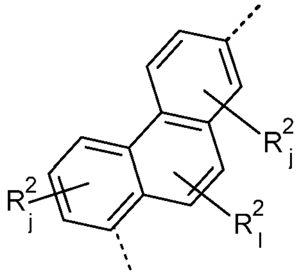
Formel ($L^1$-87)

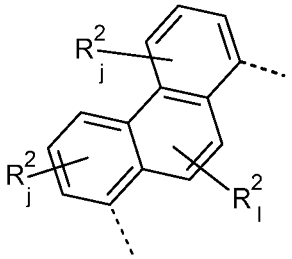
Formel ($L^1$-88)

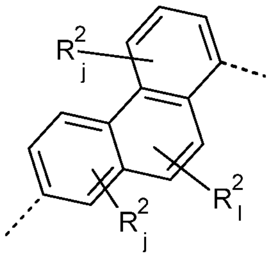
Formel ($L^1$-89)

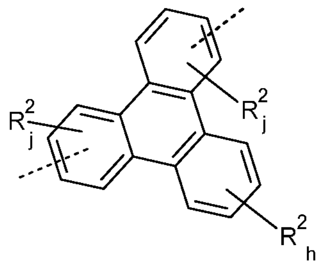
Formel ($L^1$-90)

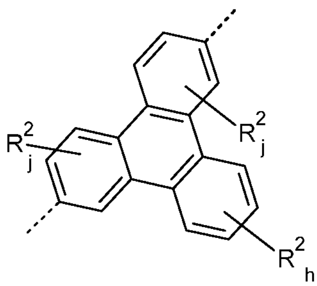
Formel ($L^1$-91)

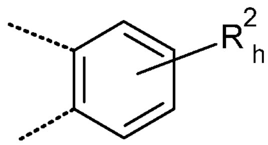
Formel ($L^1$-92)

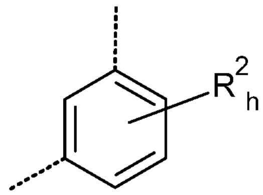
Formel ($L^1$-93)

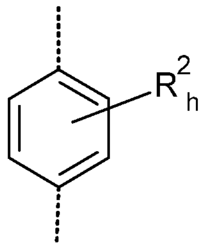
Formel ($L^1$-94)

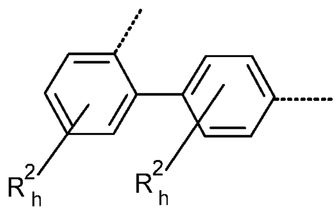
Formel ($L^1$-95)

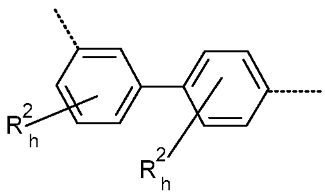
Formel ($L^1$-96)

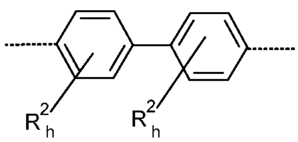
Formel ($L^1$-97)

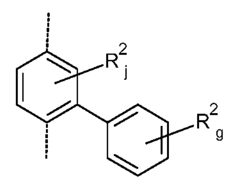
Formel ($L^1$-98)

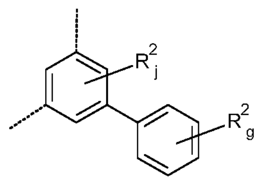
Formel ($L^1$-99)

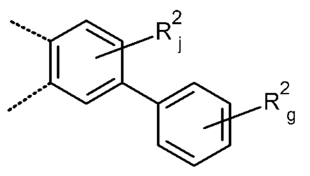

Formel ($L^1$-100)

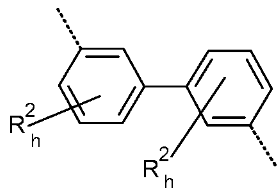

Formel ($L^1$-101)

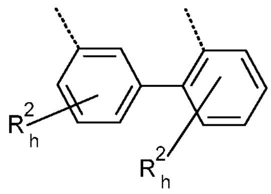

Formel ($L^1$-102)

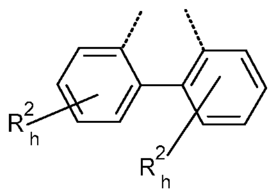

Formel ($L^1$-103)

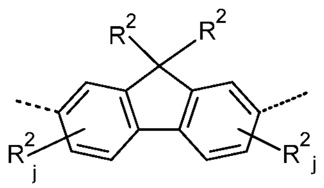

Formel ($L^1$-104)

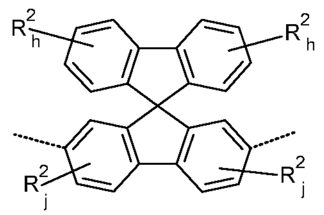

Formel ($L^1$-105)

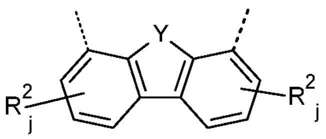

Formel ($L^1$-106)

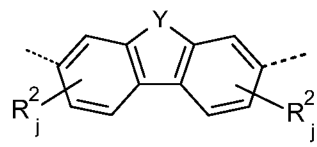

Formel ($L^1$-107)

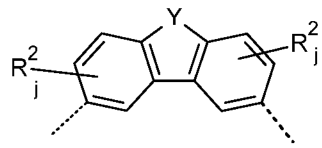

Formel ($L^1$-108)

wobei die gestrichelten Bindungen jeweils die Anbindungspositionen markieren, der Index k 0 oder 1 ist, der Index l 0, 1 oder 2 ist, der Index j bei jedem Auftreten unabhängig 0, 1, 2 oder 3 ist; der Index h bei jedem Auftreten unabhängig 0, 1, 2, 3 oder 4 ist, der Index g 0, 1, 2, 3, 4 oder 5 ist; das Symbol Y O, S oder $NR^2$, vorzugsweise O oder S ist; und das Symbol $R^2$ die zuvor, insbesondere für Formel (Ic), (IIc) oder (IIIc) genannte Bedeutung aufweist.

Vorzugsweise kann vorgesehen sein, dass die Summe der Indices k, l, g, h und j in den Strukturen der Formel ($L^1$-1) bis ($L^1$-108) jeweils höchstens 3, vorzugsweise höchstens 2 und besonders bevorzugt höchstens 1 beträgt.

Vorzugsweise kann vorgesehen sein, dass die Gruppe $L^1$ in Strukturen der Formeln (Ic), (IIc), (IIIc), (IVc), (Vc) und/oder (VIc) keine Carbazol-Gruppe umfasst, wobei dies Substituenten $R^1$, $R^2$ und $R^3$, die an die Gruppe $L^1$ gebunden sein können, einschließt. Vorzugsweise umfasst die Gruppe $L^2$ in Strukturen der Formeln ($Ar^b$-1) keine Carbazol-Gruppe, wobei dies Substituenten $R^1$, $R^2$ und $R^3$, die an die Gruppe $L^1$ gebunden sein können

Ferner kann vorgesehen sein, dass eine der Gruppen $L^1$ und/oder $L^2$ in Strukturen der Formeln (Ic), (IIc), (IIIc), (IVc), (Vc), (VIc) und/oder ($Ar^b$-1), höchstens 5, vorzugsweise höchstens 3 und besonders bevorzugt höchstens 1 Heteroatom aufweist, wobei dies Substituenten $R^1$, $R^2$ und $R^3$ einschließt, die an diese Gruppen gebunden sein können.

Weiterhin kann vorgesehen sein, dass die Gruppen $L^1$ und/oder $L^2$ insgesamt in Strukturen der Formeln (Ic), (IIc), (IIIc), (IVc), (Vc), (VIc) und/oder ($Ar^b$-1), höchstens 5, vorzugsweise höchstens 3 und besonders bevorzugt höchstens 1 Heteroatom aufweist, wobei dies Substituenten $R^1$, $R^2$ und $R^3$ einschließt, die an diese Gruppen gebunden sein können

Bevorzugte erfindungsgemäße Verbindungen umfassen eine Gruppe $L^1$, die ausgewählt ist aus einer der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103). Mit Vorteil kann die Summe der Indices k, l, g, h und j in den Strukturen der Formeln ($L^1$-1) bis ($L^1$-78) und/oder ($L^1$-92) bis ($L^1$-108), bevorzugt der Formel ($L^1$-1) bis ($L^1$-54) und/oder ($L^1$-92) bis ($L^1$-108), speziell bevorzugt der Formel ($L^1$-1) bis ($L^1$-29) und/oder ($L^1$-92) bis ($L^1$-103) jeweils höchstens 3, vorzugsweise höchstens 2 und besonders bevorzugt höchstens 1 betragen.

Bevorzugt bilden die Reste $R^2$ in den Formeln ($L^1$-1) bis ($L^1$-108) mit den Ringatomen der Arylgruppe oder Heteroarylgruppe, an die die Reste $R^2$ gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten $R^3$ ein, die an die Reste $R^2$ gebunden sein können.

In einer weiteren bevorzugten Ausführungsform der Erfindung ist $R^2$, beispielsweise bei einer Struktur gemäß Formel (Ic), (IIc) oder (IIIc) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit

5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren bevorzugten Ausführungsform der Erfindung ist $R^3$, beispielsweise bei einer Struktur gemäß Formel (Ic), (IIc) oder (IIIc) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Wenn die erfindungsgemäße Verbindung mit aromatischen oder heteroaromatischen Gruppen $R^1$ bzw. $R^2$ substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Besonders bevorzugt sind erfindungsgemäße Verbindungen, welche die folgenden Eigenschaften aufweisen:

| $Ar^a$ und $Ar^b$ | $L^1$ | Position $L^1$ an Phenylring mit $Y^1$ | vorzugsweise | Index n für $R^1$ |
|---|---|---|---|---|
| $R^1$-1 bis $R^1$-87 | Bindung oder $L^1$-1 | (Ic), (IIc), (IIIc) | (IVc), (Vc), (VIc) | 0, 1, 2 |
| $R^1$-1 | $L^1$-94 | (Ic), (IIc), (IIIc) | (IVc), (Vc), (VIc) | 0, 1 |
| $R^1$-1 | Bindung | (Ic), (IIc), (IIIc) | (IVc), (Vc), (VIc) | 0, 1 |

Besonders bevorzugt sind Verbindungen, die eine Struktur der Formel (W-1) aufweisen, wobei $Y^2$ eine Gruppe der Formel $C(R^2)_2$ ist und die Verbindungen die folgenden Eigenschaften aufweisen:

| $Ar^a$ und $Ar^b$ | $L^1$ | Position $L^1$ an Phenylring mit $Y^1$ | vorzugsweise | Index n für $R^1$ |
|---|---|---|---|---|
| $R^1$-1 bis $R^1$-87 | Bindung oder $L^1$-1 | (Ic), (IIc), (IIIc) | (IVc), (Vc), (VIc) | 0, 1, 2 |
| $R^1$-1 | $L^1$-94 | (Ic), (IIc), (IIIc) | (IVc), (Vc), (VIc) | 0, 1 |
| $R^1$-1 | Bindung | (Ic), (IIc), (IIIc) | (IVc), (Vc), (VIc) | 0, 1 |

Der Index g in Formel $R^1$-1 in zuvor genannten Tabellen ist vorzugsweise 0, 1, 2 oder 3, besonders bevorzugt 0 oder 1, speziell bevorzugt 0; der Index h in Formel $L^1$-1 beziehungsweise $L^1$-94 in zuvor genannten Tabellen ist vorzugsweise 0, 1, 2 oder 3, besonders bevorzugt 0 oder 1, speziell bevorzugt 0.

In den zuvor dargelegten Tabellen bedeutet die Zuordnung $Ar^a$ und $Ar^b$ ist $R^1$-1 bis $R^1$-87, dass sowohl die Gruppe $Ar^a$ als auch die Gruppe $Ar^b$ ausgewählt ist aus Resten der zuvor dargelegten Formeln $R^1$-1 bis $R^1$-87, vorzugsweise $R^1$-1. Die Zuordnung $L^1$ ist eine Bindung oder $L^1$-1 bedeutet, dass die Gruppe $L^1$ in den zuvor dargelegten Formeln (Ic), (IIc), (IIIc), (IVc), (Vc) und/oder (VIc) jeweils eine Bindung oder einen Rest der zuvor dargelegten Formel $L^1$-1, vorzugsweise $L^1$-94 darstellt. Die Zuordnung, dass die Position $L^1$ an Phenylring mit $Y^1$ vorzugsweise der Formel (Ic), (IIc), (IIIc) entspricht, bedeutet, dass die Gruppe $L^1$ in para-Stellung zur Gruppe $Y^1$ steht, wie dies in Formel (Ic), (IIc), (IIIc) dargestellt ist. Die Zuordnung, dass der Index n für $R^1$ 0, 1, 2 ist, bedeutet, dass in den zuvor dargelegten Formeln (Ic), (IIc), (IIIc), (IVc), (Vc) und/oder (VIc)der Index n jeweils 0, 1 oder 2, vorzugsweise 0 oder 1 und speziell bevorzugt 0 ist.

Beispiele für geeignete erfindungsgemäße Verbindungen sind die nachstehend gezeigten Strukturen gemäß den folgenden Formeln 1 bis 13, 16 bis 46, 49, 511 und 52 bis 82:

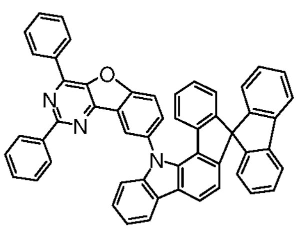

Formel 1

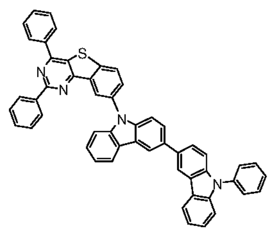

Formel 2

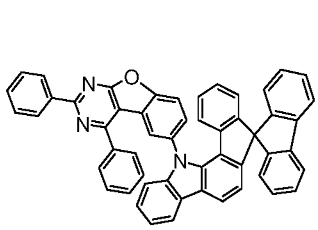

Formel 3

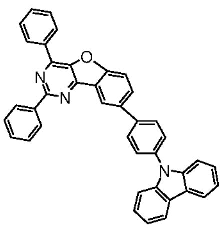

Formel 4

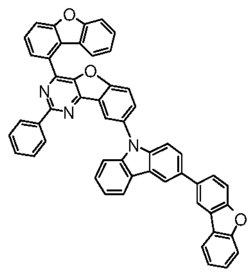

Formel 5

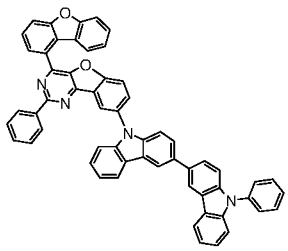

Formel 6

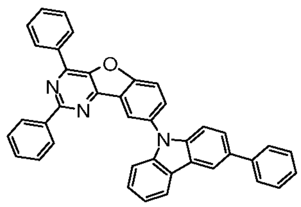

Formel 7

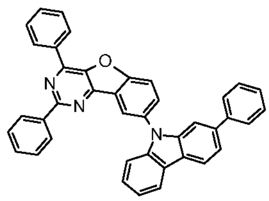

Formel 8

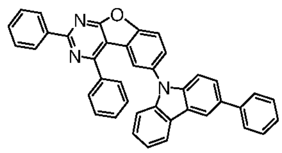

Formel 9

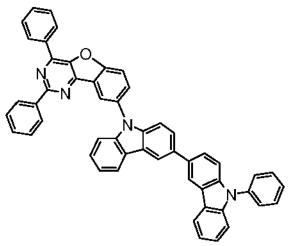

Formel 10

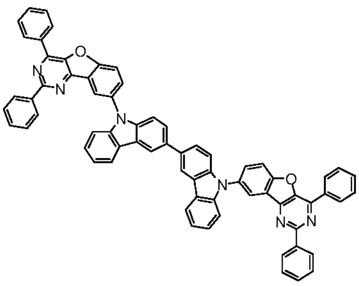

Formel 11

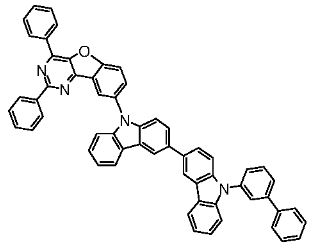

Formel 12

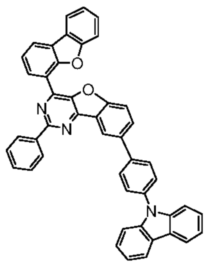

Formel 13

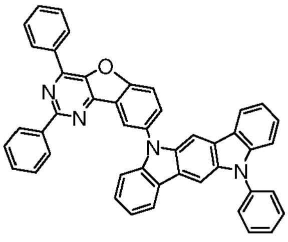
Formel 16

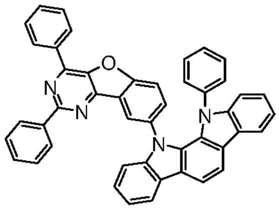
Formel 17

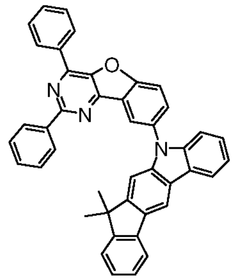
Formel 18

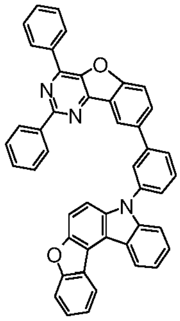
Formel 19

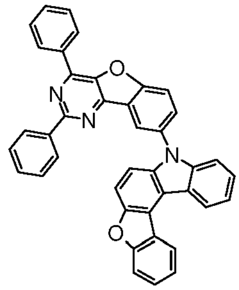
Formel 20

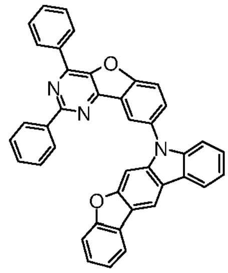
Formel 21

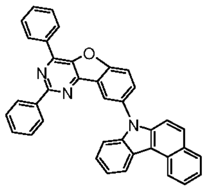
Formel 22

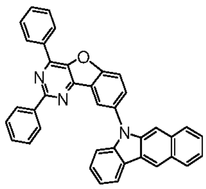
Formel 23

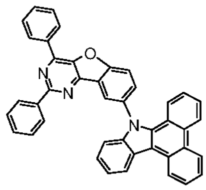
Formel 24

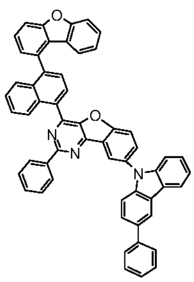
Formel 25

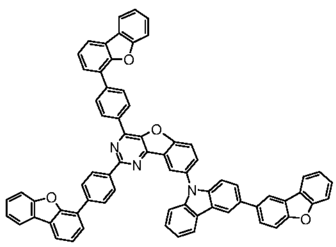
Formel 26

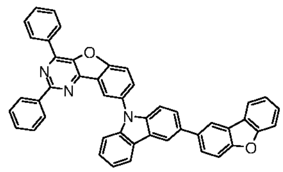
Formel 27

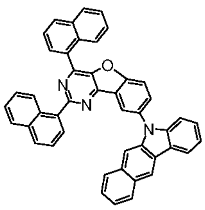
Formel 28

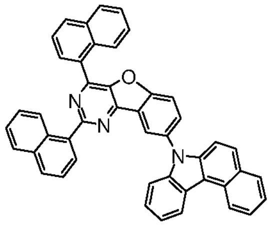
Formel 29

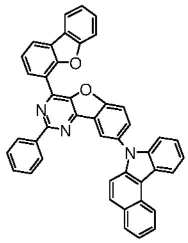
Formel 30

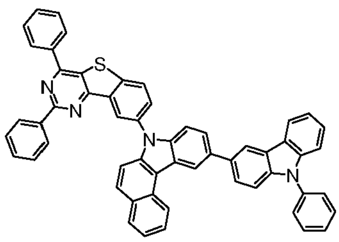

Formel 31

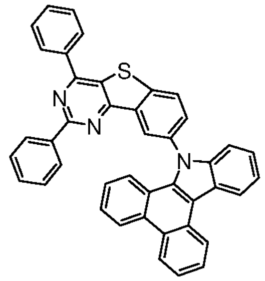

Formel 32

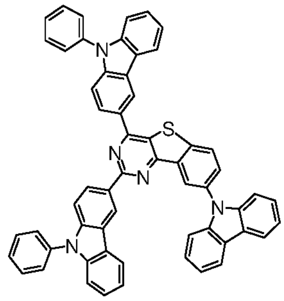

Formel 33

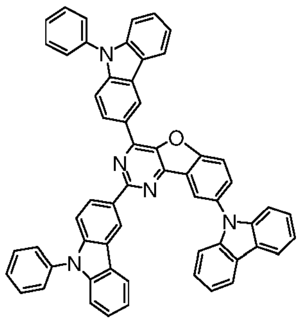

Formel 34

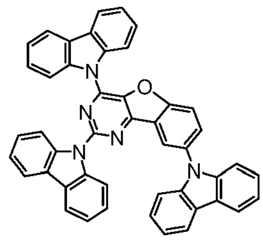

Formel 35

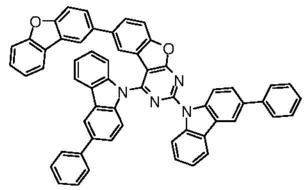

Formel 36

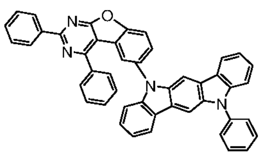

Formel 37

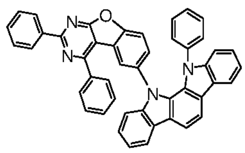

Formel 38

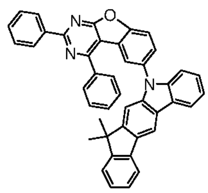

Formel 39

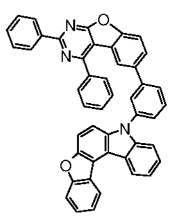

Formel 40

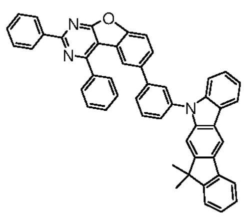

Formel 41

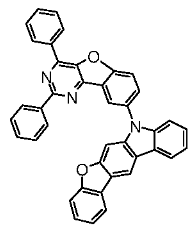

Formel 42

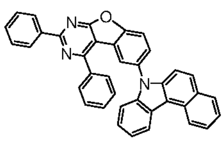

Formel 43

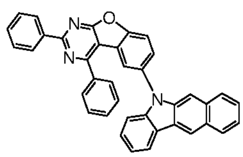

Formel 44

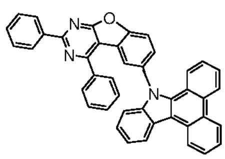

Formel 45

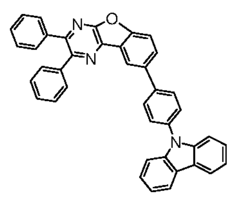

Formel 46

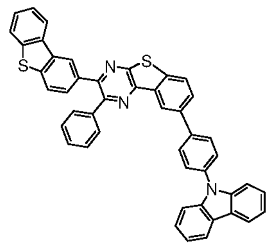

Formel 49

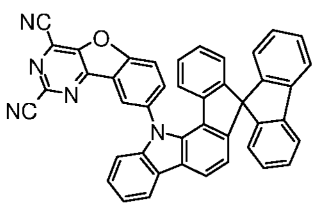

Formel 511

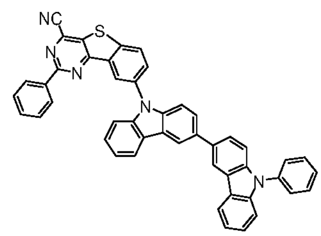

Formel 52

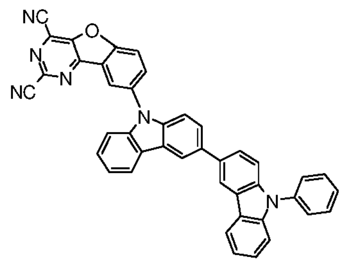

Formel 53

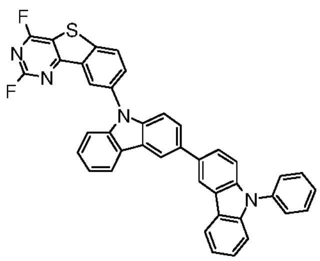

Formel 54

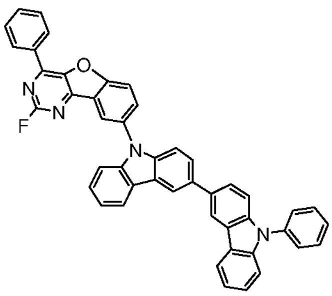

Formel 55

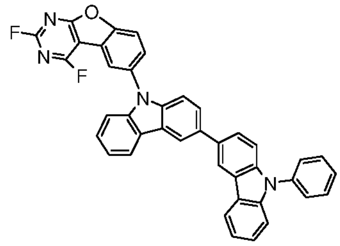

Formel 56

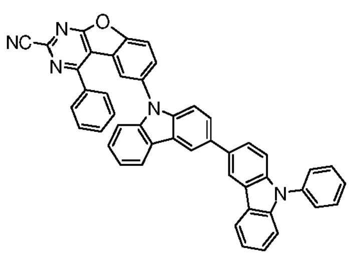

Formel 57

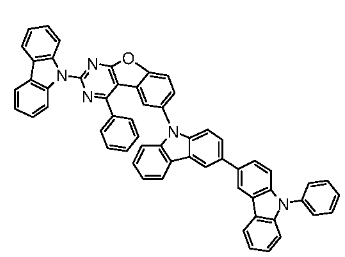

Formel 58

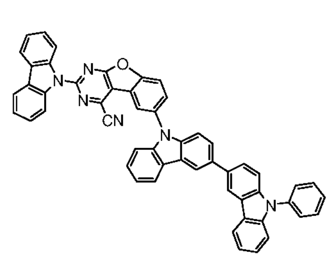

Formel 59

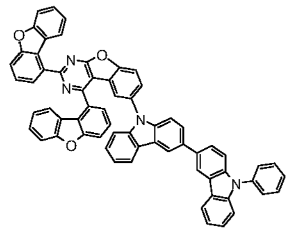

Formel 57

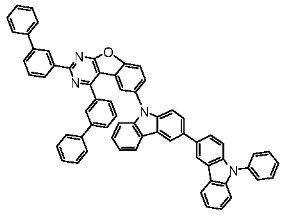

Formel 58

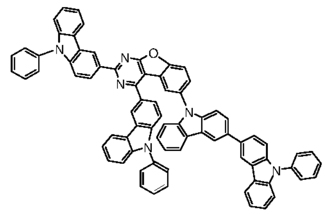

Formel 59

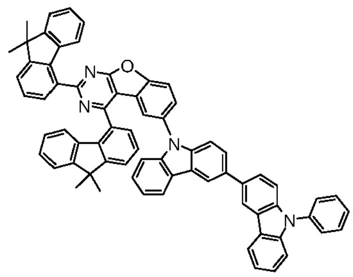

Formel 60

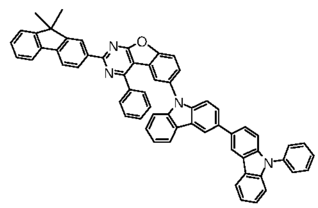

Formel 61

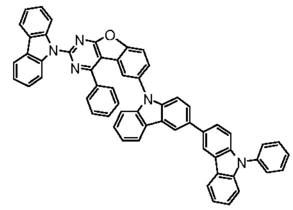

Formel 62

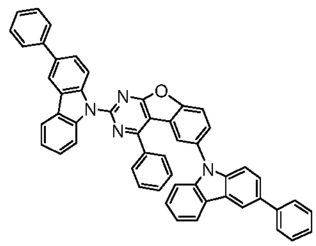

Formel 63

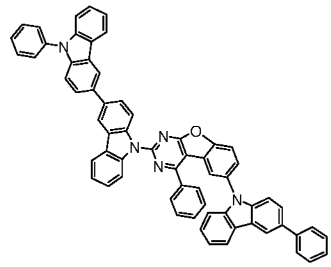

Formel 64

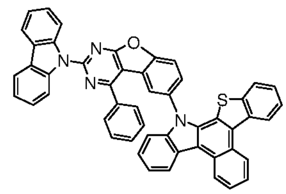

Formel 65

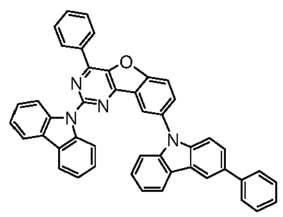

Formel 65

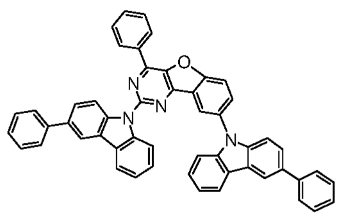

Formel 66

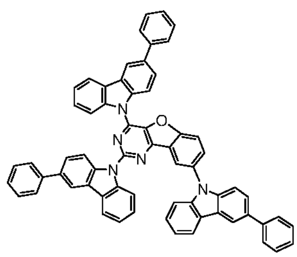

Formel 67

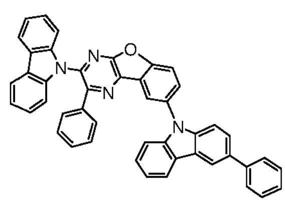

Formel 68

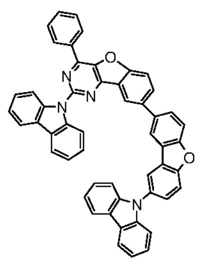

Formel 69

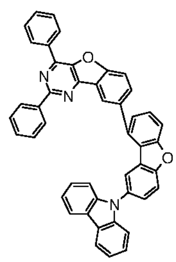

Formel 70

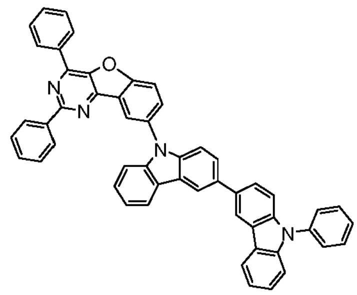

Formel 71

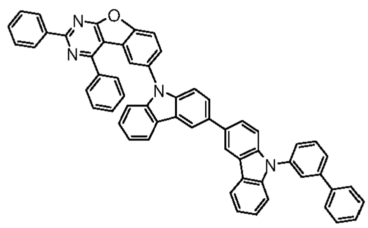

Formel 72

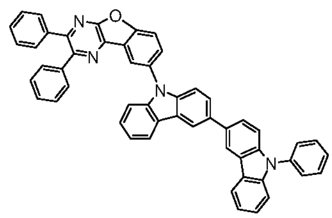

Formel 73

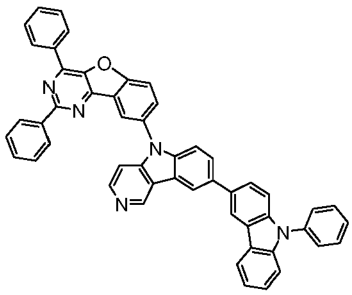

Formel 74

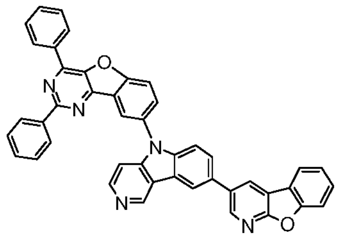

Formel 75

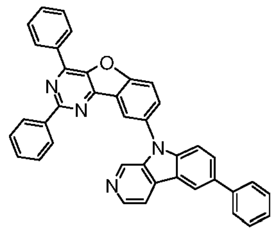

Formel 76

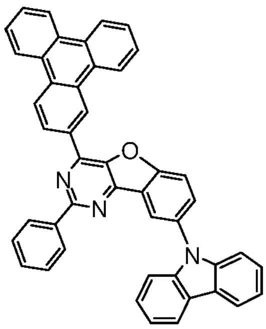

Formel (77)

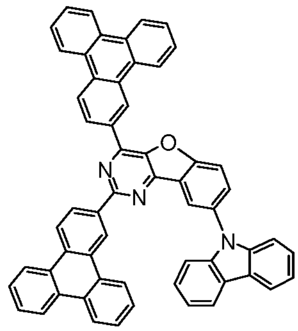

Formel (78)

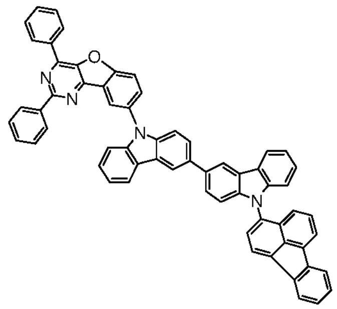

Formel (79)

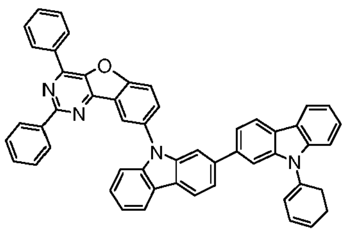

Formel (80)

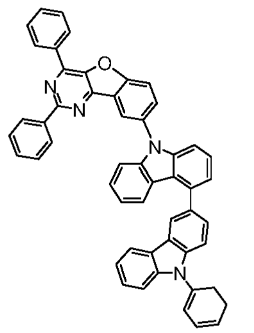

Formel (81)

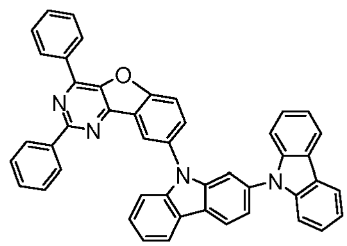

Formel (82)

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (Ic), (IIc) und/oder (IIIc), bei dem in einer Kupplungsreaktion eine Verbindung, umfassend mindestens eine Diazadibenzofuran- oder Diazadibenzothiophen-Gruppe, mit einer Gruppe, umfassend mindestens einen Carbazol-Rest, verbunden wird.

Geeignete Verbindungen mit einer Diazadibenzofuran- oder Diazadibenzothiophen-Gruppe können vielfach kommerziell erhalten werden, wobei die in den Beispielen dargelegten Ausgangsverbindungen durch bekannte Verfahren erhältlich sind, so dass hierauf verwiesen wird.

Diese Verbindungen können durch bekannte Kupplungsreaktionen mit weiteren Arylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

Synthese Schema 1

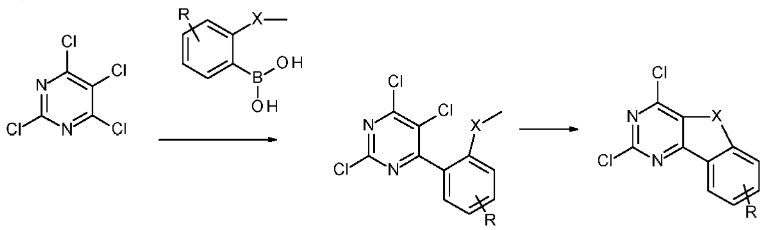

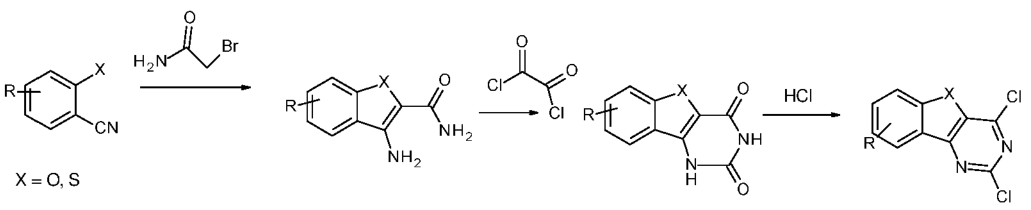

Synthese Schema 2

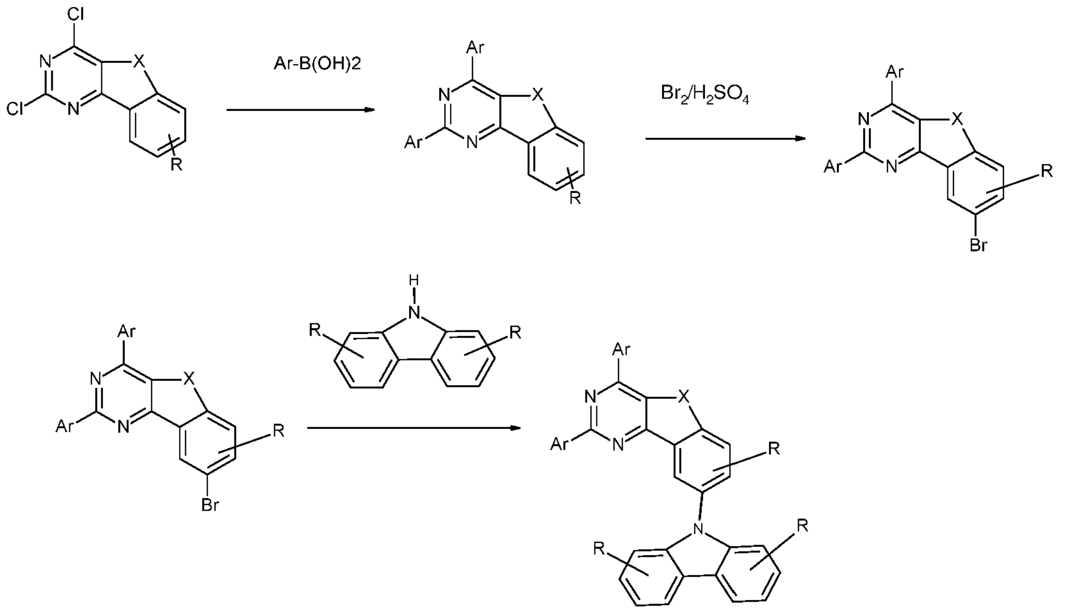


Synthese Schema 3

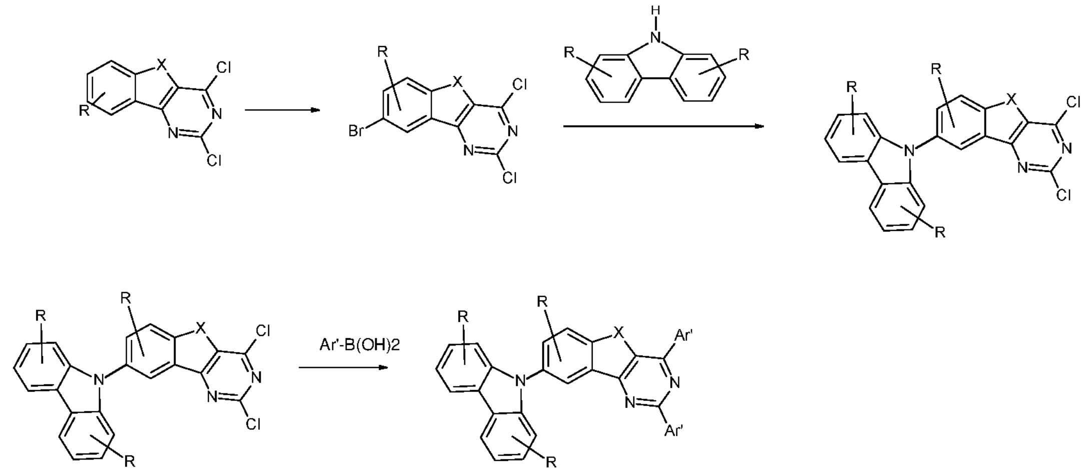

Synthese Schema 4

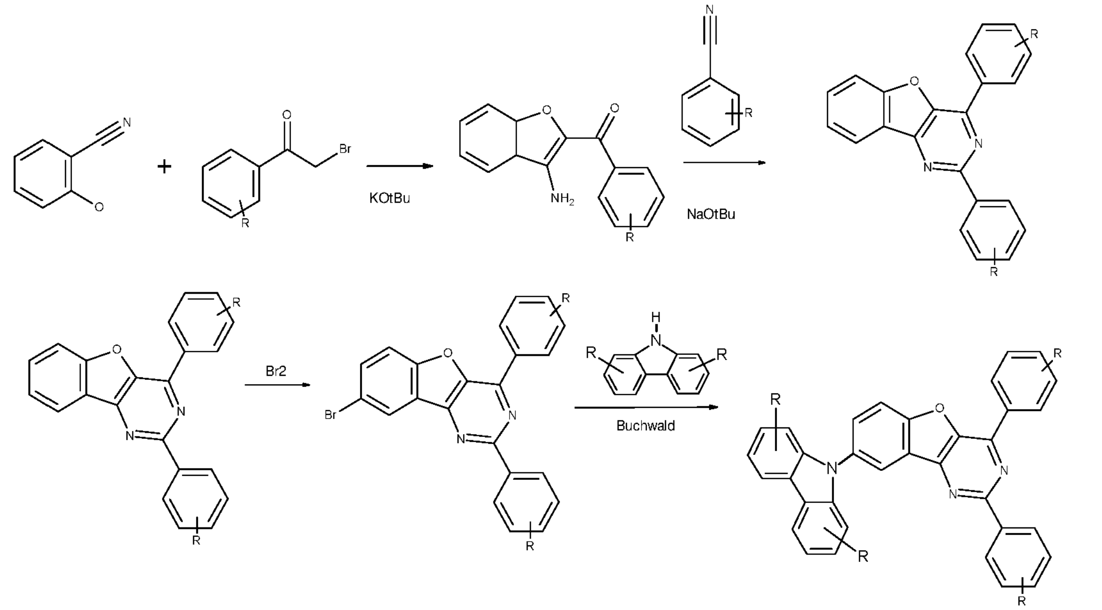


Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (Ic), (IIc) und/oder (IIIc) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityloder verzweigte Terphenyl- oder Quaterphenylgruppen, die eine Löslichkeit in gängigen organischen Lösemitteln bewirken, wie beispielsweise Toluol oder Xylol bei Raumtemperatur in ausreichender Konzentration löslich, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (Ic), (IIc) und/oder (IIIc) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (Ic), (IIc) und/oder (IIIc) oder erfindungsgemäße Verbindungen, wobei ein oder mehrere Bindungen der erfindungsgemäßen Verbindungen oder der Strukturen der Formel (Ic), (IIc) und/oder (IIIc) zum Polymer, Oligomer oder Dendrimer vorhanden sind. Je nach Verknüpfung der Strukturen der Formel (Ic), (IIc) und/oder (IIIc) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nichtkonjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (Ic), (IIc) und/oder (IIIc) bzw. die zuvor und nachfolgend ausgeführten bevorzugten Ausführungsformen zu 0.01 bis 99.9

mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (Ic), (IIc) und/oder (IIIc) bzw. die zuvor und nachfolgend ausgeführten bevorzugten Ausführungsformen bevorzugt, die eine Glasübergangstemperatur von mindestens 70°C, besond ers bevorzugt von mindestens 110°C, ganz besonders bevorzugt von mind estens 125°C und insbesondere bevorzugt von mindestens 150°C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisch funktionelles Material. Funktionelle Materialen sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Die vorliegenden Erfindung betrifft daher auch eine Zusammensetzung enthaltend wenigstens eine Verbindung umfassend Strukturen gemäß Formel (Ic), (IIc) und/oder (IIIc) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial lochtransportierende Eigenschaften auf.

Ein weiterer Gegenstand der vorliegenden stellt eine Zusammensetzung dar, enthaltend wenigstens eine Verbindung, umfassend mindestens eine Struktur gemäß Formel (Ic), (IIc) und/oder (IIIc) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen ohne Metalle wird zuerst eine Geometrieoptimierung mit der Methode "Ground

State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Für metallhaltige Verbindungen wird die Geometrie über die Methode "Ground State/ Hartree-Fock/Default Spin/LanL2MB/Charge 0/Spin Singlet" optimiert. Die Energierechnung erfolgt analog zu der oben beschriebenen Methode für die organischen Substanzen mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (Ic), (IIc) und/oder (IIIc) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angegebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011 /032626, WO 2011 /066898, WO 2011/157339, WO 2012/007086, WO 2014/008982, WO 2014/023377, WO 2014/094961, WO 2014/094960 und den noch nicht offen gelegten Anmeldungen EP 13004411.8, EP 14000345.0, EP 14000417.7 und EP 14002623.8 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

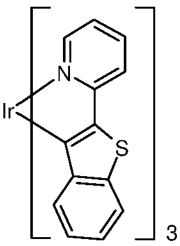
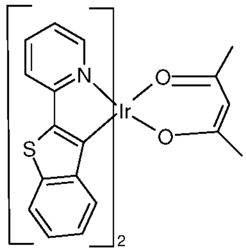
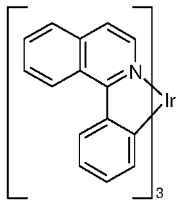

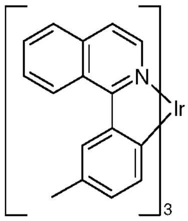
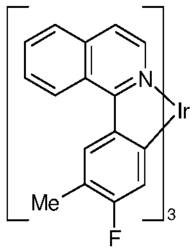
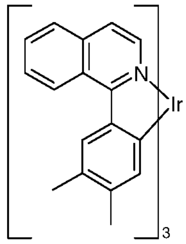

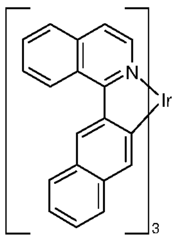
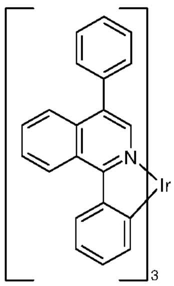
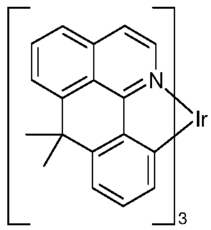

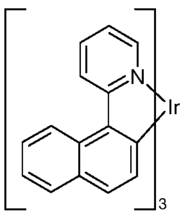
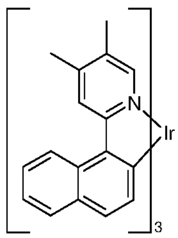
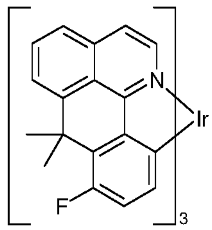

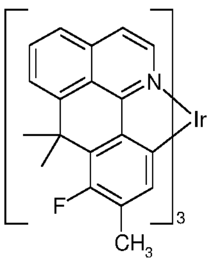
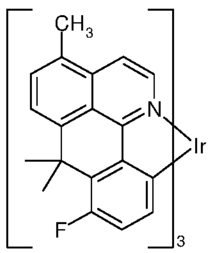
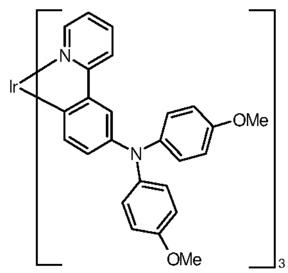

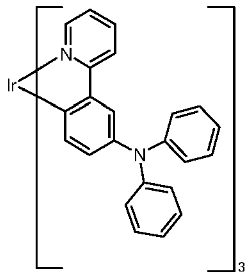
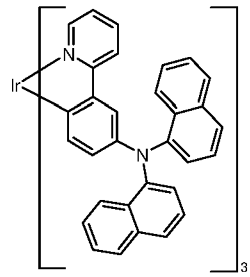
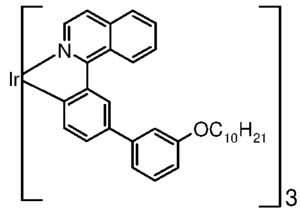

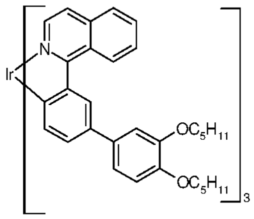
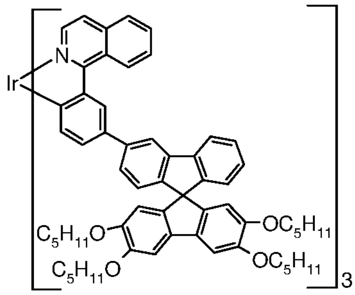
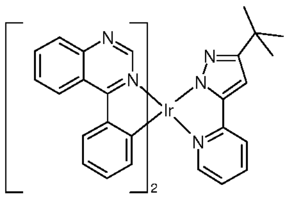

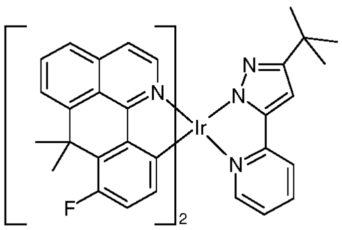
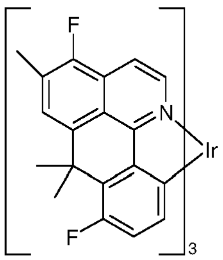
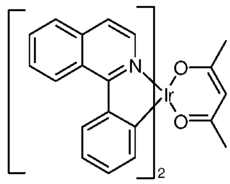

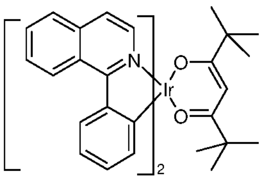
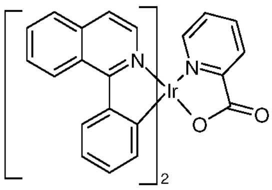
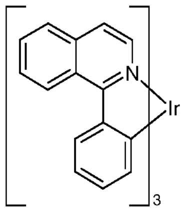

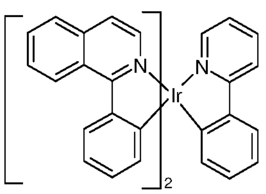
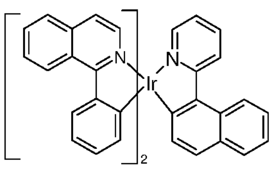
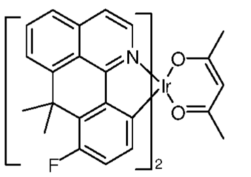

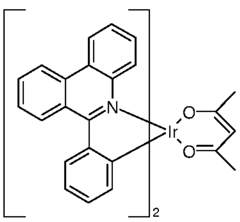
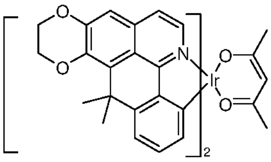
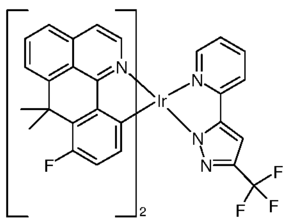

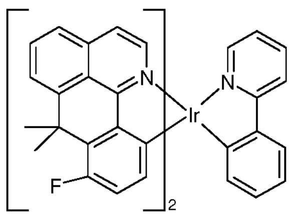
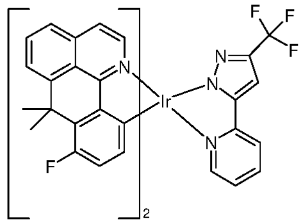
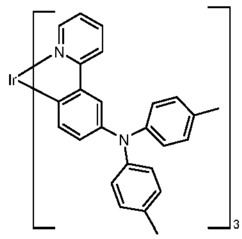

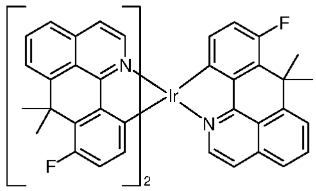
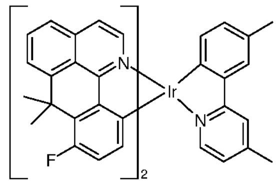
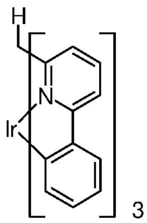

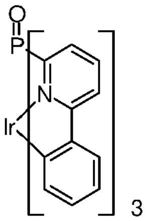
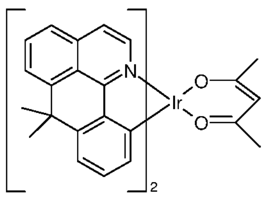
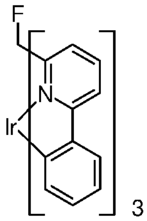

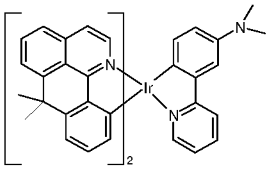
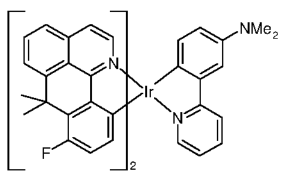
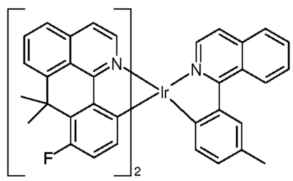

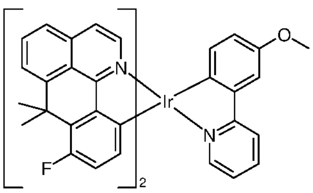
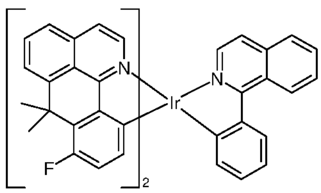
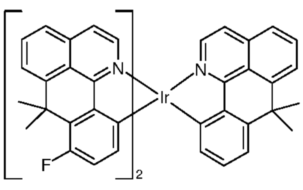

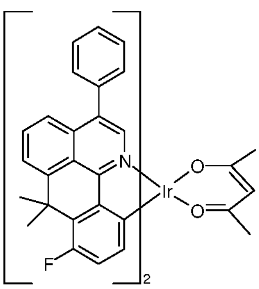
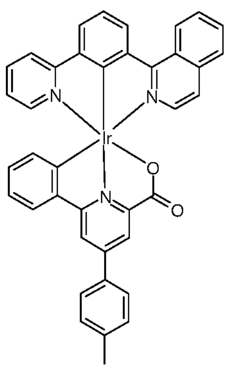
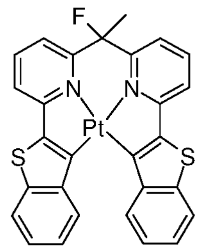

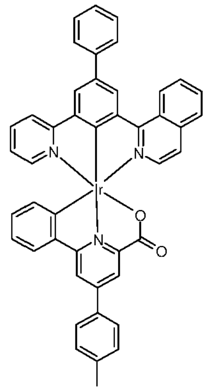
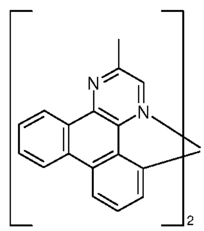
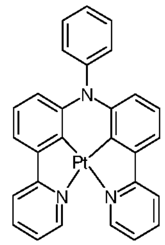

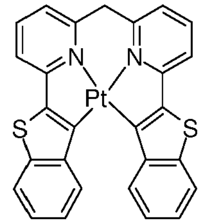
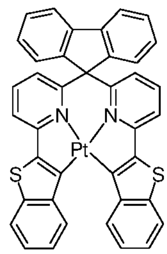
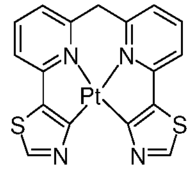

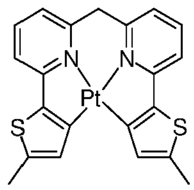
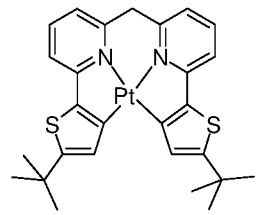
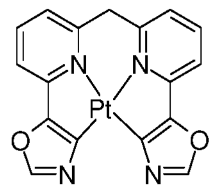

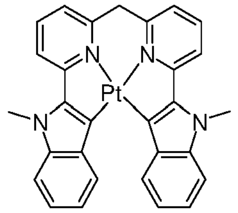
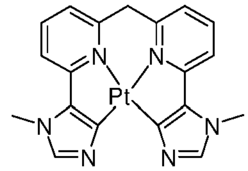
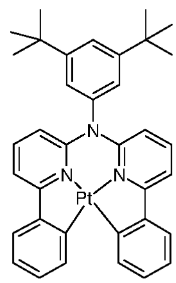

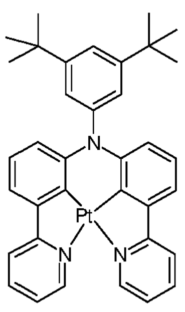
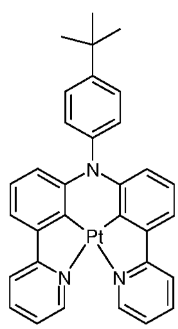
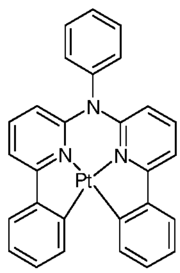

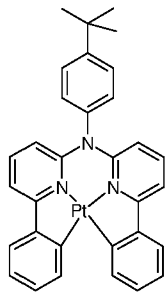
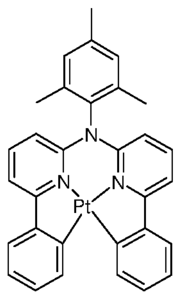
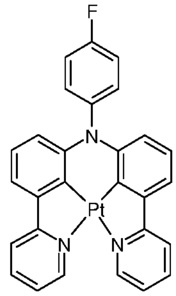
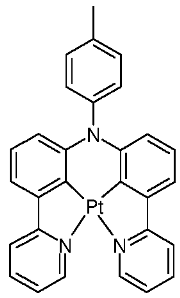
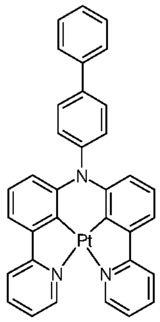
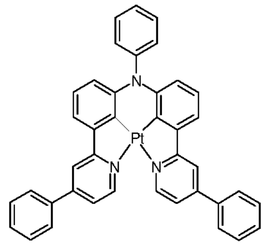
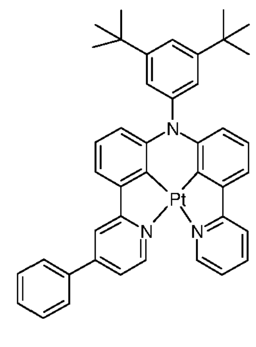
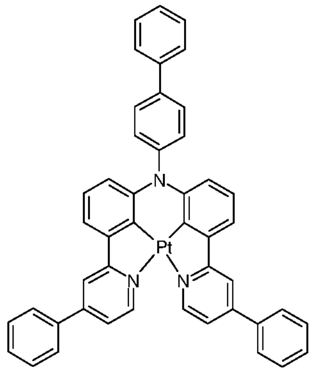
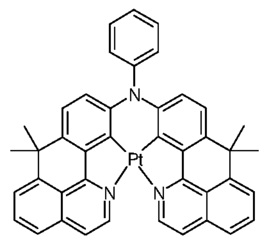
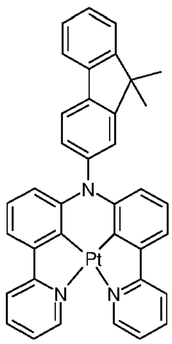
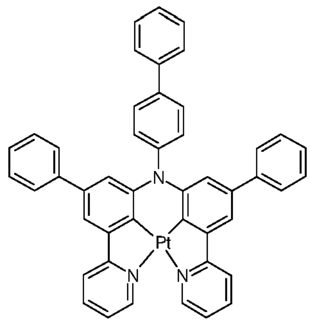
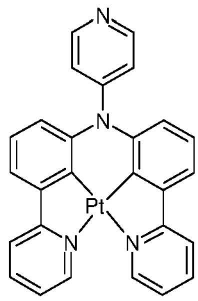

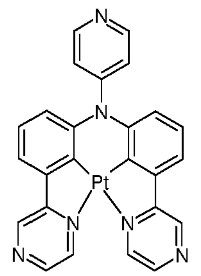

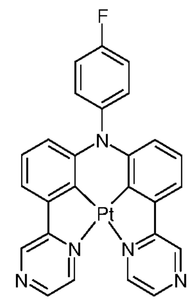

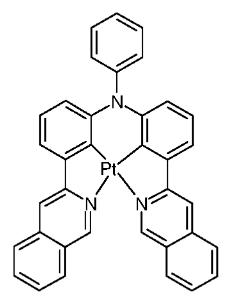

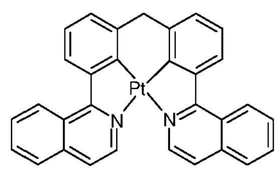

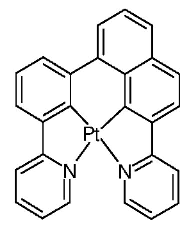

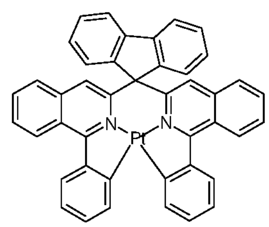

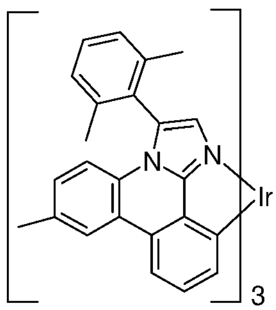

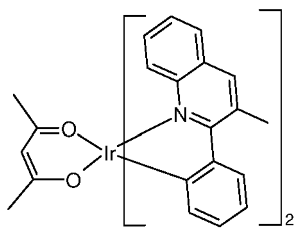

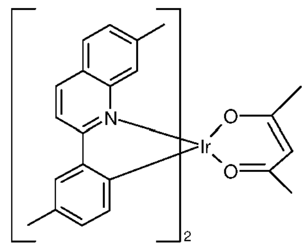

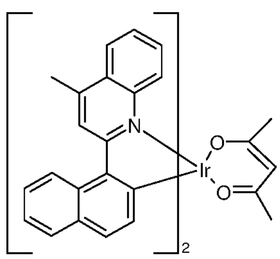

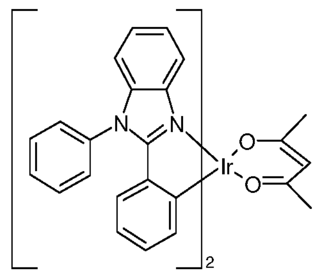

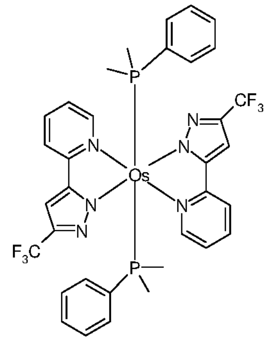

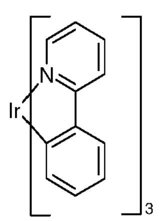

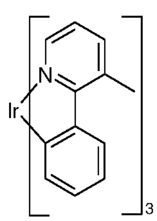

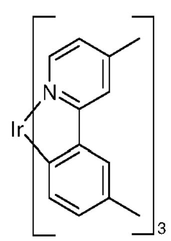

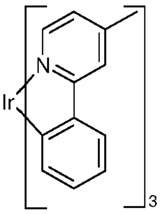
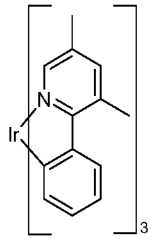
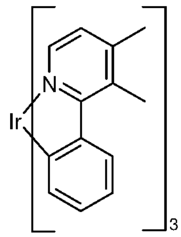
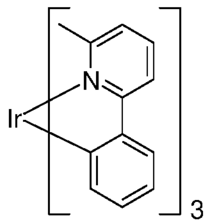
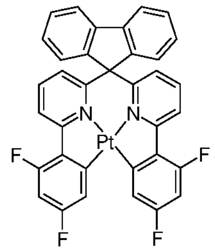
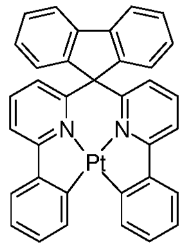
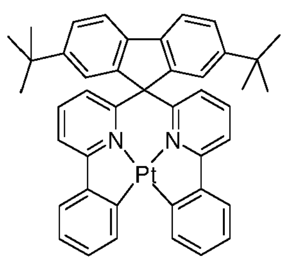
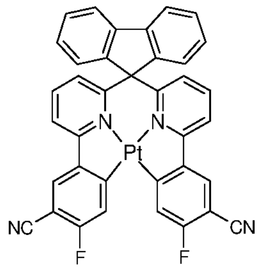
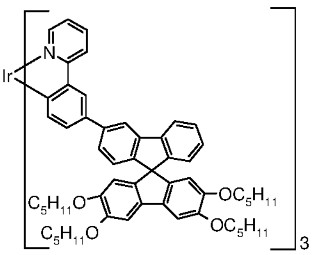
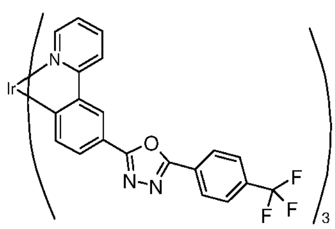
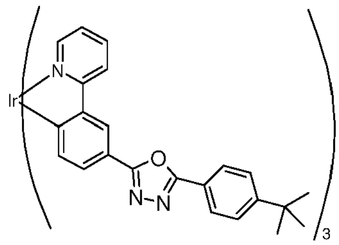
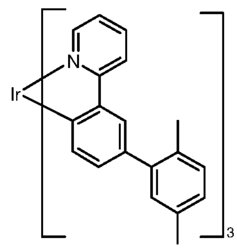
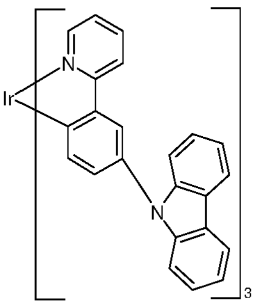
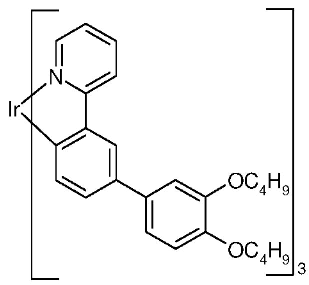
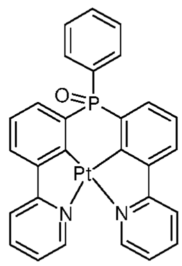

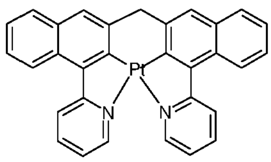
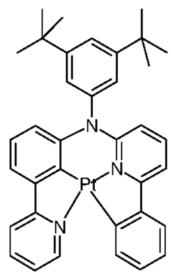
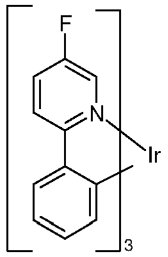

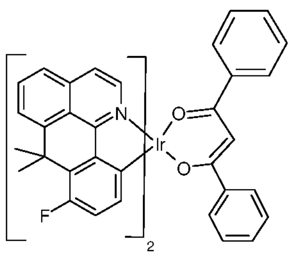
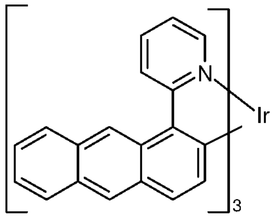
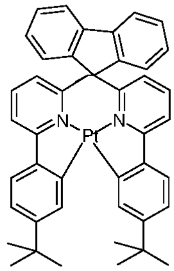

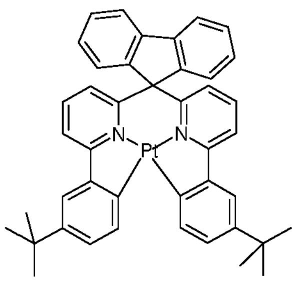
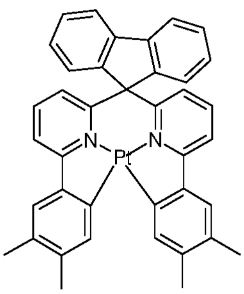
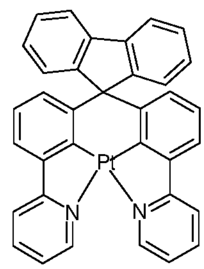

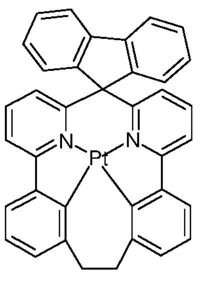
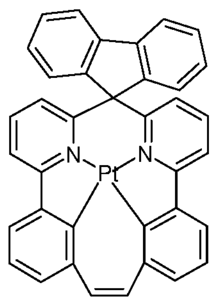
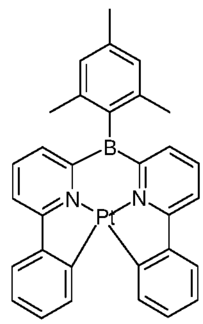

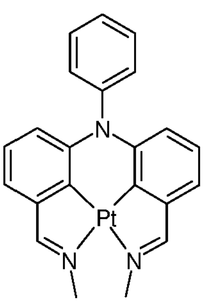
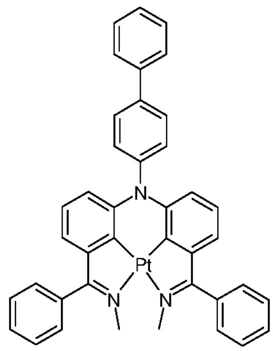
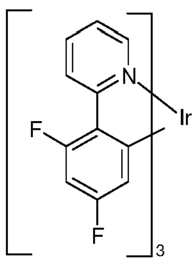

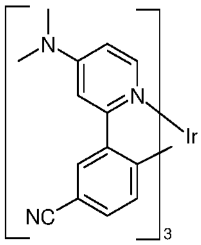
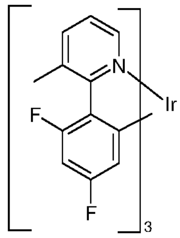
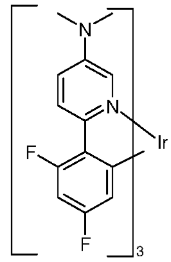

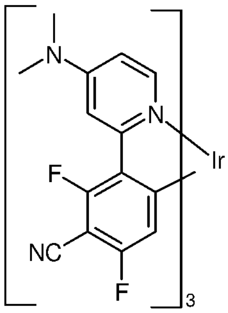
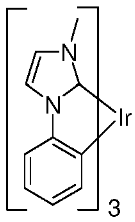
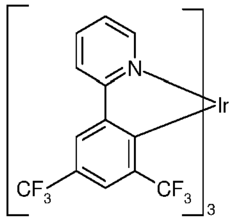

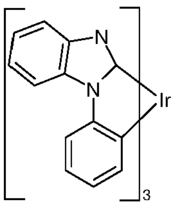
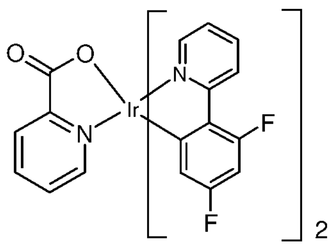
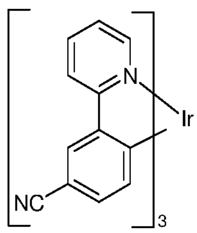

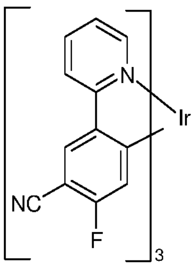
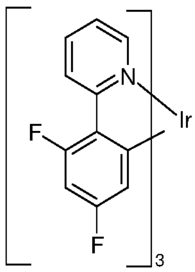
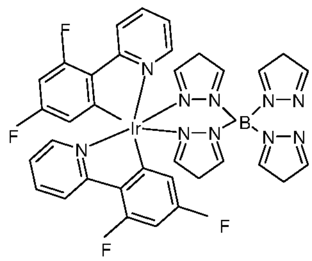

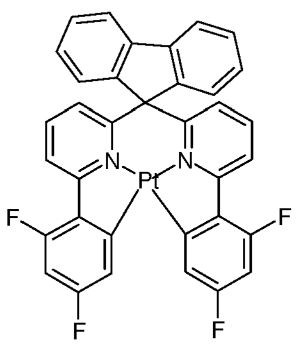
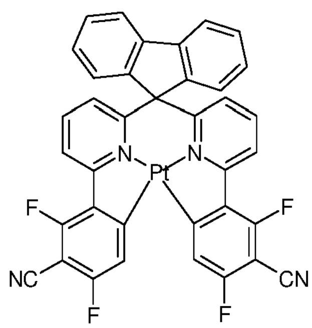
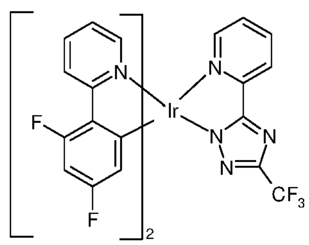

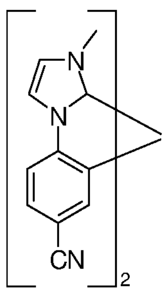
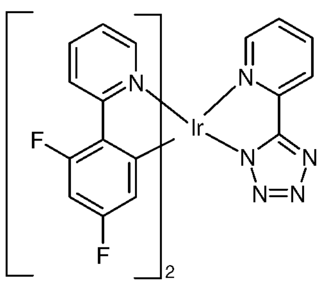
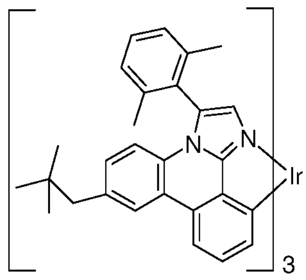

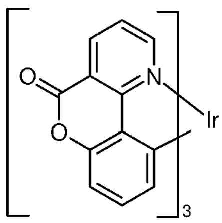
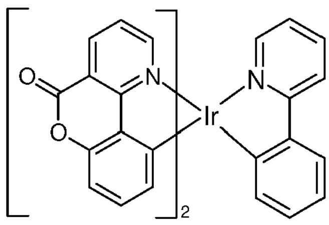
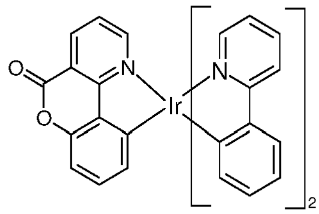

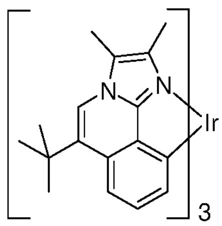
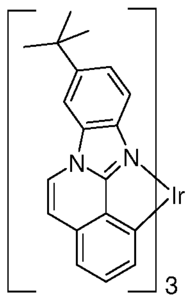
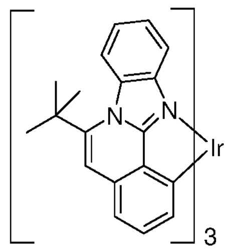

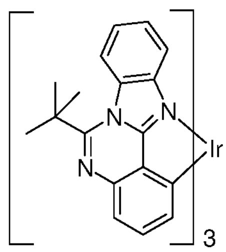
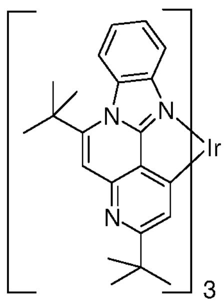
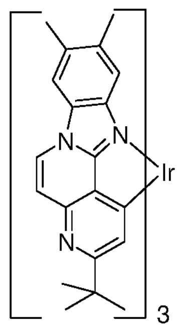

Die oben beschriebenen Verbindung, umfassend Strukturen der Formel (Ic), (IIc) und/oder (IIIc) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (Ic), (IIc) und/oder (IIIc), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (Ic), (IIc) und/oder (IIIc). Besonders bevorzugt

sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie $MoO_3$ oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die erfindungsgemäße Verbindung umfassend Strukturen gemäß Formel (Ic), (IIc) und/oder (IIIc) bzw. die oben aufgeführten bevorzugten Ausführungsformen als Matrixmaterial, vorzugsweise als elektronenleitendes Matrixmaterial in einer oder mehreren emittierenden Schichten, bevorzugt in Kombination mit einem weiteren Matrixmaterial, vorzugsweise einem lochleitenden Matrixmaterial. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den Verbindungen gemäß Formel (Ic), (IIc) und/oder (IIIc) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011 /042107, WO 2011 /088877 oder WO 2012/143080, Triphenylenderivate, z. B. gemäß WO 2012/048781, Lactame, z. B. gemäß WO 2011/116865, WO 2011/137951 oder WO 2013/064206, oder 4-Spirocarbazol-Derivate, z. B. gemäß WO 2014/094963 oder der noch nicht offen gelegten Anmeldung EP 14002104.9. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

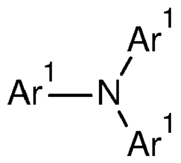

Formel (TA-1)

wobei $Ar^1$ gleich oder verschieden bei jedem Auftreten ein aromatisches oder heteroaromatisches Ringsystem mit 5 bis 60 aromatischen Ringatomen, das jeweils mit einem oder mehreren Resten $R^2$ substituiert sein kann, eine Aryloxygruppe mit 5 bis 60 aromatischen Ringatomen, die mit einem oder mehreren Resten $R^2$ substituiert sein kann, oder einer Aralkylgruppe mit 5 bis 60 aromatischen Ringatomen, die jeweils mit einem oder mehreren Resten $R^2$ substituiert sein kann, darstellt, wobei optional zwei oder mehr benachbarte Substituenten $R^2$ ein mono- oder polycyclisches, aliphatisches Ringsystem bilden können, das mit einem oder mehreren Resten $R^3$ substituiert sein kann, wobei das Symbol $R^2$ die zuvor, insbesondere für Formel (Ic), (IIc) und/oder (IIIc) genannte Bedeutung aufweist. Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^1$ gleich oder verschieden bei jedem Auftreten ausgewählt aus den oben genannten Gruppen ($R^1$-1) bis ($R^1$-87), vorzugsweise ($R^1$-1) bis ($R^1$-54), insbesondere bevorzugt ($R^1$-1) bis ($R^1$-51), speziell bevorzugt ($R^1$-1) bis ($R^1$-37), wobei Reste gemäß ($R^1$-1) ganz besonders bevorzugt sind. Hierbei gelten die zuvor für die Gruppen der Formeln ($R^1$-1) bis ($R^1$-87) dargelegten Bevorzugungen hinsichtlich der Summe der Indices und der an diese Gruppen gebundenen Reste $R^2$.

In einer bevorzugten Ausführungsform der Verbindungen der Formel (TA-1) ist mindestens eine Gruppe $Ar^1$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^1$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^1$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^1$ ausgewählt aus einer Fluoren- oder Spirobifluorengruppe, insbesondere einer 4-Fluoren- bzw. 4-Spirobifluorengruppe, und eine Gruppe $Ar^1$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^1$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

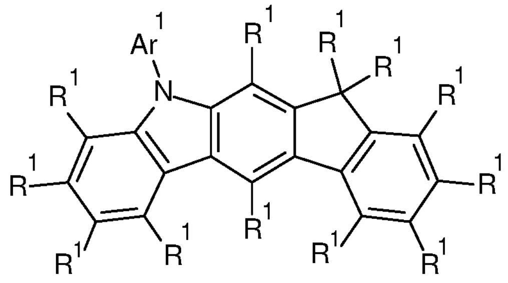

Formel (TA-2)

wobei $Ar^1$ und $R^1$ die oben insbesondere für Formeln (Ic), (IIc), (IIIc) und/oder (TA-1) aufgeführten Bedeutungen aufweisen. Dabei sind bevorzugte Ausführungsformen der Gruppe $Ar^1$ die oben aufgeführten Strukturen $R^1$-1 bis $R^1$-87, besonders bevorzugt $R^1$-1 bis $R^1$-51.

Eine bevorzugte Ausführungsform der Verbindungen der Formel (TA-2) sind die Verbindungen der folgenden Formel (TA-2a),

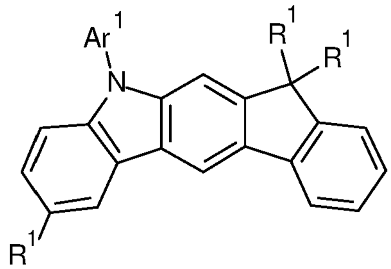

Formel (TA-2a)

wobei $Ar^1$ und $R^1$ die oben, insbesondere für Formeln (Ic), (IIc), (IIIc) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

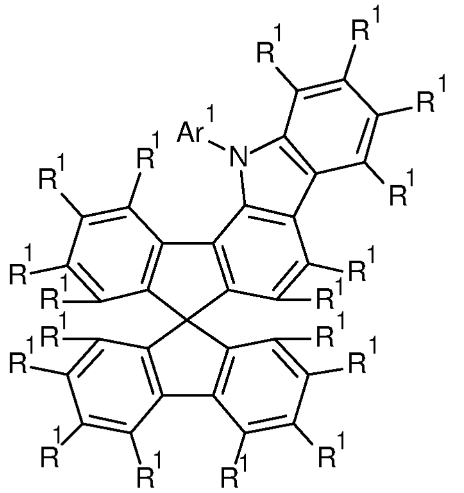

Formel (TA-3)

wobei $Ar^1$ und $R^1$ die oben, insbesondere für Formel (Ic), (IIc), (IIIc) und/oder (TA-1) aufgeführten Bedeutungen aufweisen. Dabei sind bevorzugte Ausführungsformen der Gruppe $Ar^1$ die oben aufgeführten Strukturen $R^1$-1 bis $R^1$-87, besonders bevorzugt $R^1$-1 bis $R^1$-51.

Eine bevorzugte Ausführungsform der Verbindungen der Formel (TA-3) sind die Verbindungen der folgenden Formel (TA-3a),

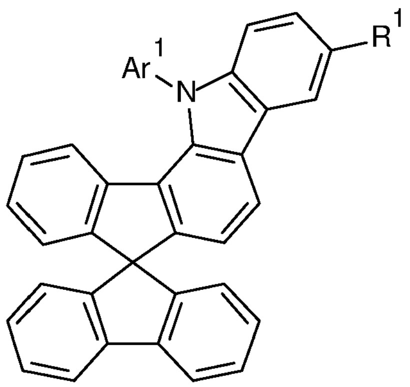

Formel (TA-3a)

wobei $Ar^1$ und $R^1$ die oben, insbesondere für Formel (Ic), (IIc) und/oder (IIIc) aufgeführten Bedeutungen aufweisen. Dabei sind bevorzugte Ausführungsformen der Gruppe $Ar^1$ die oben aufgeführten Strukturen $R^1$-1 bis $R^1$-87, besonders bevorzugt $R^1$-1 bis $R^1$-51.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

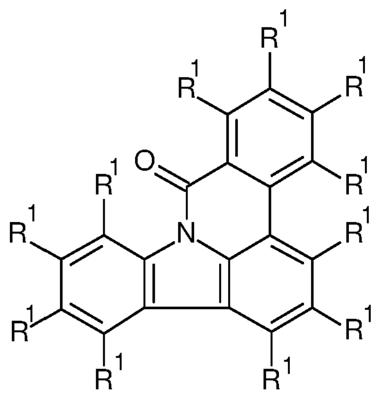

Formel (LAC-1)

wobei $R^1$ die oben, insbesondere für Formel (Ic), (IIc) und/oder (IIIc) aufgeführte Bedeutung aufweist.

Eine bevorzugte Ausführungsform der Verbindungen der Formel (LAC-1) sind die Verbindungen der folgenden Formel (LAC-1a),

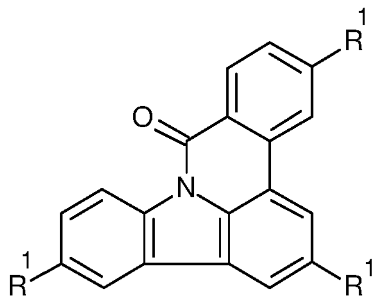

Formel (LAC-1a)

wobei $R^1$ die oben, insbesondere für Formel (Ic), (IIc) und/oder (IIIc) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formel (Ic), (IIc) und/oder (IIIc) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-79 abgebildet sind, besonders bevorzugt $R^1$-1 bis $R^1$-51.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der TriplettEmitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (Ic), (IIc) und/oder (IIIc) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend Verbindung umfassend Strukuren

gemäß Formel (Ic), (IIc) und/oder (IIIc) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (Ic), (IIc) und/oder (IIIc) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren elektronenleitenden Schichten umfasst, als elektronenleitende Verbindung.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise $MoO_3$ oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch

versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronischen Vorrichtung, insbesondere die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formel (Ic), (IIc) und/oder (IIIc) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formel (Ic), (IIc) und/oder (IIIc) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronischen Vorrichtungen, insbesondere organische Elektrolumineszenzvorrichtungen enthaltend Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen, insbesondere als elektronenleitende Materialien und/oder Matrixmaterialien, weisen eine sehr gute Lebensdauer auf.

2. Elektronischen Vorrichtungen, insbesondere organische Elektrolumineszenzvorrichtungen enthaltend Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen als elektronenleitende Materialien, Elektroneninjektionsmaterialien und/oder Matrixmaterialien weisen eine hervorragende Effizienz auf. Insbesondere ist die Effizienz deutlich höher gegenüber analogen Verbindungen, die keine Struktureinheit gemäß Formel (Ic), (IIc) und/oder (IIIc) enthalten.

3. Die erfindungsgemäßen Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen zeigen eine sehr hohe Stabilität und führen zu Verbindungen mit einer sehr hohen Lebensdauer.

4. Mit Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen kann in elektronischen Vorrichtungen, insbesondere organische Elektrolumineszenzvorrichtungen die Bildung von optischen Verlustkanäle vermieden werden. Hierdurch zeichnen sich diese Vorrichtungen durch eine hohe PL- und damit hohe EL-Effizienz von Emittern bzw. eine ausgezeichnete Energieübertragung der Matrices auf Dotanden aus.

5. Die Verwendung von Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen in Schichten elektronischer Vorrichtungen, insbesondere organischer Elektrolumineszenzvorrichtungen führt zu einer hohen Mobilität der Elektron-Leiterstrukturen.

6. Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen zeichnen sich durch eine ausgezeichnete thermische Stabilität aus, wobei Verbindungen mit einer Molmasse von weniger als ca. 1200 g/mol gut sublimierbar sind.

7. Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen weisen eine ausgezeichnete Glasfilmbildung auf.

8. Verbindungen, Oligomere, Polymere oder Dendrimere mit Strukturen gemäß Formel (Ic), (IIc) und/oder (IIIc) bzw. die zuvor und nachfolgend ausgeführten bevorzugten Ausführungsformen bilden aus Lösungen sehr gute Filme.

9. Die Verbindungen, Oligomere, Polymere oder Dendrimere umfassend Strukturen gemäß Formel (Ic), (IIc) und/oder (IIIc) bzw. die zuvor und nachfolgend ausgeführten bevorzugten Ausführungsformen weisen ein überraschend hohes Triplett-Niveau $T_1$ auf, wobei dies insbesondere Verbindungen gilt, die als elektronenleitende Materialien eingesetzt werden.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als Hostmaterial, Matrixmaterial, Elektronentransportmaterial, Elektroneninjektionsmaterial und/oder Lochblockiermaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (Ic), (IIc) und/oder (IIIc) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

## Beispiele

Die nachfolgenden Synthesen werden, sofern nicht anders angegeben, unter einer Schutzgasatmosphäre in getrockneten Lösungsmitteln durchgeführt. Die erfindungsgemäßen Verbindungen können mittels dem Fachmann bekannten Syntheseverfahren dargestellt werden.

## Synthesebeispiele

### a) 2,4-Diphenyl-benzo[4,5]furo[3,2-d]pyrimidin

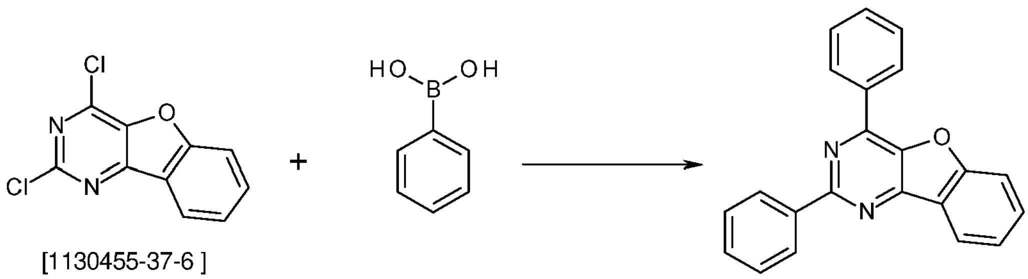

[1130455-37-6 ]

13 g (110.0 mmol) phenyl-boronsäure, 13 g (55 mmol) 2,4-Dichloro-benzo[4,5]furo[3,2-d]pyrimidin und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert und anschließend zur Trockene eingeengt. Der Rückstand wird aus Toluol und aus Dichlormethan /Heptan umkristallisiert. Ausbeute: 15 g (47 mmol), 87 % der Theorie.

Analog dazu werden die folgenden Verbindungen hergestellt:

| | Edukt 1 | Edukt 2 | Produkt | Ausbeute |
|---|---|---|---|---|
| 2a | 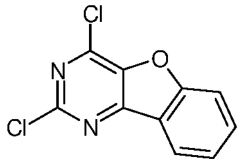 [1130455-37-6] | 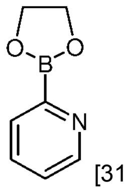 [317810-27-8] | 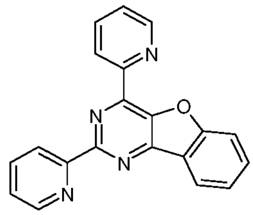 | 89% |
| 3a | 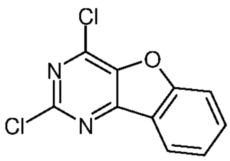 [1130455-37-6] | 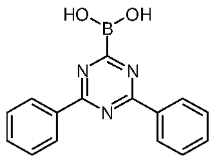 [1251825-65-6 ] | 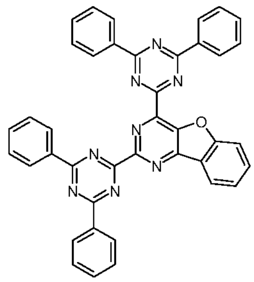 | 70% |
| 4a | 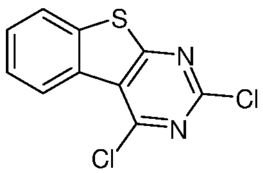 [76872-40-7] | 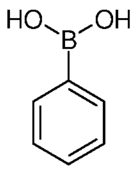 | 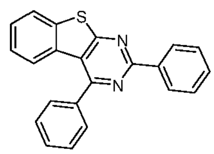 | 77% |
| 5a | 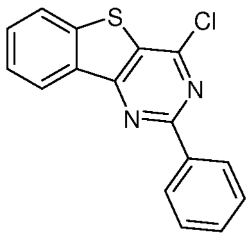 [900463-54-9 ] | 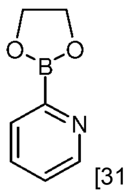 [317810-27-8] | 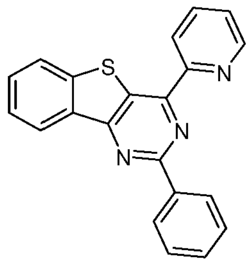 | 76% |
| 6a | 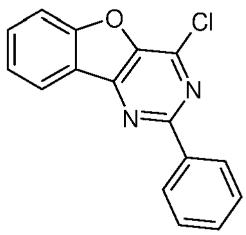 [134221-88-8 ] | 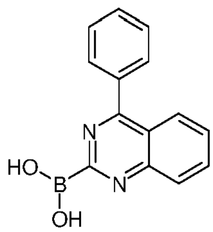 [1629973-75-6] | 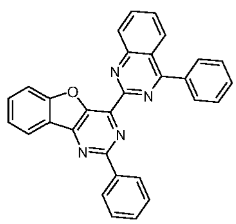 | 77% |
| 7a | 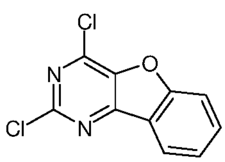 [1130455-37-6 ] | 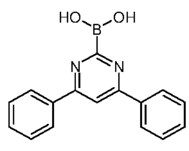 [1350818-50-6 ] | 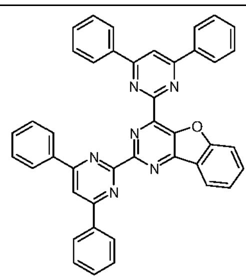 | 74% |

(fortgesetzt)

| | Edukt 1 | Edukt 2 | Produkt | Ausbeute |
|---|---|---|---|---|
| 8a | 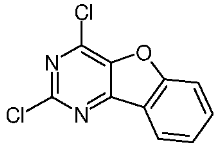 [1130455-37-6] | 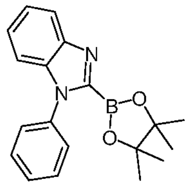 [1316275-47-4] | 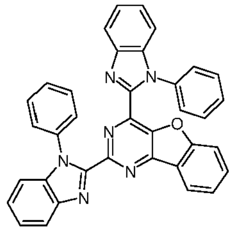 | 76% |
| 9a | 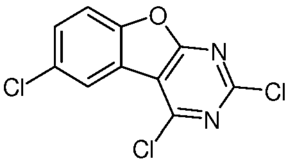 [1130455-37-6 ] | 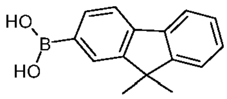 [333432-28-3] | 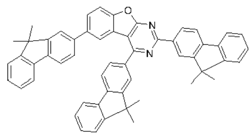 | 73% |
| 10a | 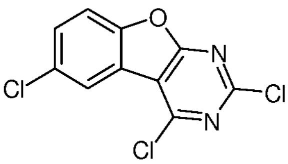 [1130455-37-6 ] | 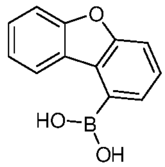 [162607-19-4 | 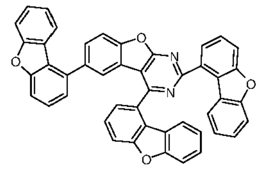 | 75% |
| 11a | 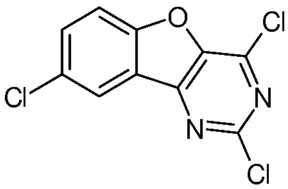 [160199-00-8] | 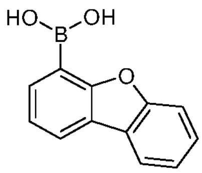 [100124-06-9 ] | 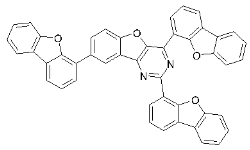 | 76% |

Das Produkt **9a** und **11a** wird via Säulenchromatographie an Kieselgel mit Toluol/Heptan (1:2) gereinigt und abschließend im Hochvakuum (p = 5 × $10^{-7}$ mbar) sublimiert (Reinheit 99,9%).

**b) 8-Bromo-2,4-diphenyl-benzo[4,5]furo[3,2-d]pyrimidin**

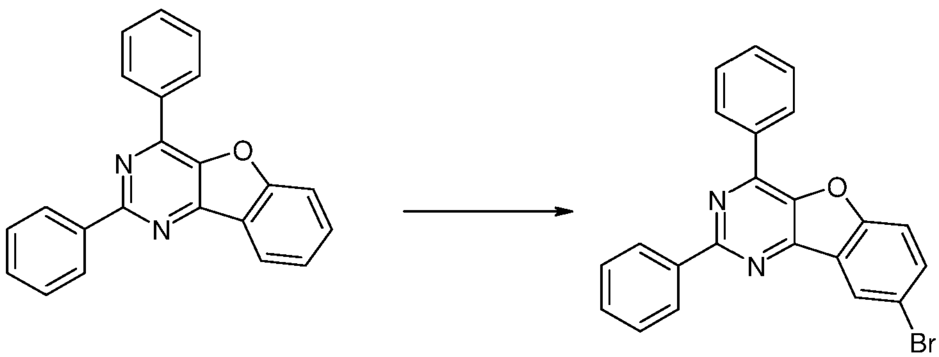

61 g (190.0 mmol) 2,4-Diphenyl-benzo[4,5]furo[3,2-d]pyrimidin werden 2000 mL Essigsäure(100%) und 2000 mL Schwefelsäure (95-98%) suspendiert. Zu dieser Suspension werden portionsweise 34 g (190 mmol) NBS zugegeben und 2 Stunden in Dunkelheit gerührt. Danach mit Wasser/Eis versetzt und Feststoff abgetrennt und mit Ethanol nachgewaschen. Der Rückstand wir im Toluol umkristallisiert. Die Ausbeute beträgt 65 g (163 mmol), entsprechend 86 %

der Theorie. Analog dazu werden die folgenden Verbindungen hergestellt:

| | **Edukt 1** | **Produkt** | **Ausbeute** |
|---|---|---|---|
| 2b | 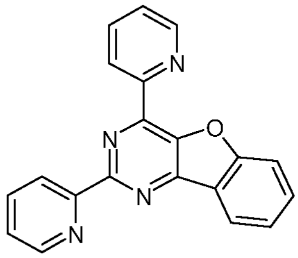 | 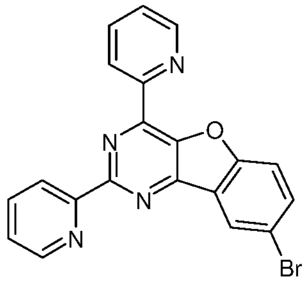 | 80% |
| 3b | 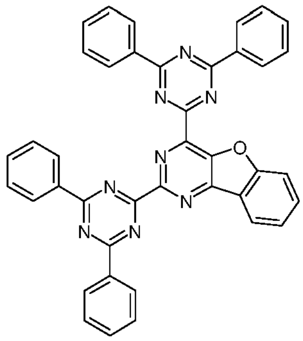 | 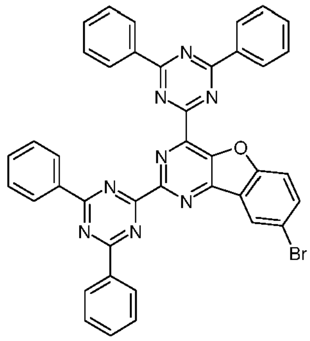 | 84% |
| 4b | 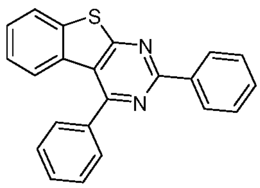 | 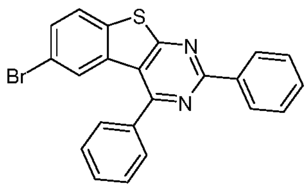 | 85% |
| 5b | 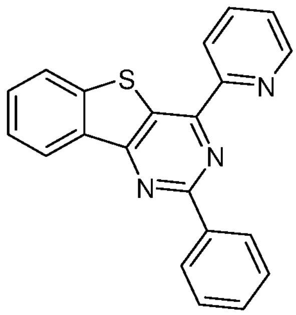 | 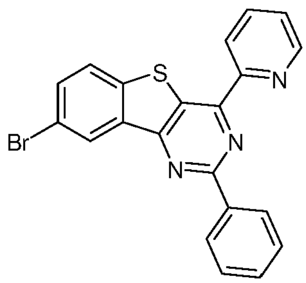 | 79% |
| 6b | 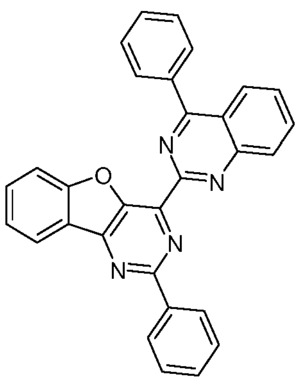 | 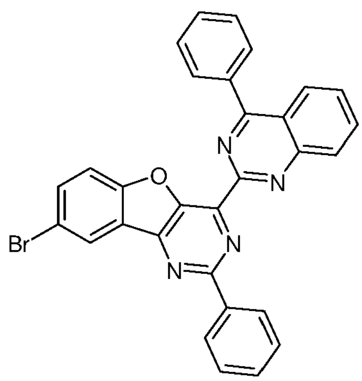 | 70% |

(fortgesetzt)

| | Edukt 1 | Produkt | Ausbeute |
|---|---|---|---|
| 7b | 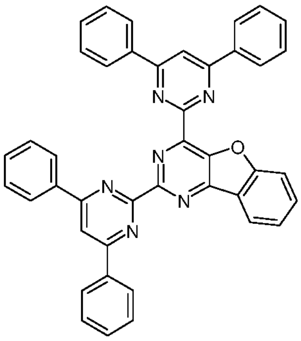 | 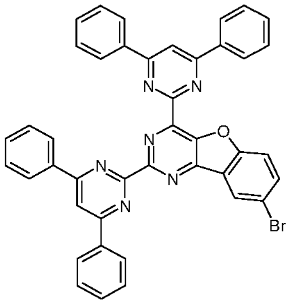 | 73% |
| 8b | 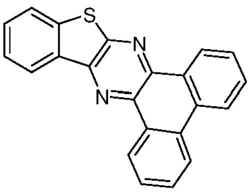 [101537-71-7 ] | 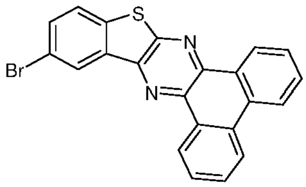 | 80% |
| 9b | 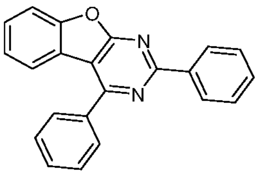 [129190-79-0 ] | 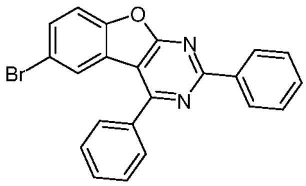 | 85% |
| 10b | 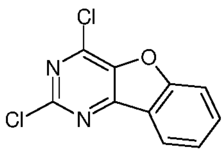 [1130455-37-6 ] | 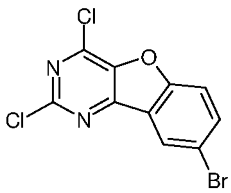 | 69% |
| 11b | 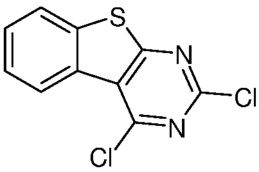 [76872-40-7] | 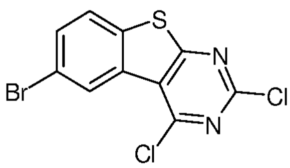 | 71% |
| 12b | 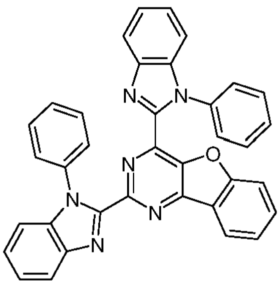 | 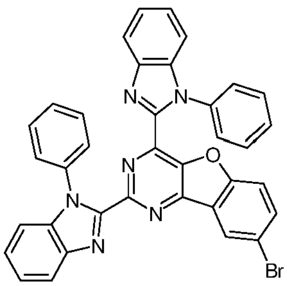 | 74% |

(fortgesetzt)

| | Edukt 1 | Produkt | Ausbeute |
|---|---|---|---|
| 13b | 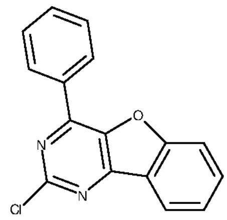<br>[1801233-17-9] | 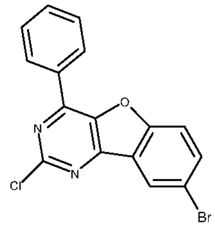 | 79% |

**c) 2,4-Diphenyl-8-(3-phenyl-carbazol-9-yl)-benzo[4,5]furo[3,2-d]pyrimidin (0c)**

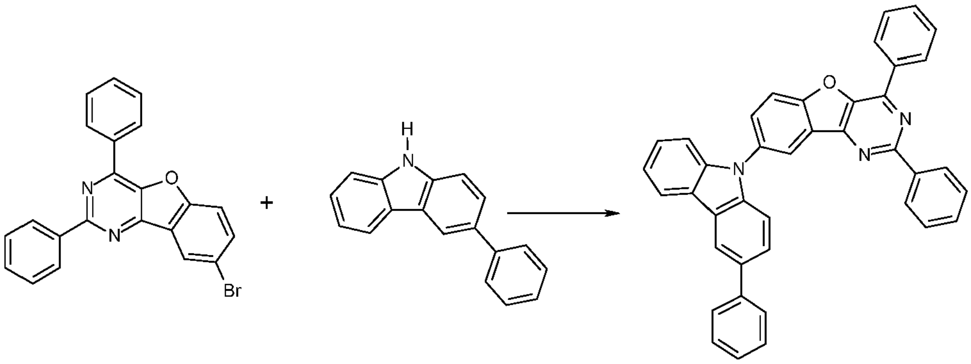

Eine entgaste Lösung von 59 g (147 mmol) 8-Bromo-2,4-diphenyl-benzo[4,5]furo[3,2-d]pyrimidin und 35,7 g (147 mmol) 3-Phenyl-9H-carbazol in 600 mL Toluol wird 1 h mit $N_2$ gesättigt. Danach wird die Lösung zuerst mit 2.09 mL (8.6 mmol) $P(tBu)_3$, dann mit 1.38 g (6.1 mmol) Palladium(II)acetat versetzt und anschließend werden 17.7 g (185 mmol) Na*Ot*Bu im festen Zustand zugegeben. Die Reaktionsmischung wird 1 h unter Rückfluss erhitzt. Nach dem Abkühlen auf Raumtemperatur werden vorsichtig 500 mL Wasser zugesetzt. Die wässrige Phase wird mit 3 × 50 mL Toluol gewaschen, über $MgSO_4$ getrocknet und das Lösungsmittel im Vakuum entfernt. Danach wird das Rohprodukt über Kieselgel mit Heptan/Essigsäureester (20/1) chromatographisch gereinigt. Der Rückstand wird aus Toluol umkristallisiert und abschließend im Hochvakuum ($p = 5 \times 10^{-6}$ mbar) sublimiert.

Die Ausbeute beträgt 68 g (120 mmol), entsprechend 82% der Theorie.

Analog können folgende Verbindungen (Verbindung 14c ist nicht Teil der Erfindung, sondern eine Referenzverbindung) erhalten werden:

| | Edukt 1 | Edukt 2 | Produkt | Ausbeute |
|---|---|---|---|---|
| 1c | 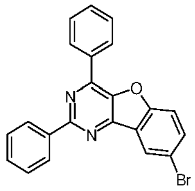 | 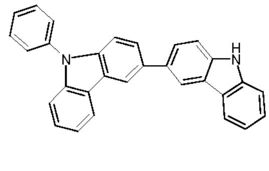<br>[ 1345202-03-0] | 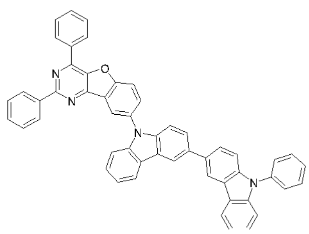 | 85% |

(fortgesetzt)

| | Edukt 1 | Edukt 2 | Produkt | Ausbeute |
|---|---|---|---|---|
| 2c | 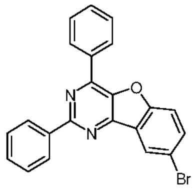 | 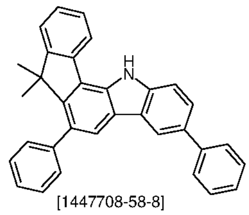 [1447708-58-8] | 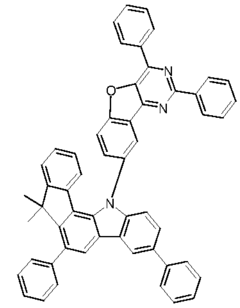 | 81% |
| 3c | 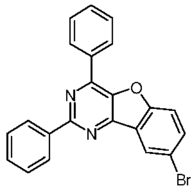 | 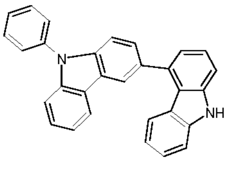 [ 1407183-66-7] | 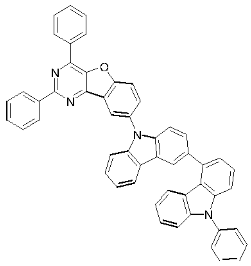 | 67% |
| 4c | 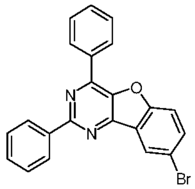 | 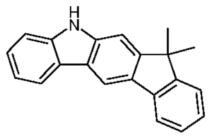 [1257220-47-5] | 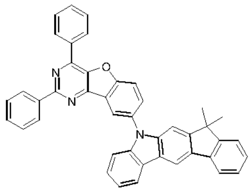 | 72% |
| 5c | 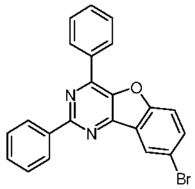 | 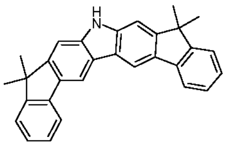 [1373281-72-1] | 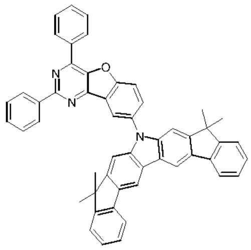 | 67% |
| 6c | 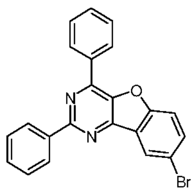 | 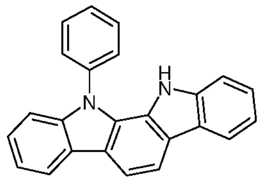 [1024598-06-8] | 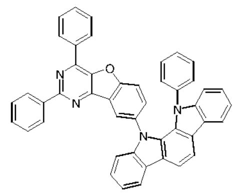 | 52% |
| 7c | 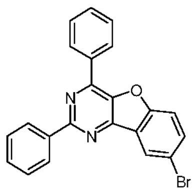 | 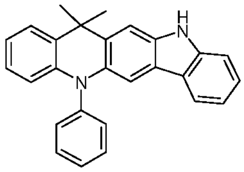 [1361126-04-6] | 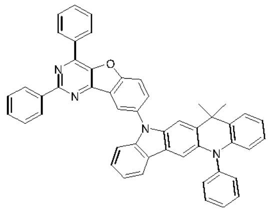 | 80% |

(fortgesetzt)

| | Edukt 1 | Edukt 2 | Produkt | Ausbeute |
|---|---|---|---|---|
| 8c | 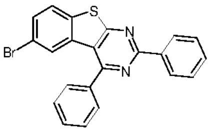 | 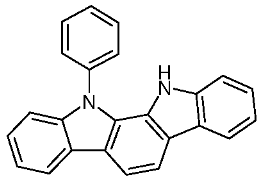 [1024598-06-8] | 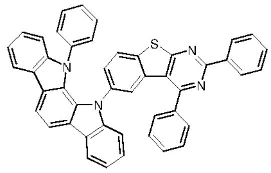 | 87% |
| 9c | 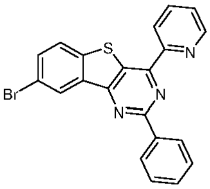 | 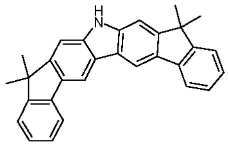 [1373281-72-1] | 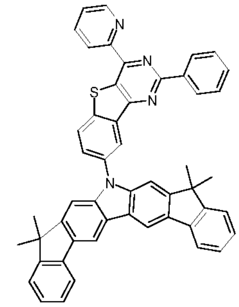 | 79% |
| 10c | 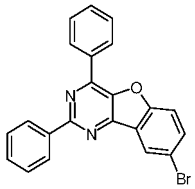 | 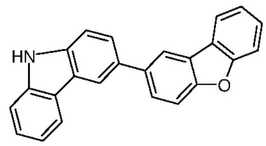 [953805-18-0] | 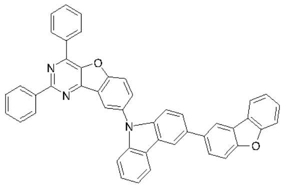 | 77% |
| 11c | 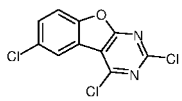 [1130455-37-6 ] | 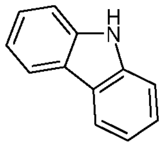 | 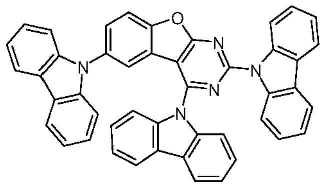 | 79% |
| 12c | 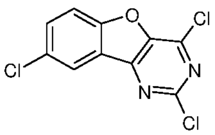 [160199-00-8] | 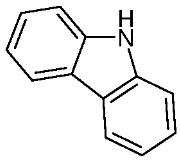 | 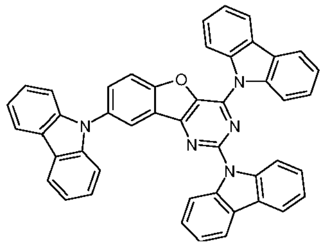 | 82% |
| 13c | 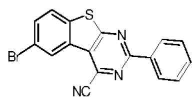 | 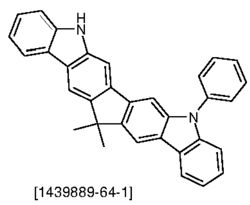 [1439889-64-1] | 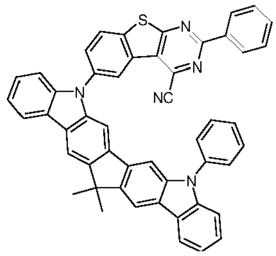 | 82% |

(fortgesetzt)

| | Edukt 1 | Edukt 2 | Produkt | Ausbeute |
|---|---|---|---|---|
| *14c | 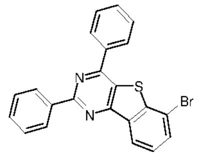 [1801233-18-0] | 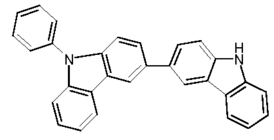 [ 1345202-03-0] | 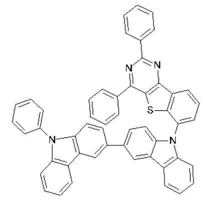 | 80% |
| 15c | 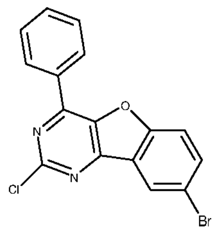 | 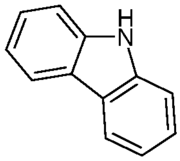 | 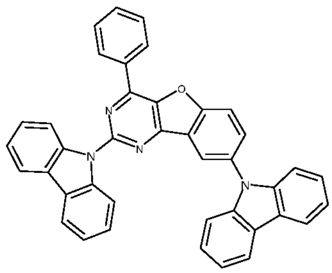 | 77% |

**d) 2,2'-Bis-carbazol-9-yl-4,4'-diphenyl-[8,8']bi[benzo[4,5]thieno[3,2-d]pyrimidinyl] (1d)**

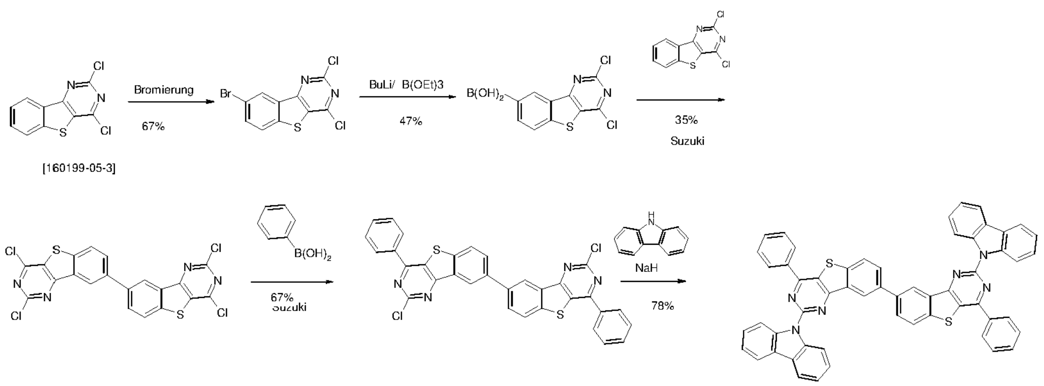


2,4-Dichloro-benzo[4,5]thieno[3,2-d]pyrimidin wird analog zu Vorschrift **b** bromiert, anschließend mit BuLi und Triethylborat zu entsprechender Boronsäure umgewandelt. Dann erfolgt die Kupplung analog zu Vorschrift **c** zu entsprechende Dimer und dann wiederum analog zu Vorschrift c erst mit Phenylboronsäure und zum Schluss durch Umsetzung mit NaH und Carbazol über nucleophile Substitution zum Zielmolekül (1d) umgesetzt.

**Herstellung der OLEDs**

In den folgenden Beispielen V1 bis E10 (siehe Tabellen 1 und 2) werden die Daten verschiedener OLEDs vorgestellt.

**Vorbehandlung für die Beispiele V1-E10:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht

immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:IC3:TEG2 (55%:35%:10%) bedeutet hierbei, dass das Material IC5 in einem Volumenanteil von 55%, IC3 in einem Anteil von 35% und TEG2 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. EQE1000 schließlich bezeichnet die externe Quanteneffizienz bei einer Betriebsleuchtdichte von 1000 cd/$m^2$.

Die Daten der verschiedenen OLEDs sind in Tabelle 2 zusammengefasst. Die Beispiele V1- V2 sind Vergleichsbeispiele gemäß dem Stand der Technik, die Beispiele E1-E10 zeigen Daten von erfindungsgemäßen OLEDs.

Im Folgenden werden einige der Beispiele näher erläutert, um die Vorteile der erfindungsgemäßen OLEDs zu verdeutlichen.

### Verwendung von erfindungsgemäßen Materialien in OLEDs

Die erfindungsgemäßen Materialien ergeben bei Einsatz in der Emissionsschicht (EML) in phosphoreszenten OLEDs wesentliche Verbesserungen gegenüber dem Stand der Technik, vor allem bezüglich der Spannung der Bauteile.

Durch Einsatz der erfindungsgemäßen Verbindungen 15c und 1d lässt sich eine Verringerung der Spannung um ca. -0.3 Volt (entspricht ca. 8%) gegenüber dem Stand der Technik erzielen (Vergleich von Beispiel E1 mit V1; Vergleich von Beispiel E2 mit V2) in Kombination mit dem phosphoreszenten roten Emitter TER5. Desweiteren zeigen die erfindungsgemäßen Beispiele E9 und E10, dass die erfindungsgemäßen Materialien auch in Kombination mit dem phosphoreszenten grünen Emitter TEG2 eine niedrige Spannung und eine hohe Effizienz in der OLED liefern.

Tabelle 1: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 125nm | SpMA3 10nm | SdT1 :TER5 (95%: 5%) 40nm | -- | ST2:LiQ (50%: 50%) 35nm | -- |
| V2 | HATCN 5nm | SpMA1 125nm | SpMA3 10nm | SdT2:TER5 (95%: 5%) 40nm | -- | ST2:LiQ (50%: 50%) 35nm | -- |
| E1 | HATCN 5nm | SpMA1 125nm | SpMA3 10nm | 15c:TER5 (95%: 5%) 40nm | -- | ST2:LiQ (50%: 50%) 35nm | -- |
| E2 | HATCN 5nm | SpMA1 125nm | SpMA3 10nm | 1d:TER5 (95%:5%) 40nm | -- | ST2:LiQ (50%: 50%) 35nm | -- |
| E3 | HATCN 5nm | SpMA1 125nm | SpMA3 10nm | 0c:TER5 (95%:5%) 40nm | -- | ST2:LiQ (50%: 50%) 35nm | -- |
| E4 | HATCN 5nm | SpMA1 125nm | SpMA3 10nm | 1c:TER5 (95%:5%) 40nm | -- | ST2:LiQ (50%: 50%) 35nm | -- |
| E5 | HATCN 5nm | SpMA1 125nm | SpMA3 10nm | 4c:TER5 (95%:5%) 40nm | -- | ST2:LiQ (50%: 50%) 35nm | -- |
| E6 | HATCN 5nm | SpMA1 125nm | SpMA3 10nm | 7c:TER5 (95%:5%) 40nm | -- | ST2:LiQ (50%: 50%) 35nm | -- |
| E7 | HATCN 5nm | SpMA1 125nm | SpMA3 10nm | 8c:TER5 (95%:5%) 40nm | -- | ST2:LiQ (50%: 50%) 35nm | -- |
| E8 | HATCN 5nm | SpMA1 125nm | SpMA3 10nm | 10c:TER5 (95%: 5%) 40nm | -- | ST2:LiQ (50%: 50%) 35nm | -- |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| E9 | HATCN 5nm | SpMA1 230nm | SpMA3 20nm | 1c:TEG2 (90%: 10%) 30nm | -- | ST2:LiQ (50%: 50%) 40nm | -- |
| E10 | HATCN 5nm | SpMA1 230nm | SpMA3 20nm | 4c:TEG2 (90%: 10%) 30nm | -- | ST2:LiQ (50%: 50%) 40nm | -- |

Tabelle 2: Daten der OLEDs

| Bsp. | U1000 (V) | EQE 1000 | CIE x/y bei 1000 cd/m$^2$ |
|---|---|---|---|
| V1 | 3.5 | 22.2 % | 0.67/0.33 |
| V2 | 3.6 | 22.4 % | 0.67/0.33 |
| E1 | 3.2 | 22.0 % | 0.67/0.33 |
| E2 | 3.3 | 22.2 % | 0.67/0.33 |
| E3 | 3.3 | 22.4 % | 0.67/0.33 |
| E4 | 3.4 | 22.1% | 0.67/0.33 |
| E5 | 3.2 | 22.3% | 0.67/0.33 |
| E6 | 3.3 | 22.5% | 0.67/0.33 |
| E7 | 3.3 | 22.2% | 0.67/0.33 |
| E8 | 3.4 | 22.3% | 0.67/0.33 |
| E9 | 3.3 | 20.5% | 0.33/0.63 |
| E10 | 3.2 | 21.1% | 0.33/0.63 |

Tabelle 3: Strukturformeln der Materialien für die OLEDs

| | |
|---|---|
| 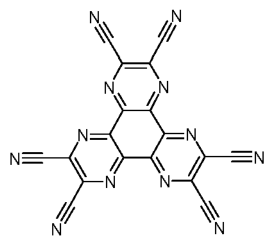 | 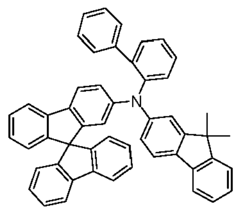 |
| HATCN | SpMA1 |
| 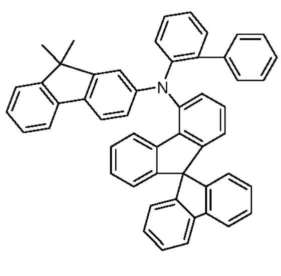 | 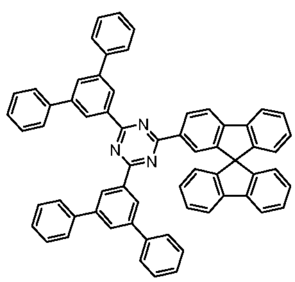 |
| SpMA3 | ST2 |

(fortgesetzt)

| | |
|---|---|
| 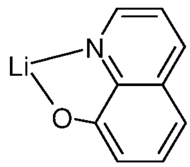 | 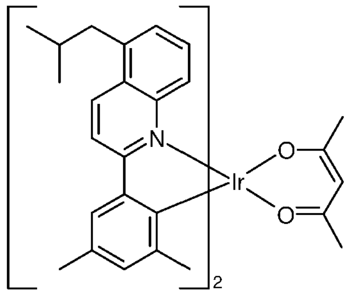 |
| LiQ | TER5 |
| 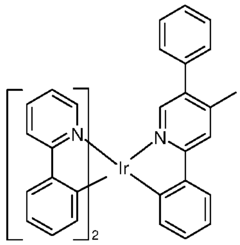 | |
| TEG2 | |
| 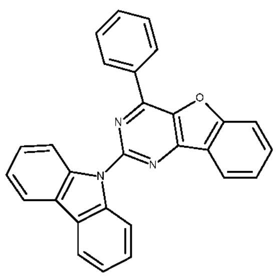 | 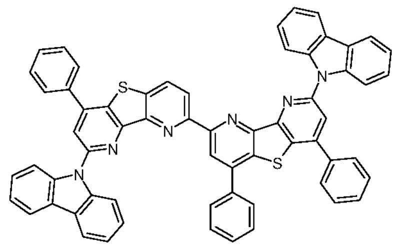 |
| SdT1 | SdT2 |
| 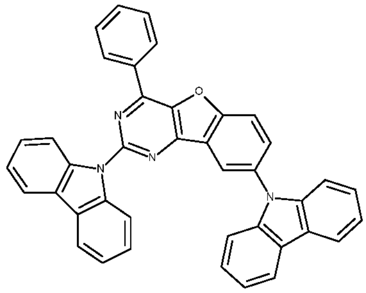 | 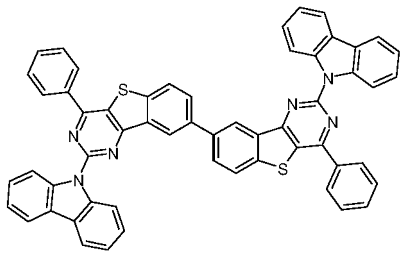 |
| 15c | 1d |
| 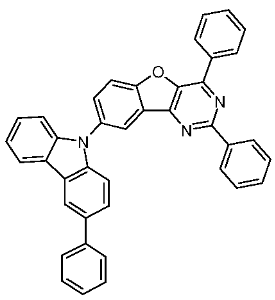 | 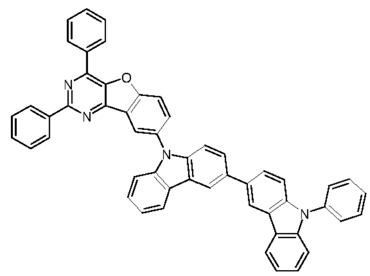 |
| 0c | 1c |

(fortgesetzt)

| | |
|---|---|
| 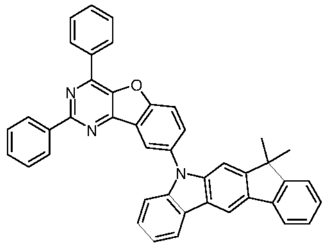 | 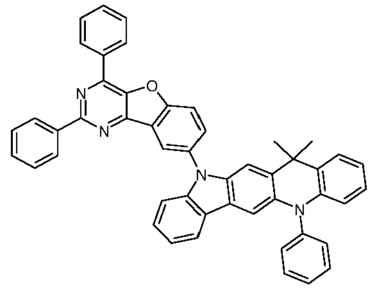 |
| 4c | 7c |
| 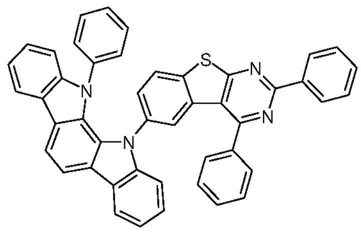 | 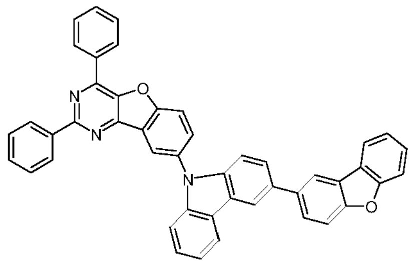 |
| 8c | 10c |

## Patentansprüche

**1.** Verbindung, umfassend Strukturen der Formeln (Ic), (IIc) und/oder (IIIc)

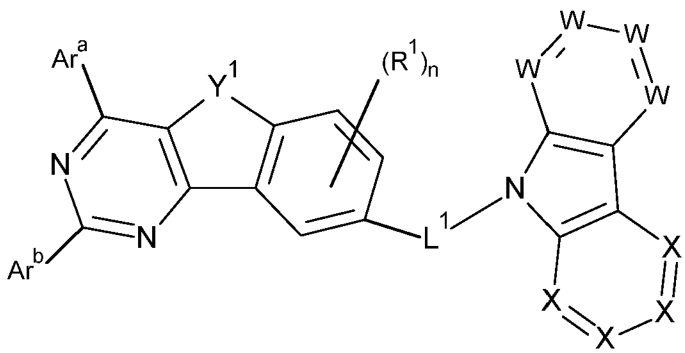

Formel (Ic)

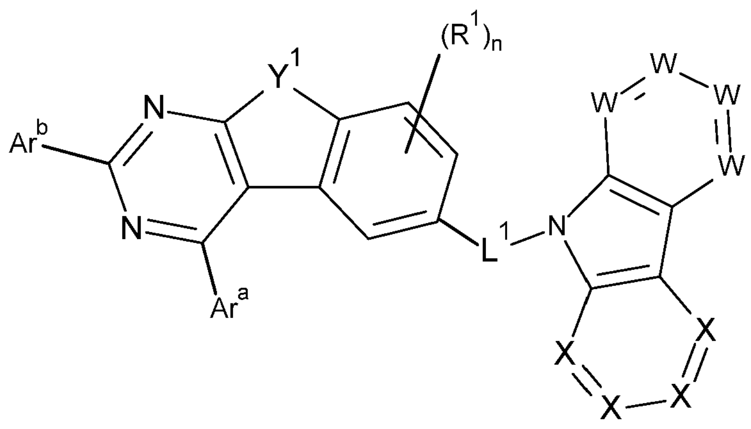

Formel (IIc)

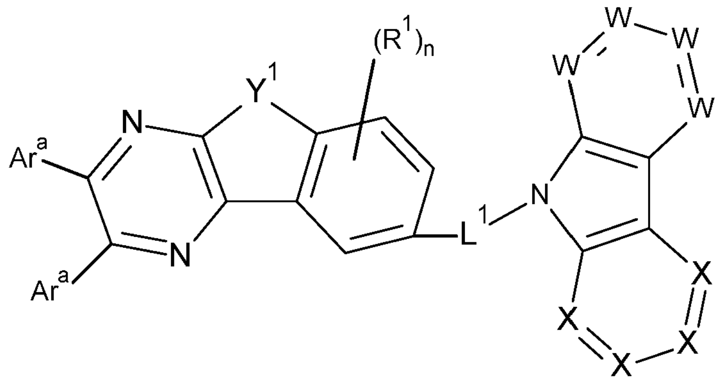

Formel (IIIc)

wobei für die verwendeten Symbole gilt:

- $Y^1$ ist O oder S;
- W ist bei jedem Auftreten gleich oder verschieden N oder $CR^1$, vorzugsweise $CR^1$, mit der Maßgabe, dass nicht mehr als zwei der Gruppen W in einem Cyclus für N stehen;
- X ist bei jedem Auftreten gleich oder verschieden N oder $CR^1$, vorzugsweise $CR^1$, mit der Maßgabe, dass nicht mehr als zwei der Gruppen X in einem Cyclus für N stehen;
- $L^1$ ist eine Bindung oder ein aromatisches oder heteroaromatisches Ringsystem mit 5 bis 30 aromatischen Ringatomen, welches durch einen oder mehrere Reste $R^1$ substituiert sein kann;
- $Ar^a$ ist bei jedem Auftreten gleich oder verschieden CN, F oder ein aromatisches oder heteroaromatisches Ringsystem mit 5 bis 30 aromatischen Ringatomen, welches durch einen oder mehrere Reste $R^1$ substituiert sein kann;
- $Ar^b$ ist bei jedem Auftreten gleich oder verschieden CN, F oder ein aromatisches oder heteroaromatisches Ringsystem mit 5 bis 30 aromatischen Ringatomen, welches durch einen oder mehrere Reste $R^1$ substituiert sein kann;
- $R^1$ ist bei jedem Auftreten gleich oder verschieden H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $-R^2C=CR^2-$, -C=C-, $Si(R^2)_2$, C=O, C=S, $C=NR^2$, -C(=O)O-, $-C(=O)NR^2-$, $NR^2$, $P(=O)(R^2)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^2$ substituiert sein kann, oder eine Aryloxy- oder Heteroaryloxygruppe mit 5 bis 40 aromatischen Ringatomen, die durch einen oder mehrere Reste $R^2$ substituiert sein kann, oder einer Aralkyl- oder

Heteroaralkylgruppe mit 5 bis 40 aromatischen Ringatomen, die mit einem oder mehreren Resten $R^2$ substituiert sein kann, oder eine Kombination dieser Systeme; dabei können zwei oder mehrere Substituenten $R^1$ auch miteinander ein mono- oder polycyclisches, aliphatisches oder aromatisches Ringsystem bilden;

$Ar^1$ ist bei jedem Auftreten gleich oder verschieden ein aromatisches oder heteroaromatisches Ringsystem mit 5 bis 30 aromatischen Ringatomen, das mit einem oder mehreren nichtaromatischen Resten $R^2$ substituiert sein kann; dabei können zwei Reste $Ar^1$, welche an dasselbe Si-Atom, N-Atom, P-Atom oder B-Atom binden, auch durch eine Einfachbindung oder eine Brücke, ausgewählt aus $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ und $P(=O)R^2$, miteinander verbrückt sein;

$R^2$ ist bei jedem Auftreten gleich oder verschieden H, D, F, Cl, Br, I, CN, $B(OR^3)_2$, CHO, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, eine geradkettige Alkyl-, Alkoxy- oder Thioalkoxygruppe mit 1 bis 40 C-Atomen oder eine verzweigte oder cyclische Alkyl-, Alkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $-R^3C=CR^3-$, $-C\equiv C-$, $Si(R^3)_2$, C=O, C=S, $C=NR^3$, -C(=O)O-, $-C(=O)NR^3-$, $NR^3$, $P(=O)(R^3)$, -O-, -S-, SO oder $SO_2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 40 aromatischen Ringatomen, das jeweils durch einen oder mehrere Reste $R^3$ substituiert sein kann, oder eine Aryloxy- oder Heteroaryloxygruppe mit 5 bis 40 aromatischen Ringatomen, die durch einen oder mehrere Reste $R^3$ substituiert sein kann, oder eine Kombination dieser Systeme; dabei können zwei oder mehrere benachbarte Substituenten $R^2$ auch miteinander ein mono- oder poly-cyclisches, aliphatisches oder aromatisches Ringsystem bilden;

$R^3$ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch H-Atome durch F ersetzt sein können; dabei können zwei oder mehrere benachbarte Substituenten $R^3$ auch miteinander ein mono- oder polycyclisches, aliphatisches oder aromatisches Ringsystem bilden;

n ist 0, 1, 2 oder 3;

wobei unter einem aromatischen oder heteroaromatischen Ringsystem ein System zu verstehen ist, das nicht notwendigerweise nur Aryl- oder Heteroarylgruppen enthält, sondern in dem auch eine oder mehrere Aryl- oder Heteroarylgruppen durch ein C-, N- oder O-Atom oder eine Carbonylgruppe unterbrochen sein können;
wobei Systeme, in denen zwei oder mehrere Arylgruppen durch eine lineare oder cyclische Alkylgruppe oder durch eine Silylgruppe unterbrochen sind, ebenso als aromatische Ringsysteme zu verstehen sind; und
wobei Systeme, in denen zwei oder mehrere Aryl- oder Heteroarylgruppen direkt aneinander gebunden sind, ebenfalls als aromatisches beziehungsweise heteroaromatisches Ringsystem zu verstehen sind.

**2.** Verbindung gemäß Anspruch 1, **dadurch gekennzeichnet, dass** zwei benachbarte Gruppen W jeweils für $CR^1$ stehen und zusammen eine Gruppe der Formel (W-1) bilden

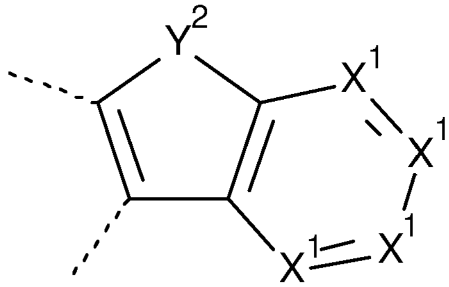

Formel (W-1)

worin

$Y^2$ N(Ar), O, S oder $C(R^2)_2$, ist,

$X^1$ bei jedem Auftreten gleich oder verschieden N oder $CR^2$, vorzugsweise $CR^2$ ist, mit der Maßgabe, dass nicht mehr als zwei der Gruppen X in einem Cyclus für N stehen, wobei $R^2$ die in Anspruch 1 genannte Bedeutung aufweist,

Ar ist bei jedem Auftreten gleich oder verschieden ein aromatisches oder heteroaromatisches Ringsystem mit 5 bis 30 aromatischen Ringatomen, welches durch einen oder mehrere Reste $R^2$ substituiert sein kann, der Rest $R^2$ die zuvor in Anspruch 1 genannte Bedeutung aufweisen, und

die gestrichelten Linien die Bindungen zu den benachbarten Atomen darstellen, wobei die Verbindungen umfassend Strukturen gemäß der Formel (I), (II) oder Formel (III) vorzugsweise höchstens eine Gruppe der Formel (W-1) pro Struktur aufweist.

**3.** Verbindungen gemäß mindestens einem der vorhergehenden Ansprüche, **dadurch gekennzeichnet, dass** die Verbindung mindestens eine der Strukturen der Formeln (IVc), (Vc) und/oder (VIc) umfasst

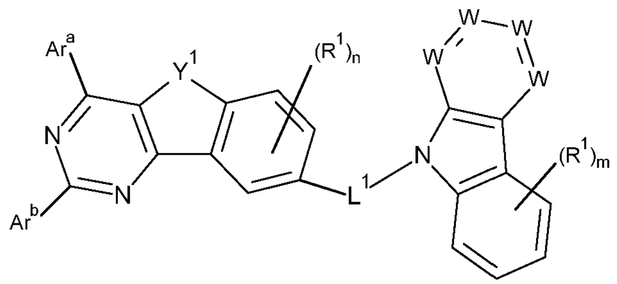

Formel (IVc)

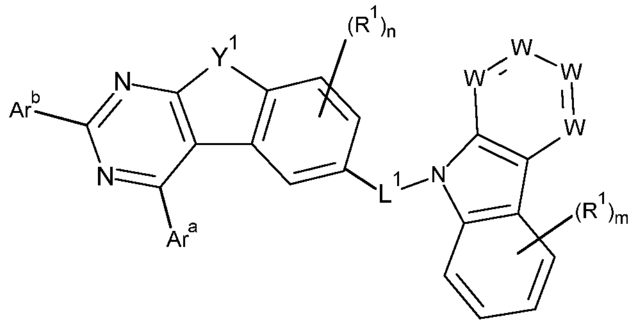

Formel (Vc)

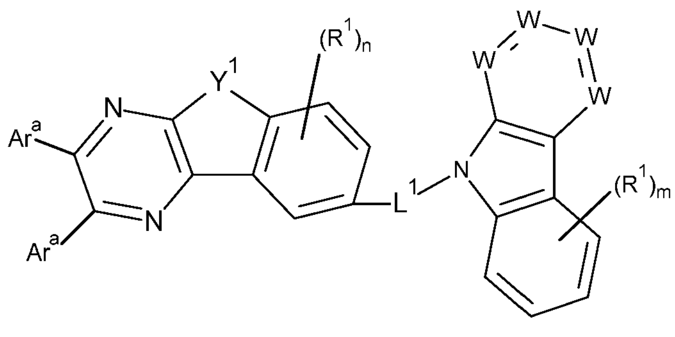

Formel (VIc)

worin die Symbole $Ar^a$, $Ar^b$, $Y^1$, $L^1$, $R^1$, n und W die in Anspruch 1 oder 2 dargelegte Bedeutung aufweisen und m 0, 1, 2, 3 oder 4 ist,
wobei Strukturen der Formel (IVc) und/oder (Vc) bevorzugt sind.

**4.** Verbindung gemäß mindestens einem der vorhergehenden Ansprüche, **dadurch gekennzeichnet, dass** die Gruppe $Ar^b$ eine Gruppe der Formel ($Ar^b$-1) darstellt

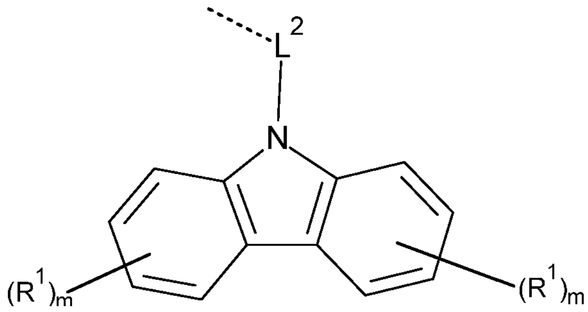

Formel ($Ar^b$-1)

worin $L^2$ eine Bindung oder ein aromatisches oder heteroaromatisches Ringsystem mit 5 bis 14 aromatischen oder heteroaromatischen Ringatomen ist, welches durch einen oder mehrere Reste $R^1$ substituiert sein kann, das Symbol $R^1$ die in Anspruch 1 genannte Bedeutung aufweist, m 0, 1, 2, 3 oder 4 ist und die gestrichelte Linie die Bindung darstellt.

**5.** Oligomer, Polymer oder Dendrimer enthaltend eine oder mehrere Verbindungen nach einem oder mehreren der Ansprüche 1 bis 4, wobei ein oder mehrere Bindungen der Verbindung zum Polymer, Oligomer oder Dendrimer vorhanden sind.

**6.** Zusammensetzung enthaltend wenigstens eine Verbindung nach einem oder mehreren der Ansprüche 1 bis 4 und/oder ein Oligomer, Polymer oder Dendrimer nach Anspruch 5 und wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

**7.** Formulierung enthaltend mindestens eine Verbindung nach einem oder mehreren der Ansprüche 1 bis 4, ein Oligomer, Polymer oder Dendrimer nach Anspruch 5 und/oder wenigstens eine Zusammensetzung nach Anspruch 6 und mindestens ein Lösungsmittel.

**8.** Verfahren zur Herstellung einer Verbindung nach einem oder mehreren der Ansprüche 1 bis 4 oder eines Oligomers, Polymers oder Dendrimers nach Anspruch 5, **dadurch gekennzeichnet, dass** in einer Kupplungsreaktion eine Verbindung, umfassend mindestens eine Diazadibenzofuran- oder Diazadibenzothiophen-Gruppe, mit einer Gruppe, umfassend mindestens einen Carbazol-Rest, verbunden wird.

**9.** Verwendung einer Verbindung nach einem oder mehreren der Ansprüche 1 bis 4, eines Oligomers, Polymers oder Dendrimers nach Anspruch 5 oder einer Zusammensetzung nach Anspruch 6 in einer elektronischen Vorrichtung als Hostmaterial, Matrixmaterial, Elektronentransportmaterial, Elektroneninjektionsmaterial und/oder Lochblockiermaterial.

**10.** Elektronische Vorrichtung, enthaltend mindestens eine Verbindung nach einem oder mehreren der Ansprüche 1 bis 4 oder ein Oligomer, Polymer oder Dendrimer nach Anspruch 5 oder eine Zusammensetzung nach Anspruch 6, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laser-dioden.

## Claims

**1.** Compound comprising structures of the formulae (Ic), (IIc) and/or (IIIc)

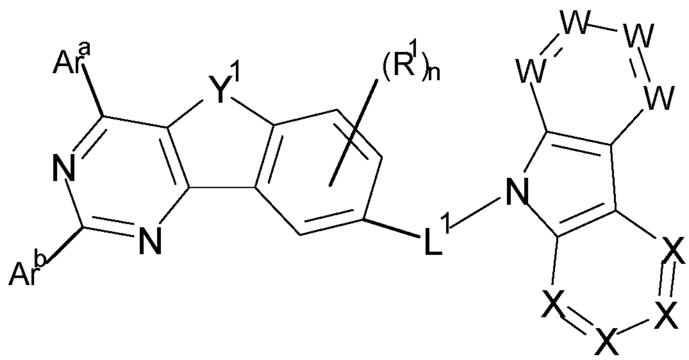

formula (Ic)

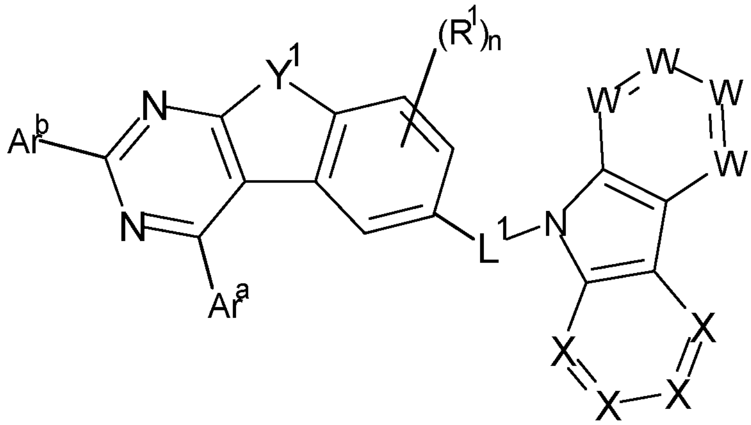

formula (IIc)

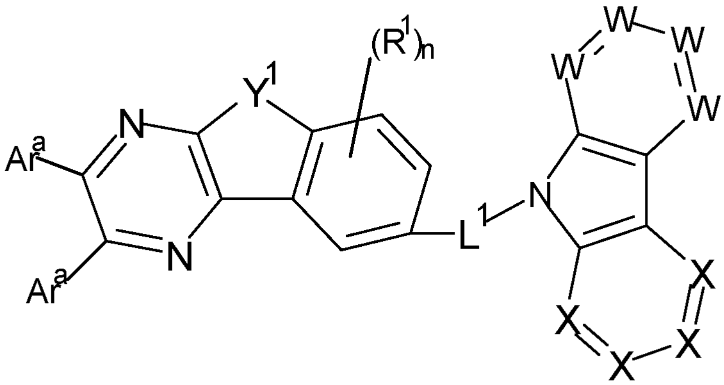

formula (IIIc)

where the following applies to the symbols used:

$Y^1$ is O or S;

W is on each occurrence, identically or differently, N or $CR^1$, preferably $CR^1$, with the proviso that not more than two of the groups W in a ring stand for N;

X is on each occurrence, identically or differently, N or $CR^1$, preferably $CR^1$, with the proviso that not more than two of the groups X in a ring stand for N;

$L^1$ is a bond or an aromatic or heteroaromatic ring system having 5 to 30 aromatic ring atoms, which may be substituted by one or more radicals $R^1$;

$Ar^a$ is on each occurrence, identically or differently, CN, F or an aromatic or heteroaromatic ring system having 5 to 30 aromatic ring atoms, which may be substituted by one or more radicals $R^1$;

$Ar^b$ is on each occurrence, identically or differently, CN, F or an aromatic or heteroaromatic ring system having 5 to 30 aromatic ring atoms, which may be substituted by one or more radicals $R^1$;

$R^1$ is on each occurrence, identically or differently, H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^2$, where one or more non-adjacent $CH_2$ groups may be replaced by $-R^2C=CR^2-$, -C=C-, $Si(R^2)_2$, C=O, C=S, $C=NR^2$, -C(=O)O-, $-C(=O)NR^2-$, $NR^2$, $P(=O)(R^2)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^2$, or an aryloxy or heteroaryloxy group having 5 to 40 aromatic ring atoms, which may be substituted by one or more radicals $R^2$, or an aralkyl or heteroaralkyl group having 5 to 40 aromatic ring atoms, which may be substituted by one or more radicals $R^2$, or a combination of these systems; two or more substituents $R^1$ may also form a mono- or polycyclic, aliphatic or aromatic ring system with one another;

$Ar^1$ is on each occurrence, identically or differently, an aromatic or heteroaromatic ring system having 5 to 30 aromatic ring atoms, which may be substituted by one or more non-aromatic radicals $R^2$; two radicals $Ar^1$ that are bonded to the same Si atom, N atom, P atom or B atom may also be bridged to one another by a single bond or a bridge selected from $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ and $P(=O)R^2$;

$R^2$ is on each occurrence, identically or differently, H, D, F, Cl, Br, I, CN, $B(OR^3)_2$, CHO, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, a straight-chain alkyl, alkoxy or thioalkoxy group having 1 to 40 C atoms or a branched or cyclic alkyl, alkoxy or thioalkoxy group having 3 to 40 C atoms, which may in each case be substituted by one or more radicals $R^3$, where one or more non-adjacent $CH_2$ groups may be replaced by $-R^3C=CR^3-$, $-C\equiv C-$, $Si(R^3)_2$, C=O, C=S, $C=NR^3$, -C(=O)O-, $-C(=O)NR^3-$, $NR^3$, $P(=O)(R^3)$, -O-, -S-, SO or $SO_2$ and where one or more H atoms may be replaced by D, F, Cl, Br, I, CN or $NO_2$, or an aromatic or heteroaromatic ring system having 5 to 40 aromatic ring atoms, which may in each case be substituted by one or more radicals $R^3$, or an aryloxy or heteroaryloxy group having 5 to 40 aromatic ring atoms, which may be substituted by one or more radicals $R^3$, or a combination of these systems; two or more adjacent substituents $R^2$ may also form a mono- or polycyclic, aliphatic or aromatic ring system with one another;

$R^3$ is on each occurrence, identically or differently, H, D, F or an aliphatic, aromatic and/or heteroaromatic hydrocarbon radical having 1 to 20 C atoms, in which, in addition, H atoms may be replaced by F; two or more adjacent substituents $R^3$ may also form a mono- or polycyclic, aliphatic or aromatic ring system with one another;

n is 0, 1, 2 or 3;

where an aromatic or heteroaromatic ring system is to be understood as meaning a system which does not necessarily contain only aryl or heteroaryl groups, but in which, in addition, one or more aryl or heteroaryl groups may be interrupted by a C, N or O atom or a carbonyl group;

where systems in which two or more aryl groups are interrupted by a linear or cyclic alkyl group or by a silyl group are likewise to be understood as aromatic ring systems; and

where systems in which two or more aryl or heteroaryl groups are bonded directly to one another are likewise to be understood as aromatic or heteroaromatic ring system respectively.

2. Compound according to Claim 1, **characterised in that** two adjacent groups W each stand for $CR^1$ and together form a group of the formula (W-1)

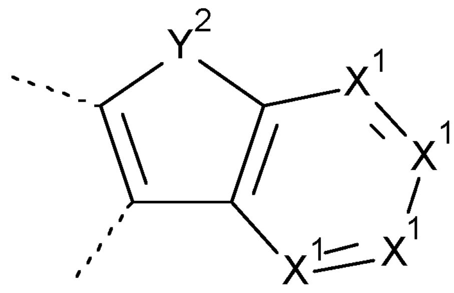

formula (W-1)

in which

$Y^2$ is N(Ar), O, S or $C(R^2)_2$,
$X^1$ is on each occurrence, identically or differently, N or $CR^2$, preferably $CR^2$, with the proviso that not more than two of the groups X in a ring stand for N, where $R^2$ has the meaning given in Claim 1,
Ar is on each occurrence, identically or differently, an aromatic or heteroaromatic ring system having 5 to 30 aromatic ring atoms, which may be substituted by one or more radicals $R^2$, the radical $R^2$ has the meaning given above in Claim 1, and

the dashed lines represent the bonds to the adjacent atoms, where the compounds comprising structures of the formula (I), (II) or formula (III) preferably contain at most one group of the formula (W-1) per structure.

3. Compounds according to at least one of the preceding claims, **characterised in that** the compound comprises at least one of the structures of the formulae (IVc), (Vc) and/or (VIc)

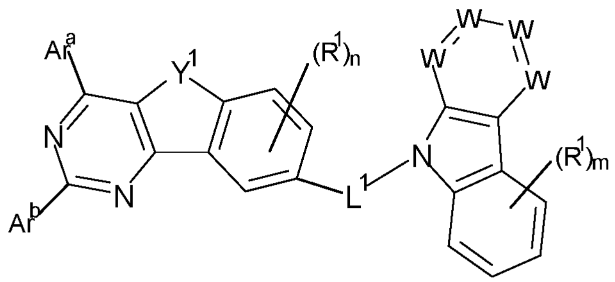

formula (IVc)

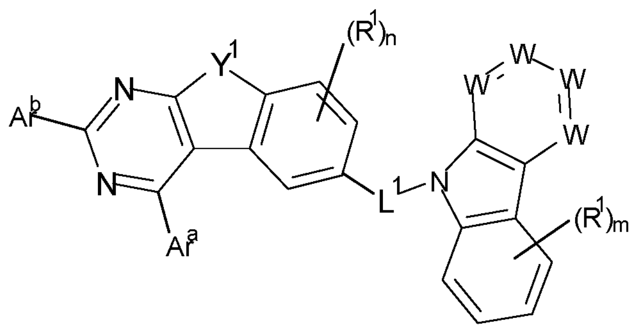

formula (Vc)

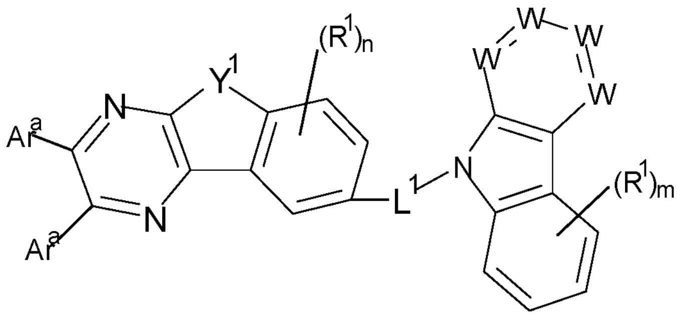

formula (VIc)

in which the symbols $Ar^a$, $Ar^b$, $Y^1$, $L^1$, $R^1$, n and W have the meaning given in Claim 1 or 2 and m is 0, 1, 2, 3 or 4, where structures of the formula (IVc) and/or (Vc) are preferred.

**4.** Compound according to at least one of the preceding claims, **characterised in that** the group $Ar^b$ represents a group of the formula ($Ar^b$-1)

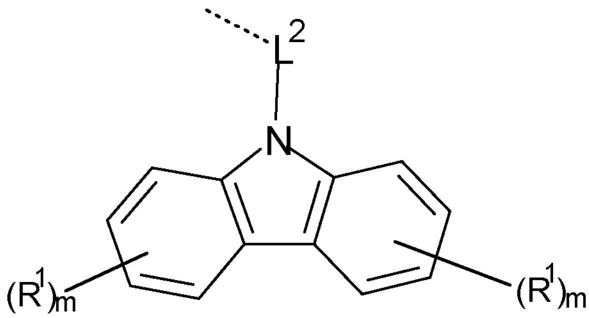

formula ($Ar^b$-1)

in which $L^2$ is a bond or an aromatic or heteroaromatic ring system having 5 to 14 aromatic or heteroaromatic ring atoms, which may be substituted by one or more radicals $R^1$, the symbol $R^1$ has the meaning given in Claim 1, m is 0, 1, 2, 3 or 4 and the dashed line represents the bond.

**5.** Oligomer, polymer or dendrimer containing one or more compounds according to one or more of Claims 1 to 4, where one or more bonds are present from the compound to the polymer, oligomer or dendrimer.

**6.** Composition comprising at least one compound according to one or more of Claims 1 to 4 and/or an oligomer, polymer or dendrimer according to Claim 5 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**7.** Formulation comprising at least one compound according to one or more of Claims 1 to 4, an oligomer, polymer or dendrimer according to Claim 5 and/or at least one composition according to Claim 6 and at least one solvent.

**8.** Process for the preparation of a compound according to one or more of Claims 1 to 4 or an oligomer, polymer or dendrimer according to Claim 5, **characterised in that** a compound containing at least one diazadibenzofuran or diazadibenzothiophene group is connected to a group containing at least one carbazole radical in a coupling reaction.

**9.** Use of a compound according to one or more of Claims 1 to 4, an oligomer, polymer or dendrimer according to Claim 5 or a composition according to Claim 6 in an electronic device as host material, matrix material, electron-transport material, electron-injection material and/or hole-blocking material.

10. Electronic device containing at least one compound according to one or more of Claims 1 to 4 or an oligomer, polymer or dendrimer according to Claim 5 or a composition according to Claim 6, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

1. Composé comprenant des structures de formules (Ic), (IIc) et/ou (IIIc)

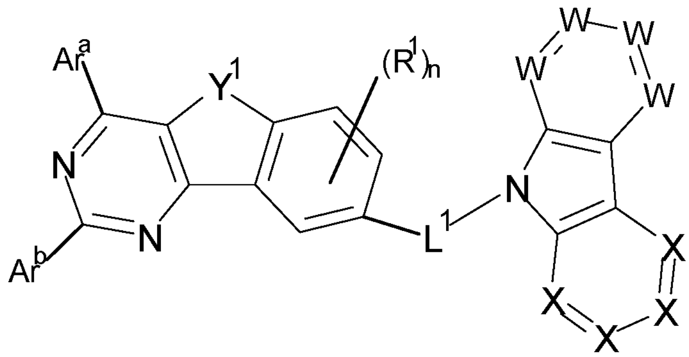

formule (Ic)

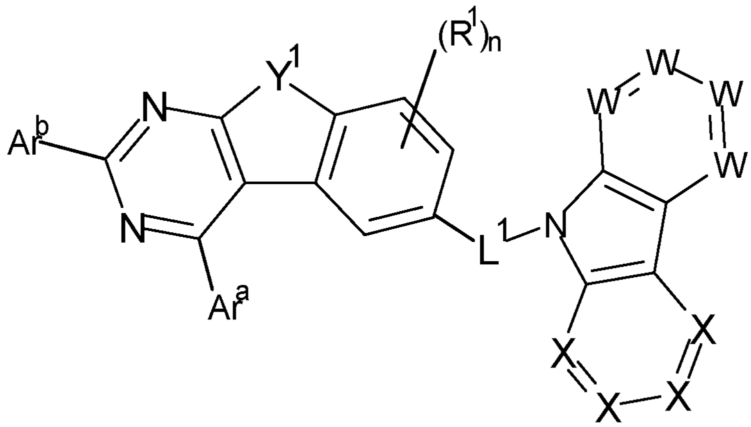

formule (IIc)

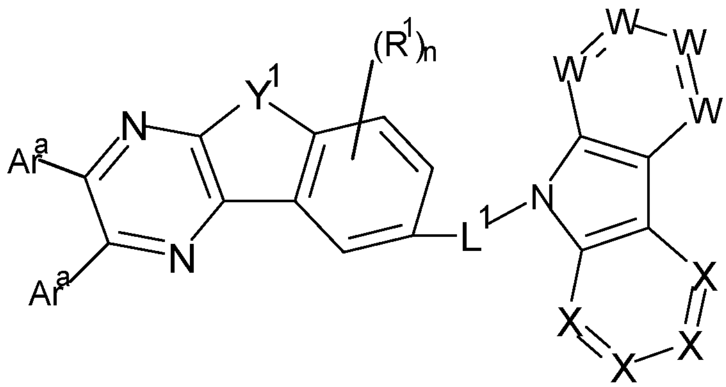

formule (IIIc)

dans lesquelles ce qui suit s'applique aux symboles utilisés :

$Y^1$ est O ou S ;
W est à chaque occurrence, de manière identique ou différente, N ou $CR^1$, préférablement $CR^1$, à condition qu'au plus deux parmi les groupements W dans un cycle représentent N ;
X est à chaque occurrence, de manière identique ou différente, N ou $CR^1$, préférablement $CR^1$, à condition qu'au plus deux parmi les groupements X dans un cycle représentent N ;
$L^1$ est une liaison ou un noyau aromatique ou hétéroaromatique ayant de 5 à 30 atomes de cycle aromatique, pouvant être substitué par un ou plusieurs radicaux $R^1$ ;
$Ar^a$ est à chaque occurrence, de manière identique ou différente, CN, F ou un noyau aromatique ou hétéroaromatique ayant de 5 à 30 atomes de cycle aromatique, pouvant être substitué par un ou plusieurs radicaux $R^1$ ;
$Ar^b$ est à chaque occurrence, de manière identique ou différente, CN, F ou un noyau aromatique ou hétéroaromatique ayant de 5 à 30 atomes de cycle aromatique, pouvant être substitué par un ou plusieurs radicaux $R^1$ ;
$R^1$ est à chaque occurrence, de manière identique ou différente, H, D, F, Cl, Br, I, CN, $NO_2$, $N(Ar^1)_2$, $N(R^2)_2$, $C(=O)Ar^1$, $C(=O)R^2$, $P(=O)(Ar^1)_2$, $P(Ar^1)_2$, $B(Ar^1)_2$, $Si(Ar^1)_3$, $Si(R^2)_3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, pouvant dans chaque cas être substitué par un ou plusieurs radicaux $R^2$, où un ou plusieurs groupements $CH_2$ non adjacents peuvent être remplacés par $-R^2C=CR^2-$, -C=C-, $Si(R^2)_2$, C=O, C=S, $C=NR^2$, -C(=O)O-, $-C(=O)NR^2-$, $NR^2$, $P(=O)(R^2)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, pouvant dans chaque cas être substitué par un ou plusieurs radicaux $R^2$, ou un groupement aryloxy ou hétéroaryloxy ayant de 5 à 40 atomes de cycle aromatique, pouvant être substitué par un ou plusieurs radicaux $R^2$, ou un groupement aralkyle ou hétéroaralkyle ayant de 5 à 40 atomes de cycle aromatique, pouvant être substitué par un ou plusieurs radicaux $R^2$, ou une combinaison de ces noyaux ; deux, ou plus, substituants $R^1$ peuvent également former un noyau mono- ou polycyclique, aliphatique ou aromatique les uns avec les autres ;
$Ar^1$ est à chaque occurrence, de manière identique ou différente, un noyau aromatique ou hétéroaromatique ayant de 5 à 30 atomes de cycle aromatique, pouvant être substitué par un ou plusieurs radicaux non aromatiques $R^2$ ; deux radicaux $Ar^1$ qui sont liés au même atome de Si, atome de N, atome de P ou atome de B peuvent également être pontés l'un avec l'autre par une liaison simple ou un pont choisi parmi $B(R^2)$, $C(R^2)_2$, $Si(R^2)_2$, C=O, $C=NR^2$, $C=C(R^2)_2$, O, S, S=O, $SO_2$, $N(R^2)$, $P(R^2)$ et $P(=O)R^2$ ;
$R^2$ est à chaque occurrence, de manière identique ou différente, H, D, F, Cl, Br, I, CN, $B(OR^3)_2$, CHO, $C(=O)R^3$, $CR^3=C(R^3)_2$, $C(=O)OR^3$, $C(=O)N(R^3)_2$, $Si(R^3)_3$, $P(R^3)_2$, $B(R^3)_2$, $N(R^3)_2$, $NO_2$, $P(=O)(R^3)_2$, $OSO_2R^3$, $OR^3$, $S(=O)R^3$, $S(=O)_2R^3$, un groupement alkyle, alcoxy ou thioalcoxy à chaîne linéaire ayant de 1 à 40 atomes de C ou un groupement alkyle, alcoxy ou thioalcoxy ramifié ou cyclique ayant de 3 à 40 atomes de C, pouvant dans chaque cas être substitué par un ou plusieurs radicaux $R^3$, où un ou plusieurs groupements $CH_2$ non adjacents peuvent être remplacés par $-R^3C=CR^3-$, -C=C-, $Si(R^3)_2$, C=O, C=S, $C=NR^3$, -C(=O)O-, $-C(=O)NR^3-$, $NR^3$, $P(=O)(R^3)$, -O-, -S-, SO ou $SO_2$ et où un ou plusieurs atomes de H peuvent être remplacés par D, F, Cl, Br, I, CN ou $NO_2$, ou un noyau aromatique ou hétéroaromatique ayant de 5 à 40 atomes de cycle aromatique, pouvant dans chaque cas être substitué par un ou plusieurs radicaux $R^3$, ou un groupement aryloxy ou hétéroaryloxy ayant de 5 à 40 atomes de cycle aromatique, pouvant être substitué par un ou plusieurs radicaux $R^3$, ou une combinaison de ces noyaux ; deux, ou plus, substituants $R^2$ adjacents peuvent également former un noyau mono- ou polycyclique, aliphatique ou aromatique les uns avec les autres ;
$R^3$ est à chaque occurrence, de manière identique ou différente, H, D, F ou un radical hydrocarboné aliphatique, aromatique et/ou hétéroaromatique ayant de 1 à 20 atomes de C, où, de plus, des atomes de H peuvent être remplacés par F ; deux, ou plus, substituants $R^3$ adjacents peuvent également former un noyau mono- ou polycyclique, aliphatique ou aromatique les uns avec les autres ;
n vaut 0, 1, 2 ou 3 ;

où le terme « noyau aromatique ou hétéroaromatique » doit être entendu comme signifiant un système qui ne contient pas nécessairement que des groupements aryle ou hétéroaryle, mais dans lequel, de plus, un ou plusieurs groupements aryle ou hétéroaryle peuvent être interrompus par un atome de C, N ou O ou un groupement carbonyle ;
où les systèmes dans lesquels deux, ou plus, groupements aryle sont interrompus par un groupement alkyle linéaire ou cyclique ou par un groupement silyle doivent de même être entendus comme étant des noyaux

aromatiques ; et

où les systèmes dans lesquels deux, ou plus, groupements aryle ou hétéroaryle sont liés directement l'un avec l'autre doivent de même être entendus comme étant un noyau aromatique ou hétéroaromatique, respectivement.

**2.** Composé selon la revendication 1, **caractérisé en ce que** deux groupements adjacents W représentent chacun $CR^1$ et forment ensemble un groupement de formule (W-1)

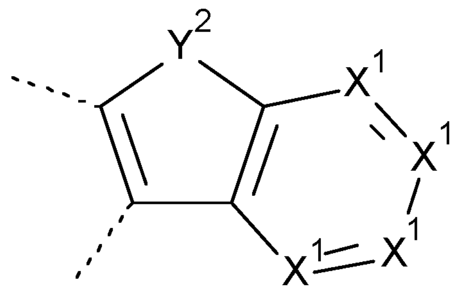

formule (W-1)

dans laquelle

$Y^2$ est N(Ar), O, S ou $C(R^2)_2$,

$X^1$ est à chaque occurrence, de manière identique ou différente, N ou $CR^2$, préférablement $CR^2$, à condition qu'au plus deux parmi les groupements X dans un cycle représentent N, où $R^2$ revêt la signification donnée selon la revendication 1,

Ar est à chaque occurrence, de manière identique ou différente, un noyau aromatique ou hétéroaromatique ayant de 5 à 30 atomes de cycle aromatique, pouvant être substitué par un ou plusieurs radicaux $R^2$, le radical $R^2$ revêtant la signification donnée ci-dessus selon la revendication 1, et

les lignes en pointillés représentent les liaisons aux atomes adjacents, où les composés comprenant des structures de formule (I), (II) ou de formule (III) préférablement contiennent au plus un groupement de formule (W-1) par structure.

**3.** Composés selon au moins l'une parmi les revendications précédentes, **caractérisé en ce que** le composé comprend au moins l'une des structures de formules (IVc), (Vc) et/ou (VIc)

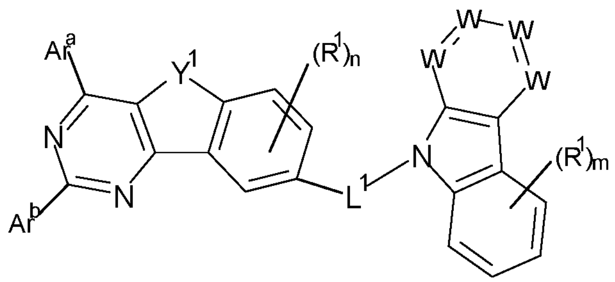

formule (IVc)

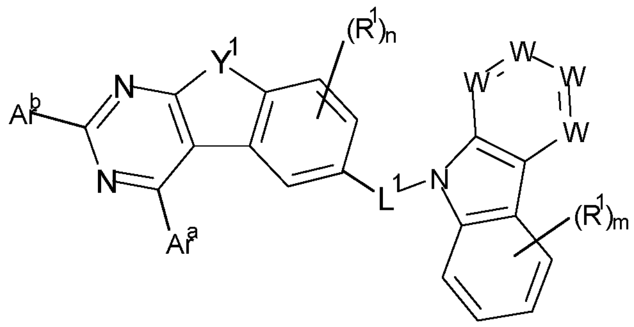

formule (Vc)

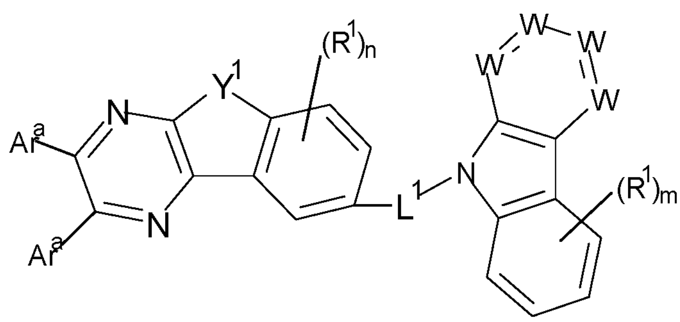

formule (VIc)

dans lesquelles les symboles $Ar^a$, $Ar^b$, $Y^1$, $L^1$, $R^1$, n et W revêtent la signification donnée selon la revendication 1 ou 2 et m vaut 0, 1, 2, 3 ou 4, où les structures de formule (IVc) et/ou (Vc) sont préférées.

4. Composé selon au moins l'une parmi les revendications précédentes, **caractérisé en ce que** le groupement $Ar^b$ représente un groupement de formule ($Ar^b$-1)

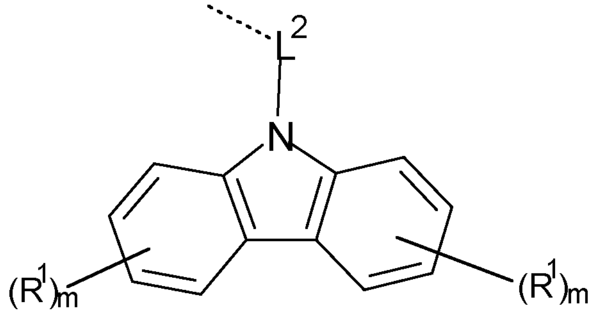

formule ($Ar^b$-1)

dans laquelle $L^2$ est une liaison ou un noyau aromatique ou hétéroaromatique ayant de 5 à 14 atomes de cycle aromatique ou hétéroaromatique, pouvant être substitué par un ou plusieurs radicaux $R^1$, le symbole $R^1$ revêtant la signification donnée selon la revendication 1, m vaut 0, 1, 2, 3 ou 4 et la ligne en pointillés représente la liaison.

5. Oligomère, polymère ou dendrimère contenant un ou plusieurs composés selon l'une ou plusieurs parmi les revendications 1 à 4, dans lequel une ou plusieurs liaisons sont présentes du composé au polymère, à l'oligomère ou au dendrimère.

6. Composition comprenant au moins un composé selon l'une ou plusieurs parmi les revendications 1 à 4 et/ou un oligomère, polymère ou dendrimère selon la revendication 5 et au moins un autre composé choisi dans le groupe

constitué par les émetteurs fluorescents, les émetteurs phosphorescents, les matériaux hôtes, les matériaux de matrice, les matériaux de transport d'électrons, les matériaux d'injection d'électrons, les matériaux de transport de trous, les matériaux d'injection de trous, les matériaux de blocage d'électrons et les matériaux de blocage de trous.

**7.** Formulation comprenant au moins un composé selon l'une ou plusieurs parmi les revendications 1 à 4, un oligomère, polymère ou dendrimère selon la revendication 5 et/ou au moins une composition selon la revendication 6 et au moins un solvant.

**8.** Procédé de préparation d'un composé selon l'une ou plusieurs parmi les revendications 1 à 4 ou d'un oligomère, polymère ou dendrimère selon la revendication 5, **caractérisé en ce qu'**un composé contenant au moins un groupement diazadibenzofurane ou diazadibenzothiophène est relié à un groupement contenant au moins un radical carbazole, dans une réaction de couplage.

**9.** Utilisation d'un composé selon l'une ou plusieurs parmi les revendications 1 à 4, d'un oligomère, polymère ou dendrimère selon la revendication 5 ou d'une composition selon la revendication 6, dans un dispositif électronique comme matériau hôte, matériau de matrice, matériau de transport d'électrons, matériau d'injection d'électrons et/ou matériau de blocage de trous.

**10.** Dispositif électronique contenant au moins un composé selon l'une ou plusieurs parmi les revendications 1 à 4 ou un oligomère, polymère ou dendrimère selon la revendication 5 ou une composition selon la revendication 6, le dispositif électronique étant préférablement choisi dans le groupe constitué par les dispositifs électroluminescents organiques, les circuits intégrés organiques, les transistors organiques à effet de champ, les transistors organiques à couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- JP 5604848 B
- WO 2015182872 A1
- KR 20160026661
- WO 2014157599 A1
- WO 2017119654 A1
- US 2015325799 A1
- WO 2015108301 A1
- EP 2910555 A1
- EP 842208 A
- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- EP 13004411
- EP 14000345
- EP 14000417
- EP 14002623
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- EP 14002104
- WO 2010108579 A
- WO 2005053051 A

- WO 2009030981 A


## In der Beschreibung aufgeführte Nicht-Patentliteratur


- **D. M. KOLLER et al.** *Nature Photonics,* 2008, 1-4
- **B. M. S. ARNOLD et al.** *Appl. Phys. Lett.,* 2008, vol. 92, 053301